# EUROPEAN PATENT APPLICATION

(11) **EP 4 674 416 A1**
(43) Date of publication of application: **07.01.2026**
(21) Application number: 24763910.7
(22) Date of filing: 27.02.2024
(51) Int. Cl.: A61K 31/454, A61K 31/501, A61K 31/4545, A61P 21/02, A61P 25/00, A61P 25/14, A61P 25/16, A61P 25/28, C07D 413/14, C07D 417/10, C07D 417/14, C07K 14/47, C12N 15/09

(54) **PROTEIN AGGREGATE DECOMPOSITION PROMOTING COMPOSITION AND PHARMACEUTICAL COMPOSITION FOR PREVENTION OR TREATMENT OF NEURODEGENERATIVE DISEASES ASSOCIATED WITH PROTEIN AGGREGATE FORMATION**

(30) Priority: 28.02.2023 JP 2023030021
(71) Applicant: NAGASAKI UNIVERSITY, Nagasaki 852-8521 (JP)
(72) Inventor: MATSUMOTO Gen, Nagasaki-shi, Nagasaki 852-8521 (JP); MIZUTA Satoshi, Nagasaki-shi, Nagasaki 852-8521 (JP)
(74) Representative: Witte, Weller & Partner Patentanwälte mbB
(86) International application number: PCT/JP2024/007056
(87) International publication number: WO 2024/181431

(57) **Abstract**

An object of the present invention is to provide a composition for promoting the degradation of a protein aggregate, and a pharmaceutical composition for prevention or treatment of a neurodegenerative disease associated with protein aggregate formation. The above problem is solved by a compound represented by General Formula (I) described below.

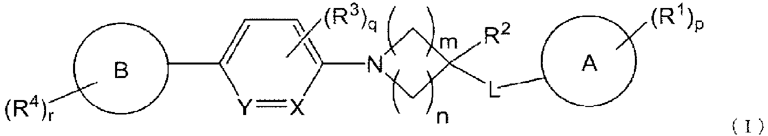

## Description

### Technical Field

The present invention relates to a composition for promoting the degradation of a protein aggregate, a pharmaceutical composition for prevention or treatment of a neurodegenerative disease associated with protein aggregate formation, a novel compound or a salt thereof, and methods for producing the same.

### Background Art

Neurodegenerative diseases typified by Alzheimer's disease and Parkinson's disease are diseases in which nerve cells are degenerated and die. Accumulation of protein aggregates is commonly observed pathologically in degenerated nerve cells. Such protein aggregates are not observed in brain regions other than the lesion site. This suggests that formation of protein aggregates is one of causes of neurodegenerative diseases, and that suppression of the accumulation of protein aggregates may lead to a common therapeutic agent for neurodegenerative diseases.

In diseases such as Alzheimer's disease (AD), a part of frontotemporal lobar degeneration (FTLD), progressive supranuclear palsy (PSP), and corticobasal degeneration (CBD), accumulation of aggregates of hyperphosphorylated tau proteins is pathologically confirmed, and these diseases are collectively referred to as tauopathies. On the other hand, in diseases such as Parkinson's disease (PD), multiple sclerosis (MS), dementia with Lewy bodies (DLB), and REM sleep behavior disorder (RBD), α-synuclein proteins form aggregates, and these diseases are collectively referred to as synucleinopathies. In addition, TDP-43, which is considered to be a cause of FTLD and amyotrophic lateral sclerosis (ALS), and prion protein (PrP), which is a cause of Creutzfeldt-Jakob disease (CJD) and bovine spongiform encephalopathy (BSE), also form aggregates in diseased cells. A common feature of these diseases is that, as the disease progresses, denatured proteins that form aggregates serve as templates to convert normal proteins into denatured forms, thereby propagating the aggregates between cells and expanding the disease lesion area.

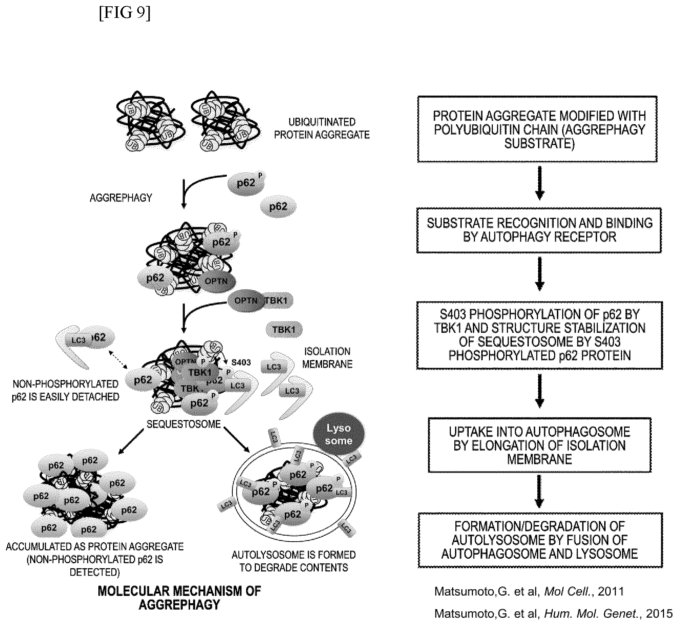

Protein aggregates are degraded in lysosomes via aggregate-selective autophagy. The process of recognizing aggregates and transporting them to lysosomes is specifically called aggrephagy. Polyubiquitin chains are added to aggregates, and recognized by autophagy receptors, whereby the aggregates are encapsulated in autophagosomes (see FIG. 9). The present inventors have reported that the serine residue at position 403 (S403) of the p62/SQSTM1 protein, which is the most commonly present among autophagy receptors, is phosphorylated, and thus binds to polyubiquitin chains with high affinity, and promotes the efficiency of uptake by autophagosomes (Non-Patent Documents 1 and 2).

### Citation List

### Non-Patent Document

Non-Patent Document 1: Matsumoto, G., et al. (2011) Serine 403 phosphorylation of p62 regulates selective autophagic clearance of ubiquitinated protein. Mol. Cell 44, 279-289
Non-Patent Document 2: Matsumoto, G., et al. (2015) TBK1 controls autophagic engulfment of Parkin-recruited depolarized mitochondria through p62 phosphorylation. Hum Mol Genet, 24, 4429-4442

### Summary of Invention

### Technical Problem

An object of the present invention is to provide a compound promoting the degradation of a protein aggregate by promoting the phosphorylation of serine at position 403 of human p62/SQSTM1 shown in SEQ ID NO: 1, and to provide a pharmaceutical for prevention or treatment of a neurodegenerative disease associated with protein aggregate formation, the pharmaceutical containing the compound as an active ingredient. Another object of the present invention is to provide a novel compound or a salt thereof and methods for producing the same.

### Solution to Problem

The present invention includes the following inventions in order to solve the above problems.
[1] A composition for promoting degradation of a protein aggregate, the composition containing a compound represented by General Formula (I) described below or a salt thereof: 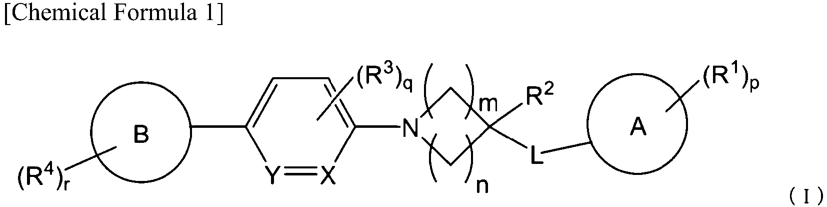
   where R¹s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylaminoamide group, a carboxyl group, an amino group, a nitro group, a C₁₋₆ alkylthio group, a C₁₋₆ haloalkylthio group, a C₁₋₆ alkylsulfinyl group, a C₁₋₆ haloalkylsulfinyl group, a C₁₋₆ alkylsulfonyl group, and a C₁₋₆ haloalkylsulfonyl group;
   R² is a hydrogen atom, a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a cyano group, a C(=S)NH₂ group, an amide group, or an SF₅ group;
   R³s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylamino group, a C₁₋₆ alkoxyamino group, a carboxyl group, and an amino group;
   R⁴s are selected from the group consisting of a halogen atom, a C₁₋₆ alkyl group, a C₁₋₆ haloalkyl group, a hydroxyl group, a hydroxy C₁₋₆ alkyl group, a carboxyl group, a C₁₋₆ alkylamino group, an amino group, and a functional group represented by a formula described below, 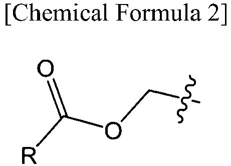
   (where R is a halogen atom, a C₁₋₆ alkyl group, a C₁₋₆ haloalkyl group, a C₃₋₈ cycloalkyl group, a C₁₋₆ alkylamino group, a C₁₋₆ haloalkylamino group, a C₁₋₆ alkylamide group, a C₁₋₆ haloalkylamide group, or a phenyl group, which may be substituted with a halogen atom, a C₁₋₆ alkyl group, or a C₁₋₆ haloalkyl group);
   L is -O-, -S-, -NH-, a C₁₋₆ alkylene group, which may be substituted with R, a C₂₋₆ alkenylene group, which may be substituted with R, a C₂₋₆ alkynylene group, which may be substituted with R, or a chemical bond;
   X and Y may be the same or different and are each N, CH or CR³;
   a ring A is an aromatic hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom;
   a ring B is an aromatic ring hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom, the heteroatoms including at least one nitrogen atom;
   p is 0, 1, 2, 3, 4 or 5;
   q is 0, 1 or 2;
   r is 0, 1, 2 or 3;
   when p, q and/or r are 2 or more, two or more R¹s, R³s and/or R⁴s may each form a ring together with a part of a ring to be substituted;
   m and n may be the same or different and are each 1, 2 or 3; and
   one or more of R¹s, R³s and Rs may be substituted with biotin or a derivative thereof.
[2] The composition according to [1], in which the ring B is selected from the group consisting of phenyl, naphthyl, oxazolyl, thiazolyl, imidazolyl, pyrrolyl, pyrazolyl, isoxazolyl, isothiazolyl, 1,2,4-oxadiazol-5-yl, 1,2,4-oxadiazol-3-yl, 1,3,4-oxadiazolyl, 1,2,5-oxadiazolyl, 1,2,3-oxadiazolyl, 1,2,4-thiadiazol-5-yl, 1,2,4-thiadiazol-3-yl, 1,2,5-thiadiazolyl, 1,3,4-thiadiazolyl, 1,2,3-thiadiazolyl, 1,2,4-triazolyl, 1,2,3-triazolyl, and tetrazolyl.
[3] The composition according to [1], in which the ring B has any one of structures described below: 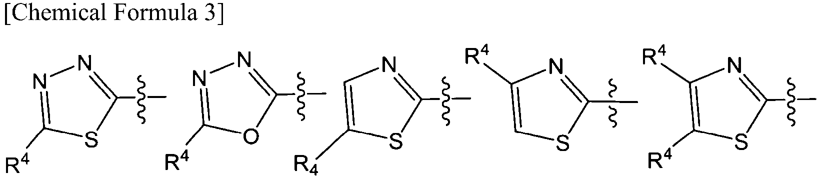 where R⁴ is as defined in [1].
[4] The composition according to [1], in which R⁴ is: 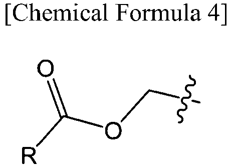 where R is as defined in [1].
[5] The composition according to [1], in which 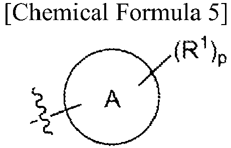 has any one of structures described below 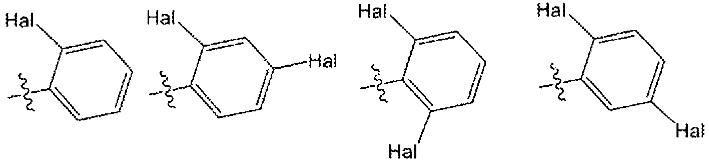 where Hals may be the same or different and are each halogen or C₁₋₆ haloalkyl.
[6] The composition according to [1], in which X and Y are both CH.
[7] A pharmaceutical composition for prevention or treatment of a neurodegenerative disease associated with protein aggregate formation, the pharmaceutical composition containing the composition according to any one of [1] to [6].
[8] The pharmaceutical composition according to [7], in which the neurodegenerative disease associated with protein aggregate formation is selected from the group consisting of Alzheimer's disease, progressive supranuclear palsy, corticobasal degeneration, frontotemporal lobar degeneration, Parkinson's disease, multiple system atrophy, dementia with Lewy bodies, REM sleep behavior disorder, striatonigral degeneration, Creutzfeldt-Jakob disease, Gerstmann-Straussler-Scheinker disease, amyotrophic lateral sclerosis, Huntington's disease, spinocerebellar degeneration, and dentatorubropallidoluysian atrophy.
[9] A composition for promoting p62/SQSTM1 phosphorylation, the composition comprising the compound represented by General Formula (I) or the salt thereof described in [1].
[10] The composition according to [8], in which a phosphorylation promoting site is serine at position 403 in an amino acid sequence of human p62/SQSTM1 shown in SEQ ID NO: 1, or serine corresponding to the serine at position 403 of SEQ ID NO: 1 in an amino acid sequence of p62/SQSTM1 of a non-human animal.
[11] A compound represented by General Formula (II) described below or a salt thereof: 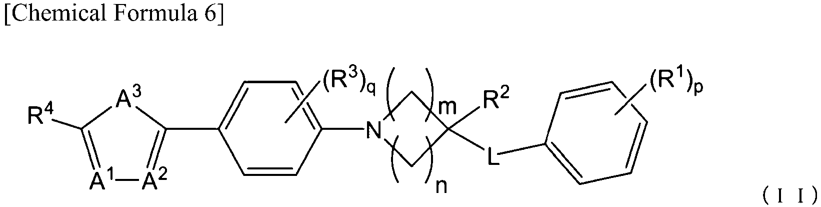
   where R¹s are independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylaminoamide group, a carboxyl group, an amino group, a nitro group, a C₁₋₆ alkylthio group, a C₁₋₆ haloalkylthio group, a C₁₋₆ alkylsulfinyl group, a C₁₋₆ haloalkylsulfinyl group, a C₁₋₆ alkylsulfonyl group, and a C₁₋₆ haloalkylsulfonyl group;
   R² is a hydrogen atom, a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a cyano group, a C(=S)NH₂ group, an amide group, or an SF₅ group;
   R³s are independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylamino group, a C₁₋₆ alkoxyamino group, a carboxyl group, and an amino group;
   R⁴s are selected from the group consisting of a halogen atom, a C₁₋₆ alkyl group, a C₁₋₆ haloalkyl group, a hydroxyl group, a hydroxy C₁₋₆ alkyl group, a carboxyl group, a C₁₋₆ alkylamino group, an amino group, and 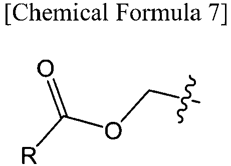
   (where R is a halogen atom, a C₁₋₆ alkyl group, a C₁₋₆ haloalkyl group, a C₃₋₈ cycloalkyl group, a C₁₋₆ alkylamino group, a C₁₋₆ haloalkylamino group, a C₁₋₆ alkylaminoamide group, a C₁₋₆ haloalkylamide group, or a phenyl group, where the phenyl group may be substituted with a C₁₋₆ alkyl group, a C₁₋₆ haloalkyl group, or a halogen atom);
   L is -O-, -S-, -NH-, -NR-, C₁₋₆ alkylene, which may be substituted with R, C₂₋₆ alkenylene, which may be substituted with R, C₂₋₆ alkynylene, which may be substituted with R, or a chemical bond;
   A¹, A² and A³ may be the same or different, and are independently selected from the group consisting of a sulfur atom, a nitrogen atom, CR⁴ (where R⁴ is a halogen atom, a C₁₋₆ alkyl group, a C₁₋₆ haloalkyl group, a hydroxyl group, a hydroxy C₁₋₆ alkyl group, a carboxyl group, or an amino group) and CH;
   p is 1, 2 or 3;
   q is 0 or 1; and
   m and n may be the same or different, and are 1, 2, or 3,
   provided that (i) compounds in which A¹ and A² are nitrogen atoms, A³ is an oxygen atom, and R⁴ is CH₂OH, and (ii) compounds in which A¹ and A² are nitrogen atoms, A³ is a sulfur atom, and R⁴ is CH₂-C(=O)-OCH₃ are excluded.
[12] A compound represented by a formula described below or a salt thereof: 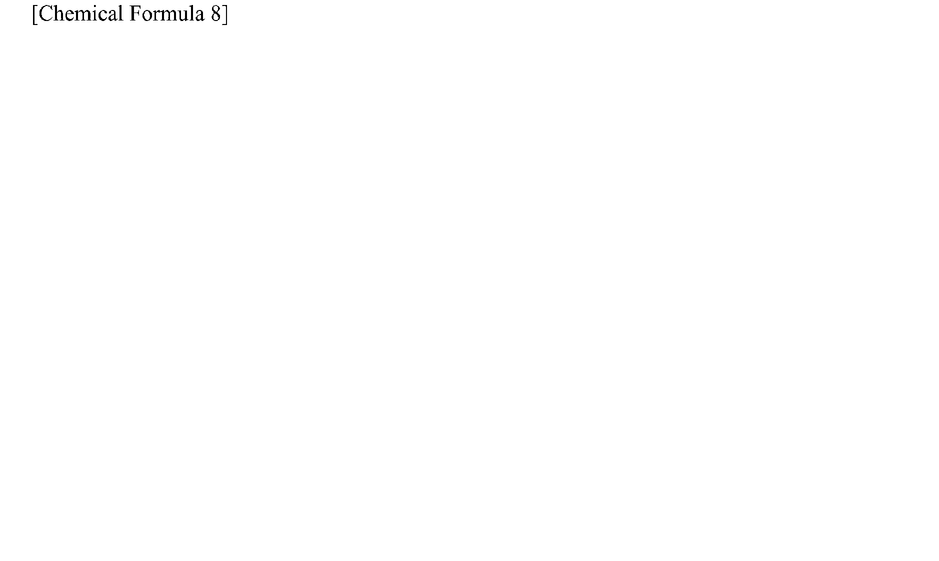 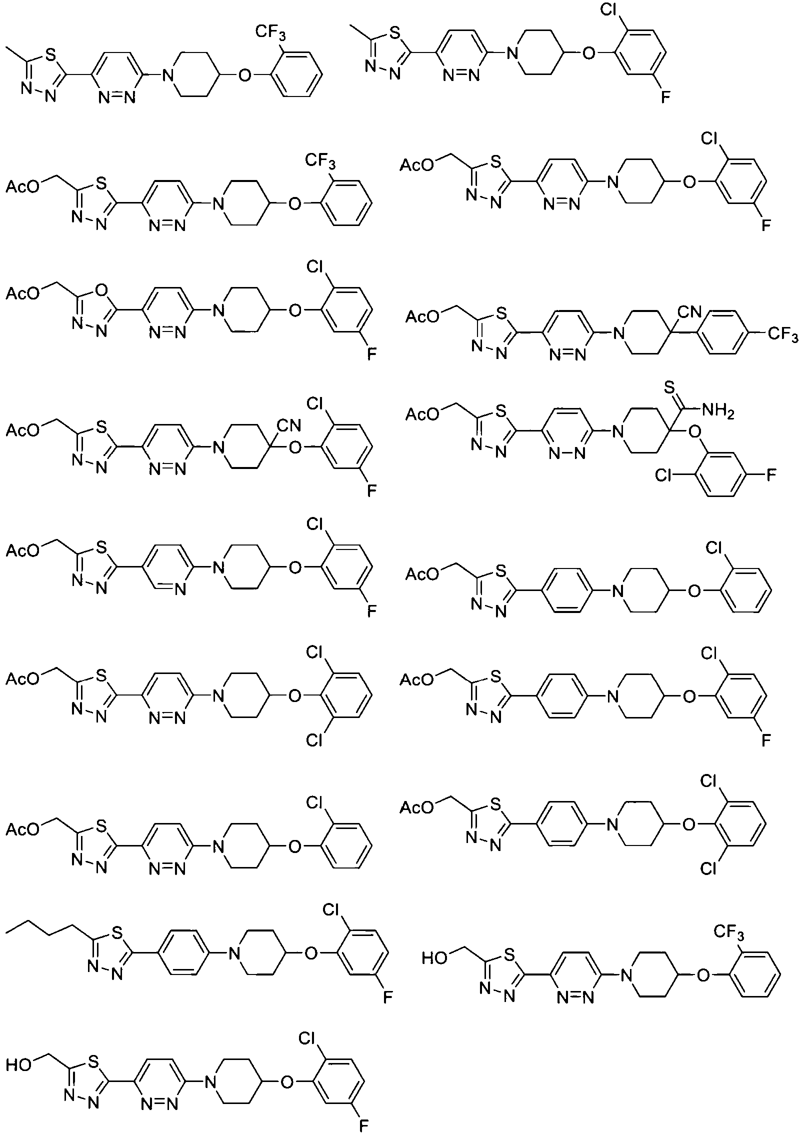 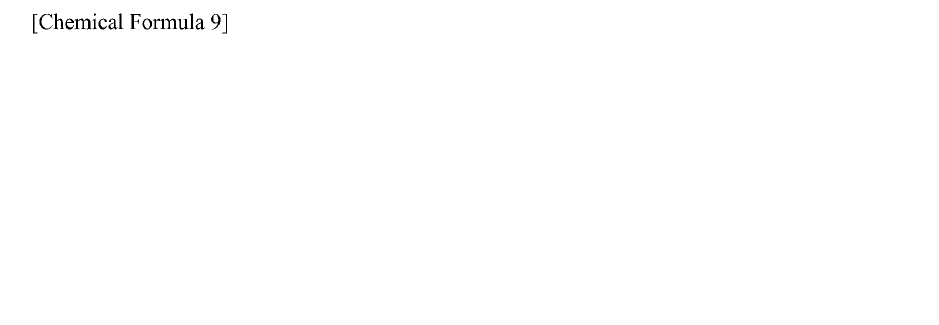 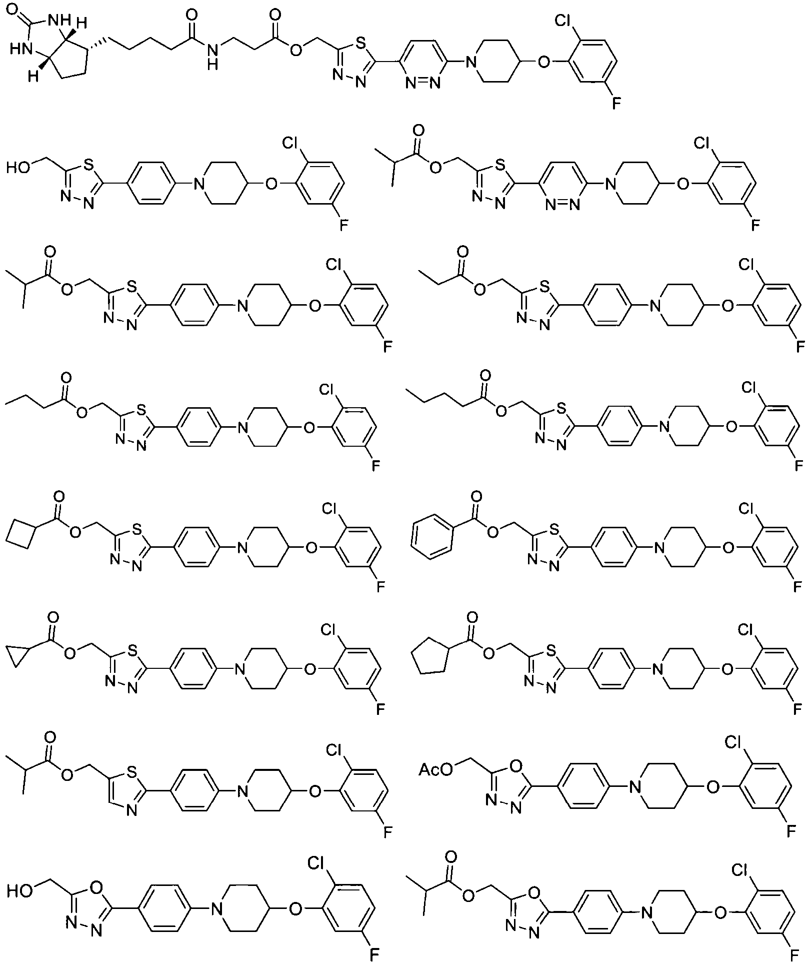
[13] A compound represented by a formula described below or a salt thereof:

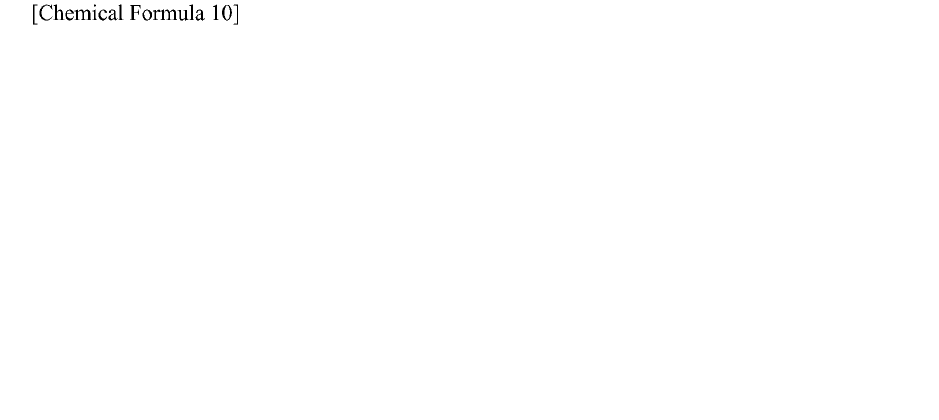

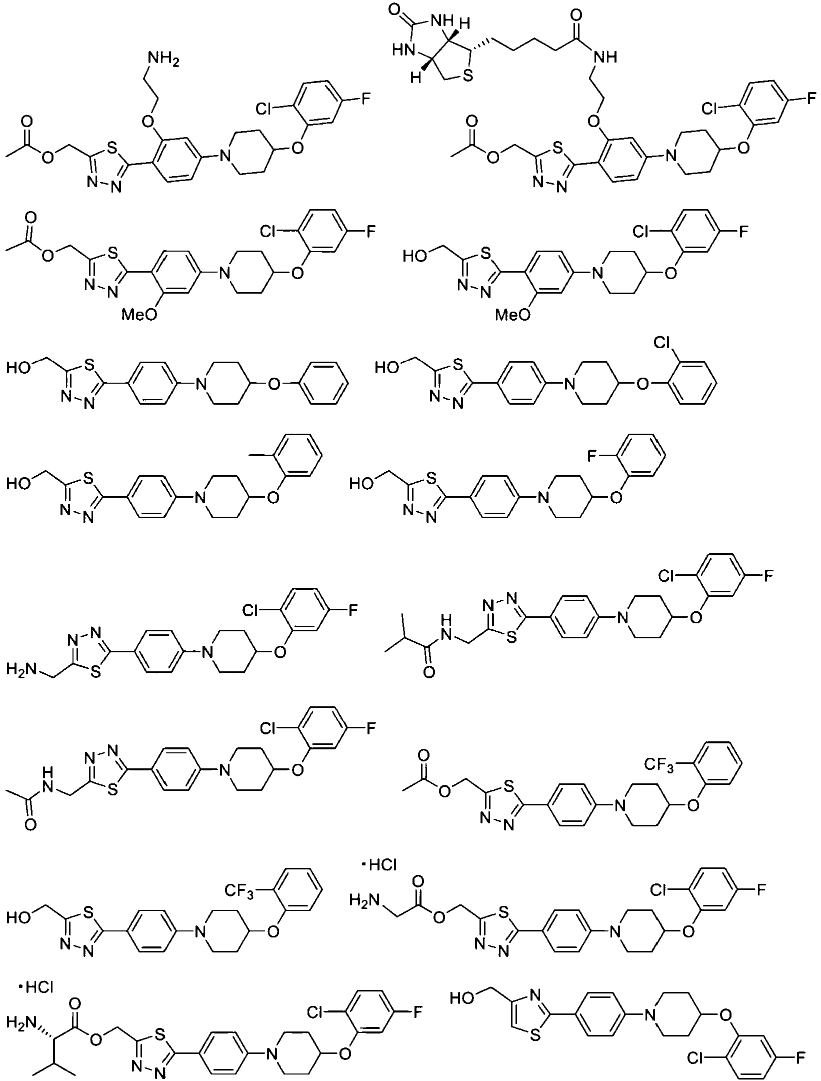

### Advantageous Effects of Invention

The present invention can provide a compound that promotes the degradation of a protein aggregate by promoting the phosphorylation of serine at position 403 of human p62/SQSTM1 represented by SEQ ID NO: 1. In addition, the present invention can provide a pharmaceutical for prevention or treatment of a neurodegenerative disease associated with protein aggregate formation, the pharmaceutical containing the compound of the present invention as an active ingredient. Further, the present invention can provide a novel compound or a salt thereof and methods for producing the same.

### Brief Description of Drawings

FIG. 1 shows results of Western blotting evaluation of an intracellular accumulated amount of S403 phosphorylated p62 in cultured cells treated with an ADI derivative (the compound of the present invention), in which (A) shows results of N2a cells, and (B) shows results of HEK293 cells.
FIG. 2 shows results of Western blotting evaluation of an intracellular accumulated amount of S403 phosphorylated p62 in N2a cells, which constantly and stably expressed mouse p62 protein (RFP-GFP-p62) in which red fluorescent protein (RFP) and green fluorescent protein (GFP) were linearly linked, after the N2a cells were treated with an ADI derivative alone, co-treated with an ADI derivative and bafilomycin A, or co-treated with an ADI derivative and BX795, in order to examine the phosphorylation promoting mechanism of the ADI derivative.
FIG. 3 shows results of quantitative PCR measurement of expression levels of genes expected to increase in expression level after treatment of N2a cells with an ADI derivative.
FIG. 4 shows results of quantitative PCR measurement of expression levels of the same genes as in FIG. 3 after forced oral administration of an ADI derivative to mice for 2 weeks and collection of the hippocampus.
FIG. 5 shows images obtained by photographing cells of a self-made tau aggregation cell line treated or not treated with an ADI derivative at an arbitrary time defined as "time 0", and the same cells (including daughter cells) after 2 days.
FIG. 6 shows (A) a temporal change in average number of aggregates every 1 hour, with the time about 1 hour after addition of a drug at which fixed-point observation is started as t = 0 and (B) an average value of proportions of cells occupying the visual field (defined as cell densities) in three wells when a proportion in a state where cells occupy the entire visual field is 100, both in the same experiment as in FIG. 5.
FIG. 7 shows results of brain tissue sections immunostained with an anti-phosphorylated tau antibody after forced oral administration of an ADI derivative to 36-week-old PS19 mice, which are neurodegenerative disease model animals forming amyloid protein aggregates, three times a week for 10 weeks.
FIG. 8 shows results of measuring a thickness of a CA1 nerve cell layer of the hippocampus in the same experiment as in FIG. 7, in which (A) shows images of brain tissue sections stained by immunofluorescence with an anti-NeuN monoclonal antibody, and (B) shows results of measuring a width of the CA1 nerve cell layer.
FIG. 9 is an explanatory diagram of a molecular mechanism of aggrephagy.

### Description of Embodiments

### [Compound and Salt thereof Used in Present Invention]

The compound of the present invention is sometimes referred to herein as ADI derivative or NUT compound. The compound is denoted as "ADI-X" or "NUT-X", where the same integer of about from 1 to 100 is substituted for "X". For example, the compound is sometimes denoted as ADI-14 (the same compound as NUT-14) or NUT-29 (the same compound as ADI-29). The hyphen before X may be omitted.

The compounds preferably used in the present invention are as described in [1] to [13] above.

Further, in the definition of a compound represented by General Formula (I) described below or a salt thereof: 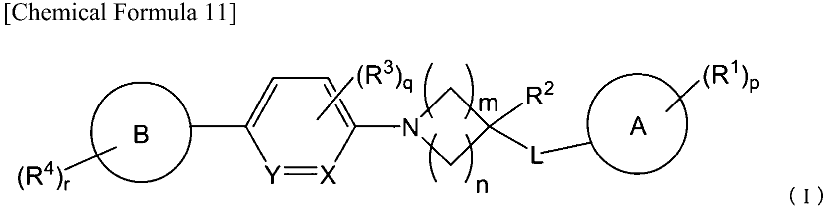
[where R¹s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylaminoamide group, a carboxyl group, an amino group, a nitro group, a C₁₋₆ alkylthio group, a C₁₋₆ haloalkylthio group, a C₁₋₆ alkylsulfinyl group, a C₁₋₆ haloalkylsulfinyl group, a C₁₋₆ alkylsulfonyl group, and a C₁₋₆ haloalkylsulfonyl group;
R² is a hydrogen atom, a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a cyano group, a C(=S)NH₂ group, an amide group, or an SF₅ group;
R³s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylamino group, a C₁₋₆ alkoxyamino group, a carboxyl group, and an amino group;
R⁴'s are selected from the group consisting of a halogen atom, a C₁₋₆ alkyl group, a C₁₋₆ haloalkyl group, a hydroxyl group, a hydroxy C₁₋₆ alkyl group, a carboxyl group, a C₁₋₆ alkylamino group, an amino group, and a functional group represented by a formula described below, 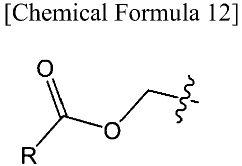
(where R is a halogen atom, a C₁₋₆ alkyl group, a C₁₋₆ haloalkyl group, a C₃₋₈ cycloalkyl group, a C₁₋₆ alkylamino group, a C₁₋₆ haloalkylamino group, a C₁₋₆ alkylamide group, a C₁₋₆ haloalkylamide group, or a phenyl group, which may be substituted with a halogen atom, a C₁₋₆ alkyl group, or a C₁₋₆ haloalkyl group);
L is -O-, -S-, -NH-, a C₁₋₆ alkylene group, which may be substituted with R, a C₂₋₆ alkenylene group, which may be substituted with R, a C₂₋₆ alkynylene group, which may be substituted with R, or a chemical bond;
X and Y may be the same or different and are each N, CH or CR³;
a ring A is an aromatic hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom;
a ring B is an aromatic ring hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom, the heteroatoms including at least one nitrogen atom;
p is 0, 1, 2,3,4 or 5;
q is 0, 1 or 2;
r is 0, 1, 2 or 3;
when p, q and/or r are 2 or more, two or more R¹s, R³s and/or R⁴s may each form a ring together with a part of a ring to be substituted;
m and n may be the same or different and are each 1, 2 or 3; and
one or more of R¹s, R³s and Rs may be substituted with biotin or a derivative thereof],
when p, q and/or r is 2 or more, two R¹s, R³s and/or R⁴s may form a ring together with part of the rings (that is, the ring A, the 6-membered ring containing X and Y, and the ring B) in which the respective substituents are present.

Examples of the ring formed by R¹, R³ and/or R⁴ include, but are not limited to, saturated or unsaturated 3- to 6-membered rings which may contain from 1 to 3 heteroatoms selected from the group consisting of a nitrogen atom, a sulfur atom and an oxygen atom, preferably saturated 5- to 6-membered rings which may contain from 1 to 2 of the heteroatoms. Specific examples of the ring include the following rings, but are not limited thereto.

### [Chemical Formula 13]

### [Example of Case where R⁴ Forms Ring A, R³ Forms 6-Membered Ring Containing X and Y, and R¹ Forms Ring A and Above Ring]

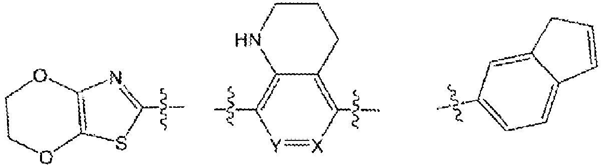

When X and/or Y are CR³, the number of R³'s in X and/or Y is not included in q.

The symbol "Hal" in the chemical formulas herein refers to halogen or a C₁₋₆ haloalkyl group, more preferably halogen.

In addition, the contents of the phrases "a compound represented by General Formula (I)", "a compound represented by General Formula (I) or a salt thereof", and the like used herein may include a compound represented by General Formula (II) or a salt thereof, and further, compounds produced in Examples described later.

In the present invention, the term "alkyl group" preferably refers to, but is not limited to, a C₁-C₆ alkyl group. Examples of the alkyl group include, but are not limited to, linear or branched alkyl groups such as a methyl group, an ethyl group, a propyl group, an isopropyl group, a butyl group, an isobutyl group, a tertiary butyl group, a pentyl group, an isopentyl group, a neopentyl group, a 2,3-dimethylpropyl group, and a benzyloxy group.

The above description is similarly applied to alkyl moieties in the "... alkyl groups" (for example, a haloalkyl group, an alkoxyalkyl group, a hydroxyalkyl group, and a sulfanylalkyl group) and the "alkyl ... groups" (for example, an alkylthio group, an alkylsulfanyl group, an alkylsulfonyl group, an alkylaminoamide group, and an alkylamino group). The same applies to the following description of each term.

The term "alkenyl group" preferably refers to, but is not limited to, a C₂-C₆ alkenyl group. Examples of the alkenyl group include, but are not limited to, linear or branched alkenyl groups such as a vinyl group, an allyl group, an isopropenyl group, a 1-butenyl group, and a 2-butenyl group.

The term "alkynyl group" preferably refers to, but is not limited to, a C₂-C₆ alkynyl group. Examples of the alkynyl group include, but are not limited to, linear or branched alkynyl groups such as an ethynyl group, a 1-propynyl group, a 2-propynyl group, a 1-butynyl group, and a 2-butynyl group.

The term "halogen" preferably refers to, but is not limited to, a chlorine atom, a bromine atom, an iodine atom or a fluorine atom, more preferably a chlorine atom or a fluorine atom.

The term "alkylene group" refers to a divalent substituent obtained by further removing one hydrogen atom at an arbitrary position from the "alkyl group" defined above. The term "alkenylene group" refers to a divalent substituent obtained by further removing one hydrogen atom at an arbitrary position from the "alkenyl group" defined above. The term "alkynylene group" refers to a divalent substituent obtained by further removing one hydrogen atom at an arbitrary position from the "alkynyl group" defined above.

The term "cycloalkyl group" preferably refers to, but is not limited to, a C₃-C₈ cycloalkyl group. Examples of the cycloalkyl group include, but are not limited to, cyclic alkyl groups such as a cyclopropyl group, a cyclobutyl group, a cyclopentyl group, a cyclohexyl group, and a cyclooctyl group.

The term "alkoxy group" preferably refers to, but is not limited to, a C₁-C₆ alkoxy group. Examples of the alkoxy group include, but are not limited to, linear or branched alkoxy groups such as a methoxy group, an ethoxy group, a propoxy group, an isopropoxy group, a butoxy group, a tertiary butoxy group, a pentyloxy group, an isopentyloxy group, and a neopentyloxy group.

The term "aromatic hydrocarbon" preferably refers to, but is not limited to, an aromatic hydrocarbon having from 5 to 10 carbon atoms, more preferably phenyl or naphthyl, and further preferably phenyl or the like.

The term "aromatic heterocycle" preferably refers to, but is not limited to, a 5- or 6-membered aromatic heterocycle containing from 1 to 4 heteroatoms selected from the group consisting of a nitrogen atom, a sulfur atom and an oxygen atom, more preferably a 5- or 6-membered aromatic heterocycle containing from 2 to 3 heteroatoms, and further preferably a 5-membered aromatic heterocycle containing 3 heteroatoms. Examples of the aromatic hydrocarbon include, but are not limited to, oxazolyl, thiazolyl, imidazolyl, pyrrolyl, pyrazolyl, isoxazolyl, isothiazolyl, 1,2,4-oxadiazol-5-yl, 1,2,4-oxadiazol-3-yl, 1,3,4-oxadiazolyl, 1,2,5-oxadiazolyl, 1,2,3-oxadiazolyl, 1,2,4-thiadiazol-5-yl, 1,2,4-thiadiazol-3-yl, 1,2,5-thiadiazolyl, 1,3,4-thiadiazolyl, 1,2,3-thiadiazolyl, 1,2,4-triazolyl, 1,2,3-triazolyl, and tetrazolyl, more preferably 1,2,4-oxadiazol-5-yl, 1,2,4-oxadiazol-3-yl, 1,3,4-oxadiazolyl, 1,2,5-oxadiazolyl, 1,2,3-oxadiazolyl, 1,2,4-thiadiazol-5-yl, 1,2,4-thiadiazol-3-yl, 1,2,5-thiadiazolyl, 1,3,4-thiadiazolyl, and 1,2,3-thiadiazolyl, and further preferably 1,2,4-thiadiazol-5-yl, 1,2,4-thiadiazol-3-yl, 1,2,5-thiadiazolyl, 1,3,4-thiadiazolyl, and 1,2,3-thiadiazolyl.

### [Composition for Promoting p62/SQSTM1 Phosphorylation]

The present invention provides a composition for promoting p62/SQSTM1 phosphorylation, the composition containing the compound represented by General Formula (I) or salt thereof. p62/SQSTM1 (hereinafter abbreviated as "p62") is a receptor protein that recognizes ubiquitin chains and directs specific proteins and organelles to autophagy, and is also called "sequestosome-1", "p62", and "A170".

The phosphorylation promoting composition of the present invention can promote the phosphorylation of serine at position 403 from the N-terminus of the amino acid sequence of human p62 shown in SEQ ID NO: 1. The phosphorylation promoting composition of the present invention can promote the phosphorylation of serine corresponding to the serine at position 403 of SEQ ID NO: 1 in the amino acid sequence of p62 of a non-human animal. The serine of p62 of a non-human animal corresponding to the serine at position 403 of SEQ ID NO: 1 can be identified by aligning the amino acid sequence of SEQ ID NO: 1 with the amino acid sequence of p62 of a non-human animal.

The serine at position 403 from the N-terminus of the amino acid sequence of human p62 shown in SEQ ID NO: 1 can be rephrased as serine at position 38 from the C-terminus of the amino acid sequence of human p62 shown in SEQ ID NO: 1. Thus, the phosphorylation promoting composition of the present invention can promote the phosphorylation of serine corresponding to the serine at position 38 from the C-terminus of SEQ ID NO: 1 in the amino acid sequence of p62 of a non-human animal. The serine of p62 of a non-human animal corresponding to the serine at position 38 from the C-terminus of SEQ ID NO: 1 can be identified by aligning the amino acid sequence of SEQ ID NO: 1 with the amino acid sequence of p62 of a non-human animal.

The amino acid sequence of p62 of a non-human animal can be obtained from known databases such as NCBI. For example, in the amino acid sequence of mouse p62 (NCBI Reference Sequence: NP_035148.1), the phosphorylation of serine at position 405 from the N-terminus and serine at position 38 from the C-terminus is promoted; in the amino acid sequence of rat p62 (NCBI Reference Sequence: NP_787037.2), the phosphorylation of serine at position 402 from the N-terminus and serine at position 38 from the C-terminus is promoted; in the amino acid sequence of bovine p62 (NCBI Reference Sequence: NP_788814.1), the phosphorylation of serine at position 403 from the N-terminus and serine at position 38 from the C-terminus is promoted; and in the amino acid sequence of monkey p62 (NCBI Reference Sequence: NP_001253287.1), the phosphorylation of serine at position 402 from the N-terminus and serine at position 38 from the C-terminus is promoted.

The promotion of the phosphorylation of serine at position 403 from the N-terminus of the amino acid sequence of human p62 shown in SEQ ID NO: 1 can be confirmed, for example, by extracting proteins from cells contacted with the compound represented by General Formula (I) or salt thereof and cells not contacted with the compound or salt thereof, and performing Western blotting using an antibody that specifically binds to p62 in which serine at position 403 is phosphorylated.

### [Composition for Promoting Degradation of Protein Aggregate]

As described above, the compound represented by General Formula (I) can promote the phosphorylation of serine at position 403 in the amino acid sequence of human p62 shown in SEQ ID NO: 1, and as a result, can activate the autophagy (aggrephagy) of the protein aggregate to promote the degradation of the protein aggregate (see FIG. 9). Accordingly, the present invention provides a composition for promoting the degradation of a protein aggregate, containing the compound represented by General Formula (I) or salt thereof. The composition for promoting the degradation of a protein aggregate of the present invention can be referred to as aggrephagy activator containing the compound represented by General Formula (I) or salt thereof.

The composition for promoting the degradation of a protein aggregate of the present invention can promote the degradation of a protein aggregate (ubiquitinated protein aggregate) modified with polyubiquitin and present in the cytoplasm. Examples of the ubiquitinated protein aggregate include a tau protein aggregate, an α-synuclein protein aggregate, a prion protein aggregate, and an intracytoplasmic TDP-43 aggregate.

The promotion of the degradation of the protein aggregate can be confirmed, for example, by observing cells that are brought into contact with the compound represented by General Formula (I) or salt thereof and cells that are not brought into contact with the compound or salt thereof using the tau aggregation cell line used in Examples described later.

### [Pharmaceutical Composition]

The composition for promoting the degradation of a protein aggregate of the present invention can be implemented as a pharmaceutical composition. That is, the present invention provides a pharmaceutical composition for prevention or treatment of a neurodegenerative disease associated with protein aggregate formation, containing the composition for promoting the degradation of a protein aggregate of the present invention. The pharmaceutical composition of the present invention can be referred to as pharmaceutical composition for prevention or treatment of a neurodegenerative disease associated with protein aggregate formation, containing the compound represented by General Formula (I) or salt thereof as an active ingredient.

Examples of the neurodegenerative disease associated with protein aggregate formation, which is a disease to which the pharmaceutical composition of the present invention is to be applied, include tauopathies such as Alzheimer's disease, progressive supranuclear palsy, corticobasal degeneration, and frontotemporal lobar degeneration, synucleinopathies such as Parkinson's disease, multiple system atrophy, dementia with Lewy bodies, REM sleep behavior disorder, and striatonigral degeneration, prion diseases such as Creutzfeldt-Jakob disease, and Gerstmann-Straussler-Scheinker disease, amyotrophic lateral sclerosis, Huntington's disease, spinocerebellar degeneration, and dentatorubropallidoluysian atrophy.

As the salt of the compound represented by General Formula (I), a pharmaceutically acceptable salt can be preferably used. The pharmaceutically acceptable salt is not particularly limited as long as it maintains the efficacy of the active ingredient and does not adversely affect the human body, and examples thereof include salts with acids such as acetic acid, propionic acid, butyric acid, formic acid, trifluoroacetic acid, maleic acid, tartaric acid, citric acid, stearic acid, succinic acid, ethylsuccinic acid, malonic acid, lactobionic acid, gluconic acid, glucoheptonic acid, benzoic acid, methanesulfonic acid, ethanesulfonic acid, 2-hydroxyethanesulfonic acid, benzenesulfonic acid, paratoluenesulfonic acid (tosic acid), lauryl sulfuric acid, malic acid, aspartic acid, glutamic acid, adipic acid, cysteine, N-acetylcysteine, hydrochloric acid, hydrobromic acid, phosphoric acid, sulfuric acid, hydroiodic acid, nicotinic acid, oxalic acid, picric acid, thiocyanic acid, undecanoic acid, acrylic acid polymers, and carboxyvinyl polymers, salts with inorganic bases such as lithium salts, sodium salts, potassium salts, and calcium salts, salts with organic amines such as morpholine and piperidine, and salts with amino acids.

The pharmaceutical compositions of the present invention can be formulated in accordance with routine means. Specifically, the pharmaceutical composition of the present invention can be formulated into oral preparations such as tablets, coated tablets, pills, powders, granules, capsules, solutions, suspensions, and emulsions; and parenteral preparations such as injections, infusions, suppositories, ointments, and patches. A blending proportion of a carrier or an additive may be appropriately set based on the range usually adopted in the pharmaceutical field. The carrier or additive that can be blended is not particularly limited, and examples thereof include various carriers such as water, physiological saline, other aqueous solvents, and aqueous or oily bases; and various additives such as excipients, binders, pH adjusters, disintegrants, absorption promoters, lubricants, diluents, thickeners, wetting agents, emulsifiers, preservatives, colorants, corrigents, and flavors.

Examples of an additive that can be mixed into a tablet, a capsule, and the like include binders such as gelatin, corn starch, tragacanth, and gum arabic, excipients such as crystalline cellulose, swelling agents such as corn starch, gelatin, and alginic acid, lubricants such as magnesium stearate, a sweetener such as sucrose, lactose, or saccharin, and a flavoring agent such as peppermint, oil of Gaultheria adenothrix, or cherry. In the case where the dispensing unit form is a capsule, the capsule can further contain a liquid carrier, such as an oil and/or fat, in addition to a material of the above type. A sterile composition for injection can be formulated according to normal pharmaceutical practice involving, for example, dissolving or suspending an active substance in a vehicle such as water for injection, a naturally occurring vegetable oil such as sesame oil or coconut oil, or the like. Examples of an aqueous liquid for an injection include physiological saline or an isotonic solution containing glucose and an additional adjuvant (e.g., D-sorbitol, D-mannitol, or sodium chloride), which may be used in combination with a suitable solubilizing agent, such as an alcohol (e.g., ethanol), a polyalcohol (e.g., propylene glycol or polyethylene glycol), or a nonionic surfactant (e.g., polysorbate 80 or HCO-50). Examples of an oily liquid used include olive oil, sesame oil or soybean oil, which may be used in combination with a solubilizing agent, such as benzyl benzoate or benzyl alcohol. In addition, it may also be blended with, for example, a buffer (e.g., a phosphate buffer or a sodium acetate buffer), a soothing agent (e.g., benzalkonium chloride or procaine hydrochloride), a stabilizer (e.g., human serum albumin or polyethylene glycol), a preservative (e.g., benzyl alcohol or phenol), and/or an antioxidant.

A content of the compound represented by General Formula (I) or salt thereof in the composition or in one preparation may be 0.001 mg to 1000 mg, or may be 0.01 mg to 100 mg. The composition or preparation thus obtained is safe and has low toxicity, and thus can be administered to, for example, humans and mammals (e.g., rats, mice, rabbits, sheep, pigs, cattle, cats, dogs, and monkeys).

A dose of the active ingredient of the pharmaceutical composition of the present invention is appropriately set in consideration of the purpose, the type of disease, the severity of disease, the age, body weight, sex, and medical history of the patient, the type of active ingredient, and/or the like. When an average human having a body weight of about from 65 to 70 kg is a target, the daily dose is preferably about from 0.02 mg to 5000 mg, and more preferably about from 0.1 mg to 200 mg. The total daily dose may be a single dose or divided doses.

The present invention includes the following inventions.

A method for preventing or treating a neurodegenerative disease associated with protein aggregate formation, including administering an effective amount of a compound represented by General Formula (I) or a salt thereof to a mammal.

A method for promoting the degradation of a protein aggregate, including administering an effective amount of a compound represented by General Formula (I) or a salt thereof to a mammal.

A method for promoting p62/SQSTM1 phosphorylation, including administering an effective amount of a compound represented by General Formula (I) or a salt thereof to a mammal.

A compound represented by General Formula (I) or a salt thereof for use in the prevention or treatment of a neurodegenerative disease associated with protein aggregate formation.

A compound represented by General Formula (I) or a salt thereof for use in promoting the degradation of a protein aggregate.

A compound represented by General Formula (I) or a salt thereof for use in promoting p62/SQSTM1 phosphorylation.

Use of a compound represented by General Formula (I) or a salt thereof for the production of a pharmaceutical for prevention or treatment of a neurodegenerative disease associated with protein aggregate formation.

Use of a compound represented by General Formula (I) or a salt thereof for the production of a degradation accelerator for a protein aggregate.

Use of a compound represented by General Formula (I) or a salt thereof for the production of a promoter for p62/SQSTM1 phosphorylation.

A compound represented by General Formula (I) or a salt thereof, a composition and a preparation containing the compound or the salt thereof, and methods for producing them.

A compound represented by General Formula (II) or a salt thereof, a composition and a preparation containing the compound or the salt thereof, and methods for producing them.

The present invention will be described in detail below by way of examples, but is not limited to these examples.

The present invention will be described in more detail below with reference to production examples, reference examples, test examples, and the like, but is not limited to these examples.

The compounds, reagents, and the like used in the production examples, examples, test examples, and the like used in the present invention are readily available from commercial sources, and can be used. For example, they can be purchased from reagent manufacturers such as Aldrich, FUJIFILM Wako Pure Chemical Corporation, Kanto Chemical Co., Inc., Nacalai Tesque, Inc., and Tokyo Chemical Industry Co., Ltd. Column chromatography was performed using silica gel (from 50 to 200 µm, Fuji Silysia Chemical Ltd.). One dimensional NMR spectra were measured using Varian 500PS, JEOL JNM-Al400, and Varian Gemini 300. Chemical shifts are described in ppm with the solvent resonance or TMS as internal standard. Multiplicities are indicated (s = singlet, d = doublet, t = triplet, dd = doublet of doublets, dt = doublet of triplets, m = multiplet, br = broad), etc.

Abbreviations used herein mean the following.
DMF: N,N-dimethylformamide
THF: tetrahydrofuran
Et₃N: triethylamine
iPr₂NEt: diisopropylethylamine
DBU: diazabicycloundecene
DMAP: 4-dimethylaminopyridine
Pd₂(dpa)₃: tris(dibenzylineacetone)dipalladium (0)
Xantophos: 4,5-bis(diphenylphosphino)-9,9-dimethylxanthene
S-phos: 2-dicyclohexylphosphino-2',6'-dimethoxy-1,1'-biphenyl
EDCI: 1-ethyl-3-(3-dimethylaminopropyl)carbodiimide hydrochloride
TFA: trifluoroacetic acid
BiotinNHS: 6aR-hexahydro-2-oxo-2,5-dioxo-1-pyrrolidinylester-1H-thieno[3aS,4d]imidazole-4S-pentane
LAH: lithium aluminum hydride

### [Production Method]

Compound 3 having the following structure can be produced by, for example, the method described in the following scheme. In Compound 3, preferably, X=Y is N=C and N=N, but is not limited thereto. 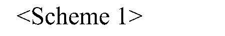 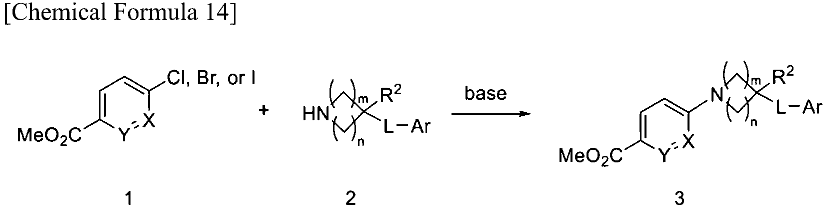 where each symbol has the same meaning as described above.

Compound 3 can be produced by reacting Compound 1 and Compound 2 described above in the presence of a base in a solvent that does not adversely affect the reaction. Examples of the solvent that does not adversely affect the reaction include, but are not limited to, methanol, ethanol, acetonitrile, DMF, THF, and 1,4-dioxane. Examples of the base include, but are not limited to, Et₃N, iPr₂NEt, DBU, alkali metal (Li, Na, K, Cs) carbonates, and hydroxides. The amount of the base to be used is usually from about 0.5 molar equivalents to about 5.0 molar equivalents, preferably from about 1 molar equivalent to about 2 molar equivalents, relative to Compound 1, but is not limited thereto. The reaction temperature is usually from room temperature to about 150°C, preferably from room temperature to about 120°C, but is not limited thereto. The room temperature herein is usually from about 10 to about 30°C, but is not limited thereto. The reaction time is usually from about 5 minutes to about 48 hours, preferably from about 10 minutes to about 24 hours, but is not limited thereto.

### [Reference Production Example 1] Production of methyl 6-(4-(2-(trifluoromethyl)phenoxy)piperidin-1-yl)pyridazine-3-carboxylate

Methyl 6-chloropyridazine-3-carboxylate (1.5 g) and 4-(2-(trifluoromethyl)phenoxy)piperidine (1.1 g) were dissolved in acetonitrile (80 mL), iPr₂NEt (1.3 mL) was added thereto, and the mixture was stirred at 120°C for 12 hours to react. The solution after the reaction was diluted with water, and then extracted with ethyl acetate. The solution after the extraction was washed with water and saturated brine in this order, and then dried over magnesium sulfate. Furthermore, the solvent was distilled off under reduced pressure, and then the residue was purified by column chromatography (eluent: ethyl acetate:hexane = 1:2) to give the target product (1.4 g, yield: 63%).

¹HNMR(500MHz, CDCl₃)δ7.89(d, J = 9.8Hz, 1H), 7.60(d, J = 8.1Hz, 1H), 7.50(t, 8.1Hz, 1H), 7.04-7.02(m, 2H), 6.91(d, J = 9.8Hz, 1H), 4.84-4.81(m, 1H), 4.12-4.08(m, 2H), 4.00(s, 3H), 3.86-3.81(m, 2H), 2.08-1.99(m, 4H).

A scheme generalized from Reference Production Example 1 is, for example, as follows. 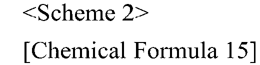 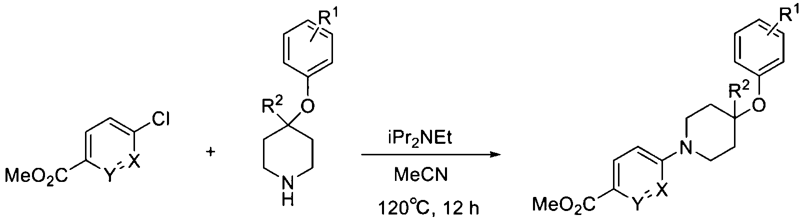 where each symbol has the same meaning as described above.

### [Reference Production Example 2] Production of methyl 6-(4-(2-chloro-5-fluorophenoxy)piperidin-1-yl)pyridazine-3-carboxylate

4-(2-Chloro-5-fluorophenoxy)piperidine (1.1 g) was used to perform reaction treatment according to the method described in Reference Production Example 1, thereby giving the target product (2.2 g, yield: 60%).

¹HNMR(500MHz, CDCl₃)δ7.90(d, J = 9.5Hz, 2H), 7.34(dd, J = 6.1, 8.8Hz, 1H), 6.92(d, J = 9.5Hz, 2H), 6.73(dd, J = 2.7, 10.1Hz, 1H), 6.70-6.66(m, 1H), 4.68(quint., 1H), 4.12-3.93(m, 4H), 4.00(s, 3H), 2.06-2.00(m, 4H).

### [Reference Production Example 3] Production of methyl 6-(4-cyano-4-(4-(trifluoromethyl)phenyl)piperidin-1-yl)pyridazine-3-carboxylate

Methyl 6-chloropyridazine-3-carboxylate (438 mg), 4-(4-(trifluoromethyl)phenyl)piperidine-4-carbonitrile (645 mg), iPr₂NEt (530 µL) and acetonitrile (50 mL) were used to perform reaction treatment according to the method described in Reference Production Example 1, thereby giving the target product (547 mg, yield: 56%).

¹HNMR(500MHz, CDCl₃)δ7.97(d, J = 9.6Hz, 2H), 7.70(d, J = 8.8Hz, 2H), 7.63(d, J = 8.6Hz, 2H), 7.00(d, J = 9.8Hz, 1H), 4.82-4.79(m, 2H), 4.03(s, 3H), 3.59-3.53(m, 2H), 2.30(d, J = 13.7Hz, 2H), 2.14(dt, J = 4.2, 13.2Hz, 2H).

### [Reference Production Example 4] Production of methyl 6-(4-(2-chloro-5-fluorophenoxy)-4-cyanoperidin-1-yl)pyridazine-3-carboxylate

Methyl 6-chloropyridazine-3-carboxylate (300 mg), 4-(2-chloro-5-fluorophenoxy)piperidine-4-carbonitrile (440 mg), iPr₂NEt (362 µL) and DMF (10 mL) were used to perform reaction treatment according to the method described in Reference Production Example 1, thereby giving the target product (551 mg, yield: 81%).

### [Reference Production Example 5] Production of methyl 6-(4-(2-chloro-5-fluorophenoxy)piperidin-1-yl)nicotinate

Methyl 6-chloronicotinate (340 mg), 4-(2-chloro-5-fluorophenoxy)piperidine (508 mg), iPr₂NEt (420 µL) and DMF (15 mL) were used to perform reaction treatment according to the method described in Reference Production Example 1, thereby giving the target product (634 mg, yield: 87%).

¹HNMR(500MHz, CDCl₃)δ8.81(d, J = 2.2Hz, 1H), 8.03(dd, J = 2.2, 9.1Hz, 1H), 7.33(dd, J = 6.1, 8.8Hz, 1H), 6.74(dd, J = 3.0, 10.3Hz, 1H), 6.69-6.64(m, 2H), 4.65-4.61(m, 1H), 3.94-3.80(m, 7H), 2.04-1.93(m, 4H).

### [Reference Production Example 6] Production of methyl 6-(4-(2-chlorophenoxy)piperidin-1-yl)pyridazine-3-carboxylate

Methyl 6-chloropyridazine-3-carboxylate (693 mg), 4-(2-chlorophenoxy)piperidine (850 mg), iPr₂NEt (1.1 mL) and acetonitrile (60 mL) were used to perform reaction treatment according to the method described in Reference Production Example 1, thereby giving the target product (1.0 g, yield: 72%).

¹HNMR(500MHz, CDCl₃)δ7.89(d, J = 9.6Hz, 1H), 7.40(dd, J = 1.7, 8.1Hz, 1H), 7.28-7.21(m, 1H), 7.01(d, J = 8.1Hz, 1H), 6.95(t, J = 7.8Hz, 1H), 6.92(d, J = 9.8Hz, 1H), 4.71(quint., J = 4.6Hz, 1H), 4.07-3.98(m, 7H), 2.05-2.02(m, 4H).

### [Reference Production Example 7] Production of methyl 6-(4-(2,6-dichlorophenoxy)piperidin-1-yl)pyridazine-3-carboxylate

Methyl 6-chloropyridazine-3-carboxylate (596 mg), 4-(2,6-dichlorophenoxy)piperidine (850 mg), iPr₂NEt (733 µL) and acetonitrile (50 mL) were used to perform reaction treatment according to the method described in Reference Production Example 1, thereby giving the target product (750 mg, yield: 56%).

¹HNMR(500MHz, CDCl₃)δ7.90(d, J = 9.8Hz, 1H), 7.33(d, J = 8.1Hz, 2H), 7.01(t, J = 7.8Hz, 1H), 6.92(d, J = 9.5Hz, 1H), 4.57(quint., J = 5.5Hz, 1H), 4.33-4.28(m, 2H), 4.01(s, 3H), 3.69-3.64(m, 2H), 2.09-2.06(m, 4H).

### [Reference Production Example 8] Production of methyl 4-(4-(2-chloro-5-fluorophenoxy)piperidin-1-yl)benzoate

Methyl 4-bromobenzoate (1.0 g) and 4-(2-chloro-5-fluorophenoxy)piperidine (1.0 g) were dissolved in 1,4-dioxane (25 mL), Pd₂(dpa)₃ (39 mg), Xantphos (62 mg), and cesium carbonate (2.2 g) were added thereto, and the mixture was stirred at 100°C for 12 hours. After the reaction, the reaction liquid was diluted with water and then extracted with ethyl acetate. The extraction liquid was washed with water and saturated brine in this order, and dried over magnesium sulfate. The solvent was distilled off under reduced pressure, and then the residue was purified by column chromatography (eluent: ethyl acetate:hexane = 1:4) to give the target product (820 mg, yield: 43%).

¹HNMR(500MHz, CDCl₃)δ7.93(d, J = 9.3Hz, 2H), 7.33(dd, J = 2.7, 8.8Hz, 1H), 6.90(d, J = 9.1Hz, 2H), 6.72(dd, J = 3.0, 10.3Hz, 1H), 6.66(ddd, J = 2.7, 7.8, 8.8Hz, 1H), 4.60-4.56(m, 1H), 3.88(s, 3H), 3.67-3.62(m, 2H), 3.43-3.39(m, 2H), 2.10-1.97(m, 4H).

A scheme generalized from Reference Production Example 8 is as follows. 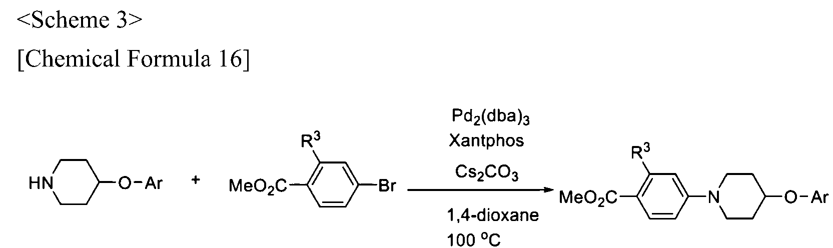 where each symbol has the same meaning as described above.

### [Reference Production Example 9] Production of methyl 4-(4-(2-chlorophenoxy)piperidin-1-yl)benzoate

Methyl 4-bromobenzoate (932 mg), 4-(2-chlorophenoxy)piperidine (850 mg), Pd₂(dpa)₃ (37 mg), Xantphos (58 mg) and cesium carbonate (2.0 g) were used to perform reaction treatment according to the method described in Reference Production Example 8, thereby giving the target product (762 mg, yield: 55%).

^{I}HNMR(500MHz, CDCl₃)δ7.93(d, J = 9.1Hz, 2H), 7.39(dd, J = 1.7, 7.8Hz, 1H), 7.24-7.20(m, 1H), 7.00(dd, J = 1.0, 8.3Hz, 1H), 6.94(dt, J = 1.3, 7.9Hz, 1H), 6.90(d, J = 7.1Hz, 2H), 4.63-4.59(m, 1H), 3.88(s, 3H), 3.69-3.64(m, 2H), 3.41-3.37(m, 2H), 2.09-1.98(m, 4H).

### [Reference Production Example 10] Production of methyl 4-(4-(2,6-dichlorophenoxy)piperidin-1-yl)benzoate

Methyl 4-bromobenzoate (932 mg), 4-(2,6-dichlorophenoxy)piperidine (850 mg), Pd₂(dpa)₃ (37 mg), Xantphos (58 mg) and cesium carbonate (1.7 g) were used to perform reaction treatment according to the method described in Reference Production Example 8, thereby giving the target product (734 mg, yield: 58%).

¹HNMR(500MHz, CDCl₃)δ7.92(d, J = 9.3Hz, 1H), 7.32(d, J = 8.1Hz, 1H), 7.00(t, J = 8.1Hz, 1H), 6.89(d, J = 9.1Hz, 2H), 4.47(quint., J = 6.4Hz, 1H), 3.87(s, 3H), 3.87-3.82(m, 2H), 3.21-3.16(m, 2H), 2.09-2.04(m, 4H).

Hereinafter, a scheme generalized from production examples of example compounds of the present invention is shown below. 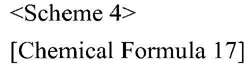 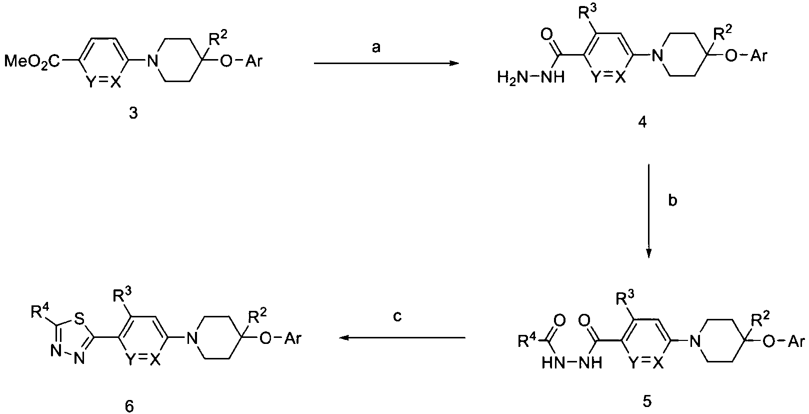 where each symbol has the same meaning as described above.

Steps (a), (b) and (c) in Scheme 4 above are described in detail below.

Step (a): Compound (4) can be produced by reacting Compound (3) with hydrazine monohydrate in a solvent that does not adversely affect the reaction. Examples of the solvent that does not adversely affect the reaction include methanol and ethanol, but are not limited thereto. The amount of hydrazine monohydrate to be used is usually from about 1 molar equivalent to about 50 molar equivalents, preferably from about 10 molar equivalents to about 50 molar equivalents, relative to Compound (4), but is not limited thereto. The reaction temperature is usually from room temperature to about 150°C, preferably from room temperature to about 90°C, but is not limited thereto. The reaction time is usually from about 5 minutes to about 48 hours, preferably from about 10 minutes to about 24 hours, but is not limited thereto.

Step (b): Compound (5) can be produced by reacting Compound (4) with a carboxylic acid halide in the presence of a base in a solvent that does not adversely affect the reaction. Examples of the solvent that does not adversely affect the reaction include dichloromethane and diethyl ether, but are not limited thereto. Examples of the carboxylic acid halide include, but are not limited to, acetyl chloride, acetoxyacetyl chloride, and benzoyl chloride. The amount of the carboxylic acid halide to be used is usually from 0.5 molar equivalents to 1.5 molar equivalents, preferably from 0.8 molar equivalents to 1.2 molar equivalents, but is not limited thereto. Examples of the base include, but are not limited to, Et₃N and iPr₂NEt. The amount of the base to be used is usually from about 0.5 molar equivalents to about 10 molar equivalents, preferably from about 0.8 molar equivalents to about 2 molar equivalents, relative to Compound (4), but is not limited thereto. The reaction temperature is usually, but not limited to, from about -78°C to about 150°C, preferably from about -20°C to about 80°C. The reaction time is usually from about 5 minutes to about 48 hours, preferably from about 10 minutes to about 24 hours, but is not limited thereto.

Step (c): Compound (6) can be produced by reacting Compound (5) with a sulfurizing agent under microwave irradiation in a solvent that does not adversely affect the reaction. Examples of the solvent that does not adversely affect the reaction include, but are not limited to, THF and 1,4-dioxane. Examples of the sulfurizing agent include, but are not limited to, phosphorus pentasulfide and Lawesson's reagents. The amount of the sulfurizing agent to be used is usually from about 0.5 molar equivalents to about 10 molar equivalents, preferably from about 0.8 molar equivalents to about 5 molar equivalents, relative to Compound (5), but is not limited thereto. The reaction temperature is usually from room temperature to about 150°C, preferably from room temperature to about 120°C, but is not limited thereto. The reaction time is usually from about 1 minute to about 1 hour, preferably from about 5 minutes to about 20 minutes, but is not limited thereto.

### [Example 1] Production of 2-(6-(4-(2-(trifluoromethyl)phenoxy)piperidin-1-yl)pyridazine-3-yl)-5-methyl-1,3,4-thiadiazole (Compound No.: NUT-9)

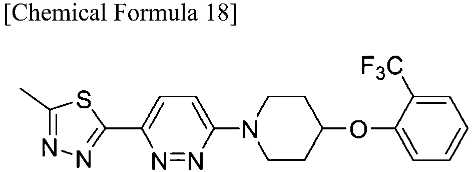

### [Step (a) of Example 1] Production of 6-(4-(2-(trifluoromethyl)phenoxy)piperidin-1-yl)pyridazine-3-carbohydrazide

Methyl 6-(4-(2-(trifluoromethyl)phenoxy)piperidin-1-yl)pyridazine-3-carboxylate (572 mg) produced in Reference Production Example 1 was dissolved in ethanol (8 mL), hydrazine monohydrate (3.0 mL) was added thereto, and the mixture was stirred at 80°C for 12 hours. After the solvent was distilled off under reduced pressure from the reaction liquid, the residue was diluted with water and then extracted with ethyl acetate. The extraction liquid was washed with saturated brine, dried over magnesium sulfate, and used as a crude product (570 mg, crude yield: 99%) in the next reaction.

### [Step (b) of Example 1] Production of N'-acetyl-6-(4-(2-(trifluoromethyl)phenoxy)piperidin-1-yl)pyridazine-3-carbohydrazide

The crude product of 6-(4-(2-(trifluoromethyl)phenoxy)piperidin-1-yl)pyridazine-3-carbohydrazide (570 mg) obtained in the above step (a) was dissolved in dichloromethane (10 mL), the reaction solution was cooled to 0°C, iPr₂NEt (522 µL) and acetyl chloride (160 µL) were added thereto, and the mixture was stirred at room temperature for 10 hours. After the reaction, the reaction liquid was diluted with water and then extracted with ethyl acetate. The extraction liquid was washed with saturated brine, and dried over magnesium sulfate. The solvent was distilled off under reduced pressure, and then the residue was purified by column chromatography (eluent: ethyl acetate:methanol = 95:5) to give the target product (112 mg, yield: 18%).

¹HNMR(500MHz, CDCl₃)δ9.86(br.s, NH), 8.13-8.06(m, NH), 7.93(d, J = 9.5Hz, 1H), 7.61(d, J = 7.4Hz, 1H), 7.51(t, J = 7.6Hz, 1H), 7.05-7.02(m, 2H), 7.99(d, J = 9.6Hz, 1H), 4.87-4.81(m, 1H), 4.14-4.07(m, 2H), 3.86-3.80(m, 2H), 2.14(s, 3H), 2.08-2.02(m, 4H).

### [Step (c) of Example 1] Production of 2-(6-(4-(2-(trifluoromethyl)phenoxy)piperidin-1-yl)pyridazine-3-yl)-5-methyl-1,3,4-thiadiazole

N'-acetyl-6-(4-(2-(trifluoromethyl)phenoxy)piperidin-1-yl)pyridazine-3-carbohydrazide (112 mg) obtained in the above step (b) was dissolved in THF (4 mL), phosphorus pentasulfide (124 mg) was added thereto, and the mixture was irradiated with microwaves at 150°C for 20 minutes. After the reaction, the solvent was distilled off under reduced pressure from the reaction liquid, and then the residue was purified by column chromatography (eluent: ethyl acetate:hexane = 1:1) to give the target product (43 mg, yield: 40%).

¹HNMR(500MHz, CDCl₃)δ8.15(d, J = 9.8Hz, 1H), 7.60(d, J = 7.8Hz, 1H), 7.49(t, J = 7.3Hz, 1H), 7.05-7.01(m, 3H), 4.84-4.81(m, 1H), 4.08-4.03(m, 2H), 3.86-3.81(m, 2H), 2.81(s, 3H), 2.09-2.02(m, 4H).

### [Example 2] Production of 2-(6-(4-(2-chloro-5-fluorophenoxy)piperidin-1-yl)pyridazine-3-yl)-5-methyl-1,3,4-thiadiazole (Compound No.: NUT-10)

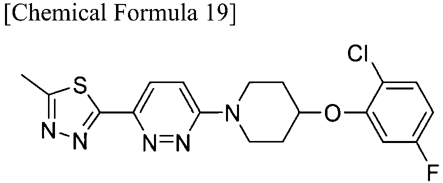

### [Step (a) of Example 2] Production of 6-(4-(2-chloro-5-fluorophenoxy)piperidin-1-yl)pyridazine-3-carbohydrazide

Methyl 6-(4-(2-chloro-5-fluorophenoxy)piperidin-1-yl)pyridazine-3-carboxylate (549 mg) produced in Reference Production Example 2 was used to perform reaction treatment according to the method described in step (a) of Example 1, thereby giving the target product as a crude product (550 mg, crude yield: 99%).

### [Step (b) of Example 2] Production of N'-acetyl-6-(4-(2-chloro-5-fluorophenoxy)piperidin-1-yl)pyridazine-3-carbohydrazide

The crude product of 6-(4-(2-chloro-5-fluorophenoxy)piperidin-1-yl)pyridazine-3-carbohydrazide (550 mg) obtained in the above step (a), iPr₂NEt (522 µL) and acetyl chloride (160 µL) were used to perform reaction treatment according to the method described in step (b) of Example 1, thereby giving the target product (123 mg, yield: 20%).

¹HNMR(300MHz, CDCl₃)δ9.85(br.s, NH), 8.03(br.s, NH), 7.94(d, J = 9.4Hz, 1H), 7.01(d, J = 9.4Hz, 1H), 6.76-6.65(m, 2H), 4.72-4.65(m, 1H), 4.03-3.89(m, 4H), 2.14(s, 3H), 2.07-2.02(m, 4H).

### [Step (c) of Example 2] Production of 2-(6-(4-(2-chloro-5-fluorophenoxy)piperidin-1-yl)pyridazine-3-yl)-5-methyl-1,3,4-thiadiazole

N'-Acetyl-6-(4-(2-chloro-5-fluorophenoxy)piperidin-1-yl)pyridazine-3-carbohydrazide (123 mg) obtained in the above step (b) and phosphorus pentasulfide (143 mg) were used to perform reaction treatment according to the method described in step (c) of Example 1, thereby giving the target product (47 mg, yield: 40%).

¹HNMR(500MHz, CDCl₃)δ8.17(d, J = 9.5Hz, 1H), 7.34(dd, J = 6.1, 8.8Hz, 1H), 7.06(d, J = 9.6Hz, 1H), 6.74(dd, J = 2.7, 10Hz, 1H), 6.68(dt, J = 2.7, 8.6Hz, 1H), 4.69(quint.J = 4.2Hz, 1H), 3.98-3.96(m, 4H), 2.83(s, 3H), 2.07-2.05(m, 4H).

### [Example 3] Production of (5-(6-(4-(2-(trifluoromethyl)phenoxy)piperidin-1-yl)pyridazine-3-yl)-1,3,4-thiadiazol-2-yl)methyl acetate (Compound No.: NUT-13)

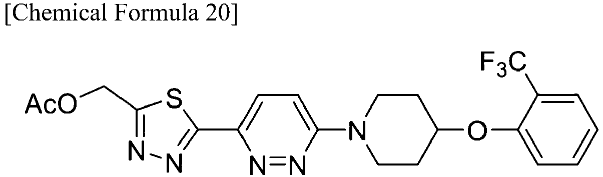

### [Step (1) of Example 3] Production of 2-(2-(6-(4-(2-(trifluoromethyl)phenoxy)piperidin-1-yl)pyridazine-3-carbonyl)hydrazinyl)2-oxoethyl acetate

6-(4-(2-(Trifluoromethyl)phenoxy) piperidin-1-yl)pyridazine-3-carbohydrazide (572 mg) obtained in step (a) of Example 1, iPr₂NEt (452 µL), and acetoxyacetyl chloride (244 µL) were used to perform reaction treatment according to the method described in step (b) of Example 1, thereby giving the target product (385 mg, yield: 63%).

¹HNMR(500MHz, CDCl₃)δ9.84(br.s, NH), 8.61(br.s, NH), 7.94(d, J = 9.6Hz, 1H), 7.61(d, J = 7.6Hz, 1H), 7.51(d, J = 7.4Hz, 1H), 7.05-7.00(m, 2H), 4.87-4.83(m, 1H), 4.73(s, 2H), 4.12-4.08(m, 2H), 3.86-3.82(m, 2H), 2.22(s, 3H), 2.11-2.08(m, 4H).

### [Step (2) of Example 3] Production of (5-(6-(4-(2-(trifluoromethyl)phenoxy)piperidin-1-yl)pyridazine-3-yl)-1,3,4-thiadiazol-2-yl)methyl acetate

2-(2-(6-(4-(2-(Trifluoromethyl)phenoxy)piperidin-1-yl)pyridazine-3-carbonyl)hydrazinyl)2-oxoethyl acetate (340 mg) obtained in the above step (1) and phosphorus pentasulfide (345 mg) were used to perform reaction treatment according to the method described in step (c) of Example 1, thereby giving the target product (155 mg, yield: 46%).

¹HNMR(500MHz, CDCl₃)δ8.18(d, J = 9.6Hz, 1H), 7.62(d, J = 7.8Hz, 1H), 7.51(d, J = 7.6Hz, 1H), 7.64-7.03(m, 2H), 5.53(s, 2H), 4.86-4.83(m, 1H), 4.09(dt, J = 4.4, 13.7Hz, 2H), 3.88-3.82(m, 2H), 2.18(s, 3H), 2.11-2.02(m, 4H).

### [Example 4] Production of (5-(6-(4-(2-chloro-5-fluorophenoxy)piperidin-1-yl)pyridazine-3-yl)-1,3,4-thiadiazol-2-yl)methyl acetate (Compound No.: NUT-14 (ADI-14))

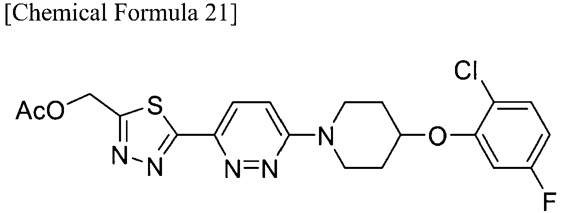

### [Step (1) of Example 4] Production of 2-(2-(6-(4-(2-chloro-5-fluorophenoxy)piperidin-1-yl)pyridazine-3-carbonyl)hydrazinyl)2-oxoethyl acetate

6-(4-(2-Chloro-5-fluorophenoxy)piperidin-1-yl)pyridazine-3-carbohydrazide (250 mg) obtained in step (a) of Example 2, iPr₂NEt (238 µL) and acetoxyacetyl chloride (111 µL) were used to perform reaction treatment according to the method described in step (b) of Example 1, thereby giving the target product (137 mg, yield: 43%).

¹HNMR(500MHz, CDCl₃)δ9.84(br.s, NH), 8.58(br.s, NH), 7.95(d, J = 9.6Hz, 1H), 7.35(dd, J = 6.1, 8.8Hz, 1H), 7.01(d, J = 9.5Hz, 1H), 7.64(dd, J = 2.7, 10.0Hz, 1H), 6.71-6.67(m, 1H), 4.76(s, 2H), 4.70-4.69(m, 1H), 4.03-3.92(m, 4H), 2.22(s, 3H), 2.07-2.04(m, 4H).

### [Step (2) of Example 4] Production of (5-(6-(4-(2-chloro-5-fluorophenoxy)piperidin-1-yl)pyridazine-3-yl)-1,3,4-thiadiazol-2-yl)methyl acetate

2-(2-(6-(4-(2-Chloro-5-fluorophenoxy)piperidin-1-yl)pyridazine-3-carbonyl)hydrazinyl)2-oxoethyl acetate (137 mg) obtained in the above step (1) and phosphorus pentasulfide (143 mg) were used to perform reaction treatment according to the method described in step (c) of Example 1, thereby giving the target product (73 mg, yield: 54%).

¹HNMR(500MHz, CDCl₃)δ8.19(d, J = 9.5Hz, 1H), 7.35(dd, J = 6.2, 8.8Hz, 1H), 7.06(d, J = 9.6Hz, 1H), 6.74(dd, J = 2.7, 10.0Hz, 1H), 6.70-6.67(m, 1H), 5.53(s, 2H), 4.69(quint., 4.4Hz, 1H), 4.02-3.96(m, 4H), 2.19(s, 3H), 2.06-2.05(m, 4H).

### [Example 5] Production of 5-(6-(4-(2-chloro-5-fluorophenoxy)piperidin-1-yl)pyridazine-3-yl)-1,3,4-oxadiazol-2-yl)methyl acetate (Compound No.: NUT-19)

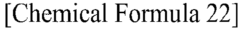

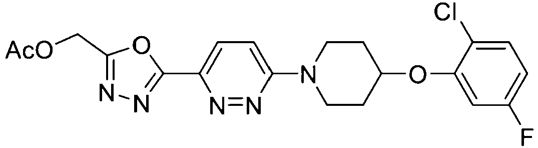

Phosphoryl chloride (4 mL) was added to 2-(2-(6-(4-(2-chloro-5-fluorophenoxy)piperidin-1-yl)pyridazine-3-carbonyl)hydrazinyl)2-oxoethyl acetate (230 mg) obtained in step (1) of Example 4, and the mixture was stirred at 100°C for 6 hours. Ice was added to the reaction liquid, and the mixture was diluted with an aqueous potassium carbonate solution and then extracted with ethyl acetate. The extraction liquid was washed with saturated brine, and dried over magnesium sulfate. The solvent was distilled off under reduced pressure, and then the residue was purified by column chromatography (eluent: ethyl acetate:methanol = 95:5) to give the target product (92 mg, yield: 42%).

¹HNMR(500MHz, CDCl₃)δ8.03(d, J = 9.5Hz, 1H), 7.32(dd, J = 5.9, 8.8Hz, 1H), 7.03(d, J = 9.8Hz, 1H), 6.72(dd, J = 3.0, 10.3Hz, 1H), 6.70-6.67(m, 1H), 5.36(s, 2H), 4.69-4.67(m, 1H), 4.04-3.92(m, 4H), 2.16(s, 3H), 2.06-2.03(m, 4H).

A scheme generalized from Example 5 is, for example, as follows. 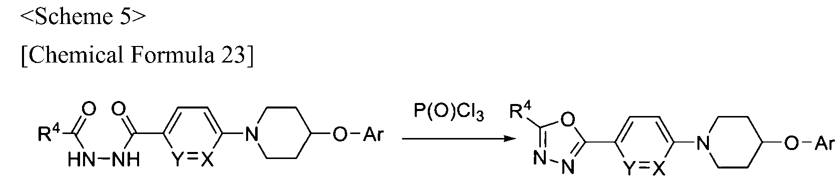 where each symbol has the same meaning as described above.

### [Example 6] Production of (5-(6-(4-cyano-4-(trifluoromethyl)phenyl)piperidin-1-yl)pyridazine-3-yl)-1,3,4-thiadiazol-2-yl)methyl acetate (Compound No.: NUT-21)

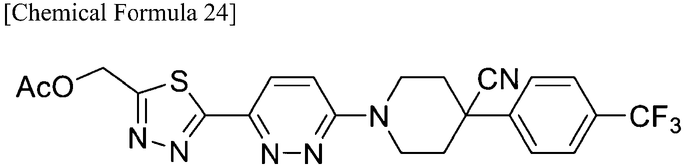

### [Step (a) of Example 6] Production of 6-(4-cyano-4-(4-(trifluoromethyl)phenyl)piperidin-1-yl)pyridazine-3-carbohydrazide

Methyl 6-(4-cyano-4-(4-(trifluoromethyl)phenyl)piperidin-1-yl)pyridazine-3-carboxylate (547 mg) obtained in Reference Production Example 3 was used to perform reaction treatment according to the method described in step (a) of Example 1, thereby giving the target product as a crude product (545 mg, crude yield: 99%).

### [Step (b) of Example 6] Production of 2-(2-(6-(4-cyano-4-(4-(trifluoromethyl)phenyl)piperidin-1-yl)pyridazine-3-carbonyl)hydrazinyl)-2-oxoethyl acetate

The crude product of 6-(4-cyano-4-(4-(trifluoromethyl)phenyl)piperidin-1-yl)pyridazine-3-carbohydrazide (545 mg) produced in the above step (a), iPr₂NEt (489 µL) and acetoxyacetyl chloride (228 µL) were used to perform reaction treatment according to the method described in step (b) of Example 1, thereby giving the target product (56 mg, yield: 8%).

¹HNMR(500MHz, CDCl₃)δ9.58(br.d, NH), 8.00(d, J = 9.6Hz, 1H), 7.71(d, J = 8.3Hz, 2H), 7.64(d, J = 8.6Hz, 2H), 7.08(d, J = 9.6Hz, 1H), 5.14(br.s, 2H), 5.04(br.s, NH), 4.89(d, J = 14.0Hz, 2H), 3.60(t, J = 13.5Hz, 2H), 2.32(d, J = 13.2Hz, 2H), 2.21-2.14(m, 5H).

### [Step (c) of Example 6] Production of (5-(6-(4-cyano-4-(trifluoromethyl)phenyl)piperidin-1-yl)pyridazine-3-yl)-1,3,4-thiadiazol-2-yl)methyl acetate

2-(2-(6-(4-Cyano-4-(4-(trifluoromethyl)phenyl)piperidin-1-yl)pyridazine-3-carbonyl)hydrazinyl)-2-oxoethyl acetate (56 mg) produced in the above step (b) and phosphorus pentasulfide (56 mg) were used to perform reaction treatment according to the method described in step (c) of Example 1, thereby giving the target product (22 mg, yield: 40%).

¹HNMR(500MHz, CDCl₃)δ8.24(d, J = 9.6Hz, 1H), 7.70(d, J = 8.3Hz, 2H), 7.63(d, J = 8.3Hz, 2H), 7.13(d, J = 9.6Hz, 1H), 5.54(s, 2H), 4.77(d, J = 14.2Hz, 2H), 3.60-3.54(m, 2H), 2.31(d, J = 13.7Hz, 2H), 2.19-2.13(m, 5H).

### [Example 7] Production of (5-(6-(4-(2-chloro-5-fluorophenoxy)-4-cyanopiperidin-1-yl)pyridazine-3-yl)-1,3,4-thiadiazol-2-yl)methyl acetate (Compound No.: NUT-25), and

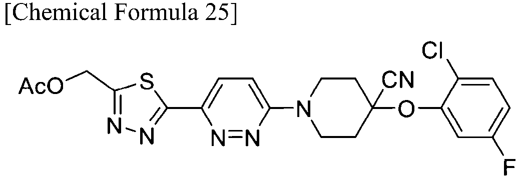

### [By-Product of Example 7] Production of (5-(6-(4-carbamothioyl-4-(2-chloro-5-fluorophenoxy)piperidin-1-yl)pyridazine-3-yl)-1,3,4-thiadiazol-2-yl)methyl acetate (Compound No.: NUT-26)

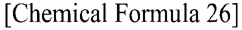

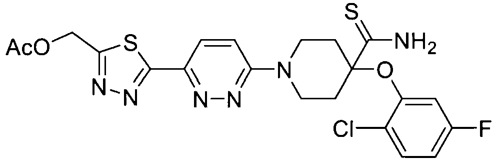

### [Step (a) of Example 7] Production of 6-(4-(2-chloro-5-fluorophenoxy)-4-cyanopiperidin-1-yl)pyridazine-3-carbohydrazide

Methyl 6-(4-(2-chloro-5-fluorophenoxy)-4-cyanoperidin-1-yl)pyridazine-3-carboxylate (508 mg) produced in Reference Production Example 4 was used to perform reaction treatment according to the method described in step (a) of Example 1, thereby giving the target product as a crude product (300 mg, crude yield: 59%).

### [Step (b) of Example 7] Production of 2-(2-(6-(4-(2-chloro-5-fluorophenoxy)-4-cyanopiperidin-1-yl)pyridazine-3-carbonyl)hydrazinyl)-2-oxoethyl acetate

A portion (195 mg) of the crude product of 6-(4-(2-chloro-5-fluorophenoxy)-4-cyanopiperidin-1-yl)pyridazine-3-carbohydrazide produced in the above step (a), iPr₂NEt (100 µL), and acetoxyacetyl chloride (54 µL) were used to perform reaction treatment according to the method described in step (b) of Example 1, thereby giving the target product (158 mg, yield: 64%).

¹HNMR(500MHz, CDCl₃)δ9.84(br.s, NH), 8.60(br.s, NH), 8.02(d, J = 9.5Hz, 1H), 7.42(dd, J = 5.9, 8.8Hz, 1H), 7.34(dd, J = 3.0, 9.3Hz, 1H), 7.04(d, J = 9.6Hz, 1H), 6.90(ddd, J = 2.7, 7.6, 8.8Hz, 1H), 4.76(s, 2H), 4.24-4.19(m, 2H), 3.84-3.80(m, 2H), 2.38-2.33(m, 4H), 2.23(s, 3H).

### [Step (c) of Example 7] Production of (5-(6-(4-(2-chloro-5-fluorophenoxy)-4-cyanoperidin-1-yl)pyridazine-3-yl)-1,3,4-thiadiazol-2-yl)methyl acetate

2-(2-(6-(4-(2-Chloro-5-fluorophenoxy)-4-cyanopiperidin-1-yl)pyridazine-3-carbonyl)hydrazinyl)-2-oxoethyl acetate (157 mg) produced in the above step (b) and phosphorus pentasulfide (156 mg) were used to perform reaction treatment according to the method described in step (c) of Example 1, thereby giving the target product (41 mg, yield: 26%).

¹HNMR(500MHz, CDCl₃)δ8.24(d, J = 9.6Hz, 1H), 7.41(dd, J = 5.9, 8.8Hz, 1H), 7.33(dd, J = 2.7, 9.3Hz, 1H), 7.09(d, J = 9.8Hz, 1H), 6.89(ddd, J = 2.9, 7.6, 9.0Hz, 1H), 5.53(s, 2H), 4.25-4.20(m, 2H), 3.84-3.79(m, 2H), 2.40-2.33(m, 4H), 2.18(s, 3H).

In addition, as a by-product of the reaction of the above step (c), (5-(6-(4-carbamothioyl-4-(2-chloro-5-fluorophenoxy)piperidin-1-yl)pyridazine-3-yl)-1,3,4-thiadiazol-2-yl)methyl acetate (20 mg, 12% yield) was also obtained.

¹HNMR(500MHz, CDCl₃)δ8.17(d, J = 9.8Hz, 1H), 7.88(br.d, NH), 7.11(br.d, NH), 7.38(dd, J = 5.6, 8.8Hz, 1H), 7.01(d, J = 9.8Hz, 1H), 6.79-6.75(m, 1H), 6.72(dd, J = 2.7, 9.5Hz, 1H), 5.52(s, 2H), 4.42(d, J = 13.5Hz, 2H), 3.39(t, J = 13.5Hz, 2H), 2.68(dt, J = 4.7, 14.2Hz, 2H), 2.34(d, J = 13.0Hz, 2H), 2.18(s, 3H).

### [Example 8] (5-(6-(4-(2-Chloro-5-fluorophenoxy)piperidin-1-yl)pyridin-3-yl)-1,3,4-thiadiazol-2-yl)methyl acetate (Compound No.: NUT-27)

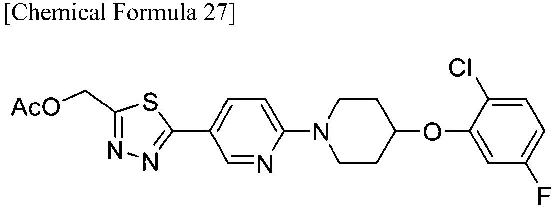

### [Step (a) of Example 8] Production of 6-(4-cyano-(2-chloro-5-fluorophenyl)piperidin-1-yl)nicotinohydrazide

Methyl 6-(4-(2-chloro-5-fluorophenoxy)piperidin-1-yl)nicotinate (547 mg) produced in Reference Production Example 5 was used to perform reaction treatment according to the method described in step (a) of Example 1, thereby giving the target product as a crude product (85 mg, crude yield: 16%).

### [Step (b) of Example 8] Production of 2-(2-(6-(4-(2-chloro-5-fluorophenyl)piperidin-1-yl)nicotinoyl)hydrazinyl-2-oxoethyl acetate

The crude product of 6-(4-cyano-(2-chloro-5-fluorophenyl)piperidin-1-yl)nicotinohydrazide (85 mg) produced in the above step (a), iPr₂NEt (49 µL) and acetoxyacetyl chloride (258 µL) were used to perform reaction treatment according to the method described in step (b) of Example 1, thereby giving the target product (56 mg, yield: 52%).

¹HNMR(500MHz, CDCl₃)δ8.93(br.s, NH), 8.66(d, J = 2.2Hz, 1H), 7.89(dd, J = 2.7, 9.1Hz, 1H), 7.34(dd, J = 5.6, 8.8Hz, 1H), 6.73(dd, J = 3.0, 10.3Hz, 1H), 6.69-6.65(m, 2H), 4.74(s, 2H), 4.66-4.62(m, 1H), 3.93-3.82(m, 4H), 2.22(s, 3H), 2.02-1.97(m, 4H).

### [Step (c) of Example 8] Production of (5-(6-(4-(2-chloro-5-fluorophenoxy)piperidin-1-yl)pyridin-3-yl)-1,3,4-thiadiazol-2-yl)methyl acetate

2-(2-(6-(4-(2-Chloro-5-fluorophenyl)piperidin-1-yl)nicotinoyl)hydrazinyl-2-oxoethyl acetate (56 mg) produced in the above step (b), phosphorus pentasulfide (56 mg) and THF (2 mL) were used to perform reaction treatment according to the method described in step (c) of Example 1, thereby giving the target product (32 mg, yield: 49%).

¹HNMR(500MHz, CDCl₃)δ8.65(d, J = 2.4Hz, 1H), 8.10(dd, J = 2.5, 9.0Hz, 1H), 7.33(dd, J = 6.1, 8.8Hz, 1H), 6.76-6.72(m, 2H), 6.68-6.65(m, 1H), 5.50(s, 2H), 4.66-4.62(m, 1H), 3.92-3.88(m, 2H), 3.84-3.80(m, 1H), 2.17(s, 3H), 2.05-1.97(m, 4H).

### [Example 9] Production of (5-(6-(4-(2-chlorophenoxy)piperidin-1-yl)pyridazine-3-yl)-1,3,4-thiadiazol-2-yl)methyl acetate (Compound No.: NUT-33)

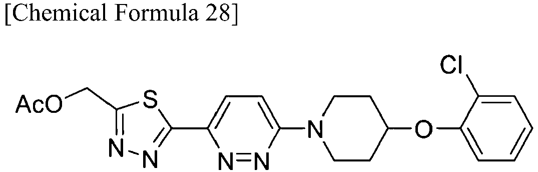

### [Step (a) of Example 9] Production of 6-(4-(2-chlorophenoxy)piperidin-1-yl)pyridazine-3-carbohydrazide

Methyl 6-(4-(2-chlorophenoxy)piperidin-1-yl)pyridazine-3-carboxylate (1.0 g) produced in Reference Production Example 6 and hydrazine monohydrate (5 mL) were used to perform reaction treatment according to the method described in step (a) of Example 1, thereby giving the target product as a crude product (347 mg, crude yield: 80%).

### [Step (b) of Example 9] Production of 2-(2-(6-(4-(2-chlorophenoxy)piperidin-1-yl)pyridazine-3-carbonyl)hydrazinyl)2-oxoethyl acetate

The crude product of 6-(4-(2-chlorophenoxy)piperidin-1-yl)pyridazine-3-carbohydrazide (347 mg) produced in the above step (a), iPr₂NEt (210 µL) and acetoxyacetyl chloride (119 µL) were used to perform reaction treatment according to the method described in step (b) of Example 1, thereby giving the target product (348 mg, yield: 78%).

¹HNMR(500MHz, CDCl₃)δ9.83(br.s, NH), 8.60(br.s, NH), 7.94(d, J = 9.6Hz, 1H), 7.41(dd, J = 1.7, 7.8Hz, 1H), 7.23(dt, J = 1.7, 8.3Hz, 1H), 7.01(d, J = 8.8Hz, 2H), 6.96(dt, J = 1.5, 8.1Hz, 1H), 4.76(s, 2H), 4.73(quint., J = 4.4Hz, 1H), 4.02-3.94(m, 4H), 2.22(s, 3H), 2.06-2.02(m, 4H).

### [Step (c) of Example 9] Production of (5-(6-(4-(2-chlorophenoxy)piperidin-1-yl)pyridazine-3-yl)-1,3,4-thiadiazol-2-yl)methyl acetate

2-(2-(6-(4-(2-Chlorophenoxy)piperidin-1-yl)pyridazine-3-carbonyl)hydrazinyl)2-oxoethyl acetate (143 mg) produced in the above step (b) and phosphorus pentasulfide (156 mg) were used to perform reaction treatment according to the method described in step (c) of Example 1, thereby giving the target product (45 mg, yield: 29%).

¹HNMR(500MHz, CDCl₃)δ8.16(d, J = 9.5Hz, 1H), 7.39(dd, J = 1.5, 7.8Hz, 1H), 7.22(dt, J = 1.5, 7.9Hz, 1H), 7.05(d, J = 9.8Hz, 1H), 6.99(dd, J = 1.2, 8.3Hz, 1H), 6.94(dt, J = 1.0, 7.6Hz, 1H), 5.51(s, 2H), 4.71(quint., J = 4.7Hz, 1H), 4.00-3.94(m, 4H), 2.17(s, 3H), 2.05-2.02(m, 4H).

### [Example 10] Production of (5-(6-(4-(2,6-dichlorophenoxy)piperidin-1-yl)pyridazine-3-yl)-1,3,4-thiadiazol-2-yl)methyl acetate (Compound No.: NUT-34)

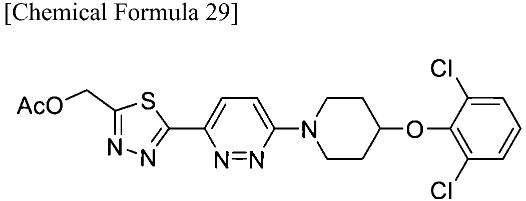

### [Step (a) of Example 10] Production of 6-(4-(2,6-dichlorophenoxy)piperidin-1-yl)pyridazine-3-carbohydrazide

Methyl 6-(4-(2,6-dichlorophenoxy)piperidin-1-yl)pyridazine-3-carboxylate (750 mg) produced in Reference Production Example 7 and hydrazine monohydrate (4 mL) were used to perform reaction treatment according to the method described in step (a) of Example 1, thereby giving the target product as a crude product (591 mg, crude yield: 79%).

### [Step (b) of Example 10] Production of 2-(2-(6-(4-(2,6-dichlorophenoxy)piperidin-1-yl)pyridazine-3-carbonyl)hydrazinyl)2-oxoethyl acetate

A portion (382 mg) of the crude product of 6-(4-(2,6-dichlorophenoxy)piperidin-1-yl)pyridazine-3-carbohydrazide produced in the above step (a), iPr₂NEt (210 µL), and acetoxyacetyl chloride (119 µL) were used to perform reaction treatment according to the method described in step (b) of Example 1, thereby giving the target product (271 mg, yield: 56%).

¹HNMR(500MHz, CDCl₃)δ9.84(br.s, NH), 8.63(br.s, NH), 7.95(d, J = 9.5Hz, 1H), 7.35(d, J = 8.3Hz, 2H), 7.03-7.00(m, 2H), 4.76(s, 2H), 4.58(quint., J = 5.2Hz, 1H), 4.30-4.25(m, 2H), 3.71-3.66(m, 2H), 2.22(s, 3H), 2.11-2.05(m, 4H).

### [Step (c) of Example 10] Production of (5-(6-(4-(2,6-dichlorophenoxy)piperidin-1-yl)pyridazine-3-yl)-1,3,4-thiadiazol-2-yl)methyl acetate

2-(2-(6-(4-(2,6-Dichlorophenoxy)piperidin-1-yl)pyridazine-3-carbonyl)hydrazinyl)2-oxoethyl acetate (154 mg) produced in the above step (b) and phosphorus pentasulfide (156 mg) were used to perform reaction treatment according to the method described in step (c) of Example 1, thereby giving the target product (45 mg, yield: 29%).

¹HNMR(500MHz, CDCl₃)δ8.18(d, J = 9.6Hz, 1H), 7.33(dd, J = 8.1Hz, 2H), 7.06(d, J = 9.8Hz, 1H), 7.02(t, J = 8.1Hz, 1H), 5.53(s, 2H), 4.58(quint., 5.6Hz, 1H), 4.02-4.27(m, 2H), 3.69-3.64(m, 2H), 2.19(s, 3H), 2.11-2.08(m, 4H).

### [Example 11] Production of (5-(4-(4-(2-chloro-5-fluorophenoxy)piperidin-1-yl)phenyl)-1,3,4-thiadiazol-2-yl)methyl acetate (Compound No.: NUT-28 (ADI-28))

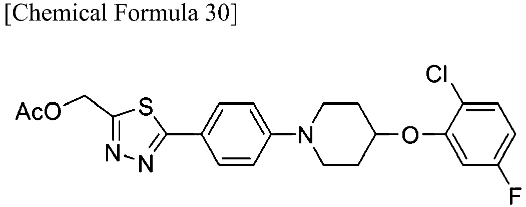

### [Step (a) of Example 11] Production of 4-(4-(2-chloro-5-fluorophenoxy)piperidin-1-yl)benzohydrazide

Methyl 4-(4-(2-chloro-5-fluorophenoxy)piperidin-1-yl)benzoate (546 mg) produced in Reference Production Example 8 and hydrazine monohydrate (2 mL) were used to perform reaction treatment according to the method described in step (a) of Example 1, thereby giving the target product as a crude product (476 mg, crude yield: 87%).

### [Step (b) of Example 11] Production of 2-(2-(4-(4-(2-chloro-5-fluorophenoxy)piperidin-1-yl)benzoyl)hydrazinyl)-2-oxoethyl acetate

A portion (400 mg) of the crude product of 4-(4-(2-chloro-5-fluorophenoxy)piperidin-1-yl)benzohydrazide produced in the above step (a), iPr₂NEt (143 µL) and acetoxyacetyl chloride (108 µL) were used to perform reaction treatment according to the method described in step (b) of Example 1, thereby giving the target product (366 mg).

¹HNMR(500MHz, CDCl₃)δ9.12(br.s, NH), 8.77(br.s, NH), 7.75(d, J = 8.6Hz, 2H), 7.33(dd, J = 6.1, 8.8Hz, 1H), 6.92(d, J = 8.8Hz, 2H), 6.72(dd, J = 2.7, 10.3Hz, 1H), 6.66(dt, J = 2.7, 8.8Hz, 1H), 4.72(s, 2H), 4.61-4.56(m, 1H), 3.66-3.61(m, 2H), 3.43-3.39(m, 2H), 2.21(s, 3H), 2.09-1.99(m, 4H).

### [Step (c) of Example 11] Production of (5-(4-(4-(2-chloro-5-fluorophenoxy)piperidin-1-yl)phenyl)-1,3,4-thiadiazol-2-yl)methyl acetate

2-(2-(4-(4-(2-Chloro-5-fluorophenoxy)piperidin-1-yl)benzoyl)hydrazinyl)-2-oxoethyl acetate (296 mg) produced in the above step (b), phosphorus pentasulfide (313 mg) and THF (8 mL) were used to perform reaction treatment according to the method described in step (c) of Example 1, thereby giving the target product (258 mg, yield: 87%).

¹HNMR(500MHz, CDCl₃)δ7.86(d, J = 8.8Hz, 2H), 7.33(dd, J = 6.1, 8.8Hz, 1H), 7.00(d, J = 8.8Hz, 1H), 6.73(dd, J = 2.7, 10.3Hz, 1H), 6.67(dt, J = 2.5, 8.1Hz, 1H), 5.50(s, 2H), 4.62-4.57(m, 1H), 3.66-3.61(m, 2H), 3.43-3.38(m, 2H), 2.18(s, 3H), 2.12-2.02(m, 4H).

### [Example 12] Production of (5-(4-(4-(2-Chlorophenoxy)piperidin-1-yl)phenyl)-1,3,4-thiadiazol-2-yl)methyl acetate (Compound No.: NUT-31)

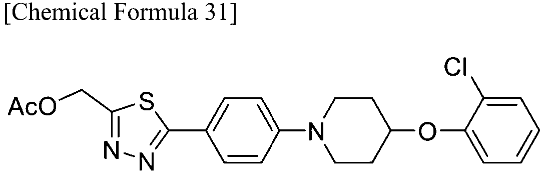

### [Step (a) of Example 12] Production of 4-(4-(2-chlorophenoxy)piperidin-1-yl)benzohydrazide

Methyl 4-(4-(2-chlorophenoxy)piperidin-1-yl)benzoate (690 mg) produced in Reference Production Example 9 and hydrazine monohydrate (2.5 mL) were used to perform reaction treatment according to the method described in step (a) of Example 1, thereby giving the target product as a crude product (345 mg, crude yield: 48%).

### [Step (b) of Example 12] Production of 2-(2-(4-(4-(2-chlorophenoxy)piperidin-1-yl)benzoyl)hydrazinyl)-2-oxoethyl acetate

The crude product of 4-(4-(2-chlorophenoxy)piperidin-1-yl)benzohydrazide (345 mg) produced in the above step (a), iPr₂NEt (210 µL) and acetoxyacetyl chloride (119 µL) were used to perform reaction treatment according to the method described in step (b) of Example 1, thereby giving the target product (248 mg, yield: 56%).

¹HNMR(500MHz, CDCl₃)δ9.25(br.s, NH), 8.89(br.s, NH), 7.75(d, J = 9.1Hz, 2H), 7.39(d, J = 7.8Hz, 1H), 7.22(t, J = 8.3Hz, 1H), 6.99(d, J = 8.3Hz, 1H), 6.95-6.89(m, 3H), 4.71(s, 2H), 4.63-4.60(m, 1H), 3.67-3.63(m, 2H), 3.41-3.37(m, 2H), 2.20(s, 3H), 2.08-1.95(m, 4H).

### [Step (c) of Example 12] Production of (5-(4-(4-(2-chlorophenoxy)piperidin-1-yl)phenyl)-1,3,4-thiadiazol-2-yl)methyl acetate

2-(2-(4-(4-(2-Chlorophenoxy)piperidin-1-yl)benzoyl)hydrazinyl)-2-oxoethyl acetate (143 mg) produced in the above step (b), phosphorus pentasulfide (156 mg) and THF (4 mL) were used to perform reaction treatment according to the method described in step (c) of Example 1, thereby giving the target product (76 mg, yield: 54%).

¹HNMR(500MHz, CDCl₃)δ7.85(d, J = 9.1Hz, 2H), 7.40(dd, J = 1.7, 8.1Hz, 1H), 7.00(dt, J = 1.7, 7.6Hz, 1H), 7.01-6.93(m, 4H), 5.50(s, 2H), 4.64-4.60(m, 1H), 3.68-3.63(m, 2H), 3.41-3.36(m, 2H), 2.17(s, 3H), 2.11-2.01(m, 4H).

### [Example 13] Production of (5-(4-(4-(2,6-dichlorophenoxy)piperidin-1-yl)phenyl)-1,3,4-thiadiazol-2-yl)methyl acetate (Compound No.: NUT-32)

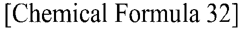

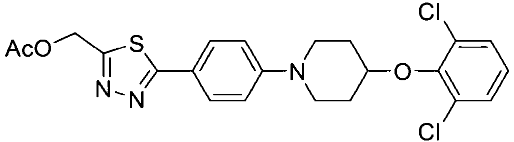

### [Step (a) of Example 13] Production of 4-(4-(2,6-dichlorophenoxy)piperidin-1-yl)benzohydrazide

Methyl 4-(4-(2,6-dichlorophenoxy)piperidin-1-yl)benzoate (734 mg) produced in Reference Production Example 10 and hydrazine monohydrate (2.5 mL) were used to perform reaction treatment according to the method described in step (a) of Example 1, thereby giving the target product as a crude product (343 mg, crude yield: 47%).

### [Step (b) of Example 13] Production of 2-(2-(4-(4-(2,6-dichlorophenoxy)piperidin-1-yl)benzoyl)hydrazinyl)-2-oxoethyl acetate

The crude product of 4-(4-(2,6-dichlorophenoxy)piperidin-1-yl)benzohydrazide (340 mg) produced in the above step (a), iPr₂NEt (188 µL) and acetoxyacetyl chloride (106 µL) were used to perform reaction treatment according to the method described in step (b) of Example 1, thereby giving the target product (348 mg, yield: 81%).

¹HNMR(500MHz, CDCl₃)δ9.30(br.s, NH), 8.96(br.s, NH), 7.75(d, J = 9.1Hz, 2H), 7.32(d, J = 8.1Hz, 2H), 7.00(t, J = 8.1Hz, 1H), 6.90(d, J = 9.1Hz, 1H), 4.70(s, 2H), 4.48(quint., J = 6.4Hz, 1H), 3.85-3.80(m, 2H), 3.22-3.16(m, 2H), 2.19(s, 3H), 2.08-2.05(m, 4H).

### [Step (c) of Example 13] Production of (5-(4-(4-(2,6-dichlorophenoxy)piperidin-1-yl)phenyl)-1,3,4-thiadiazol-2-yl)methyl acetate

2-(2-(4-(4-(2,6-Dichlorophenoxy)piperidin-1-yl)benzoyl)hydrazinyl)-2-oxoethyl acetate (154 mg) produced in the above step (b), phosphorus pentasulfide (156 mg) and THF (8 mL) were used to perform reaction treatment according to the method described in step (c) of Example 1, thereby giving the target product (258 mg, yield: 52%).

¹HNMR(500MHz, CDCl₃)δ7.82(d, J = 8.6Hz, 2H), 7.30(d, J = 8.1Hz, 2H), 7.02-6.94(m, 3H), 5.48(s, 2H), 4.46(quint., J = 5.4Hz, 1H), 3.85-3.79(m, 2H), 3.21-3.14(m, 2H), 2.16(s, 3H), 2.09-2.07(m, 4H).

### [Example 14] Production of 2-(4-(4-(2,6-dichlorophenoxy)piperidin-1-yl)phenyl)-5-butyl-1,3,4-thiadiazole (Compound No.: NUT-51)

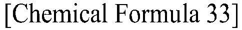

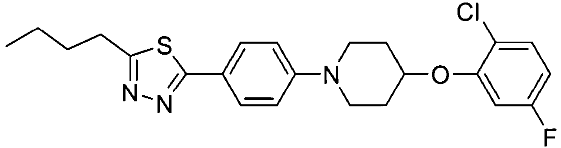

4-(4-(2-Chloro-5-fluorophenoxy)piperidin-1-yl)benzoyl)hydrazine-1-yl)-N'-pentanoylhydrazide (50 mg), phosphorus pentasulfide (52 mg) and THF (2 mL) were used to perform reaction treatment according to the method described in step (c) of Example 1, thereby giving the target product (21 mg, yield: 43%).

¹HNMR(500MHz, CDCl₃)δ7.82(d, J = 7.1Hz, 2H), 7.34-7.31(m, 1H), 6.97(d, J = 7.6Hz, 2H), 6.72(d, J = 10.1Hz, 1H), 6.68-6.64(m, 1H), 4.61-4.54(m, 1H), 3.63-3.60(m, 2H), 3.39-3.35(m, 2H), 3.11(t, J = 7.1Hz, 2H), 2.14-2.03(m, 4H), 1.80(quint., J = 7.4Hz, 2H), 1.51-1.43(m, 2H), 0.98(t, J = 7.6Hz, 3H).

### [Example 15] Production of (5-(6-(4-(2-(trifluoromethyl)phenoxy)piperidin-1-yl)pyridazine-3-yl)-1,3,4-thiadiazol-2-yl)methanol (Compound No.: NUT-11)

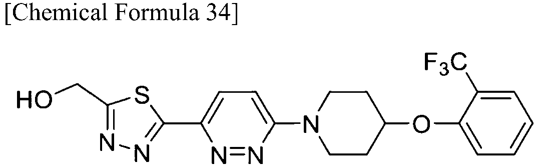

(5-(6-(4-(2-(Trifluoromethyl)phenoxy)piperidin-1-yl)pyridazine-3-yl)-1,3,4-thiadiazol-2-yl)methyl acetate (80 mg) produced in Example 3 was suspended in a mixed solution of THF (3 mL) and water (3 mL), lithium hydroxide monohydrate (35 mg) was added thereto, and the mixture was stirred at room temperature for 12 hours. After the reaction, the reaction liquid was diluted with water and then extracted with ethyl acetate. The extraction liquid was washed with saturated brine, and dried over magnesium sulfate. The solvent was distilled off under reduced pressure, and then the residue was dried under reduced pressure to give the target product (48 mg, yield: 66%).

¹HNMR(500MHz, CDCl₃)δ8.18(d, J = 9.8Hz, 1H), 7.62(d, J = 7.8Hz, 1H), 7.51(t, J = 8.6Hz, 1H), 7.07-7.03(m, 3H), 5.13(s, 2H), 4.86-4.83(m, 1H), 4.10-4.06(m, 2H), 3.88-3.82(m, 2H), 2.11-2.02(m, 4H).

### [Example 16] Production of (5-(6-(4-(2-chloro-5-fluorophenoxy)piperidin-1-yl)pyridazine-3-yl)-1,3,4-thiadiazol-2-yl)methanol (Compound No.: NUT-12)

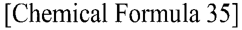

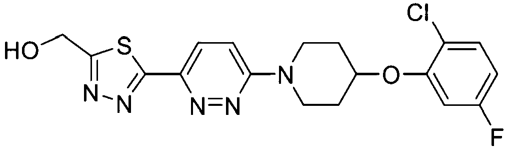

(5-(6-(4-(2-chloro-5-fluorophenoxy)piperidin-1-yl)pyridazine-3-yl)-1,3,4-thiadiazol-2-yl)methyl acetate (130 mg) produced in Example 4 and lithium hydroxide monohydrate (66 mg) were used to perform reaction treatment according to the method described in Example 15, thereby giving the target product (125 mg, yield: 93%).

¹HNMR(500MHz, CDCl₃)δ8.18(d, J = 9.5Hz, 1H), 7.35(dd, J = 5.9, 8.8Hz, 1H), 7.07(d, J = 9.8Hz, 1H), 6.74(dd, J = 2.7, 10.0Hz, 1H), 6.68(dt, J = 2.7, 7.8Hz, 1H), 5.14(s, 2H), 4.69(quint., J = 4.2Hz, 1H), 4.02-3.93(m, 4H), 2.86(br.s, OH), 2.08-2.04(m, 4H).

### [Example 17] Production of (5-(6-(4-(2-chloro-5-fluorophenoxy)piperidin-1-yl)pyridazine-3-yl)-1,3,4-thiadiazol-2-yl)methyl 3-(5-((3aS,4S,6aR)-2-oxooctahydrocyclopenta[d]imidazol-4-yl)pentanamide)propanoate (Compound No.: NUT-38)

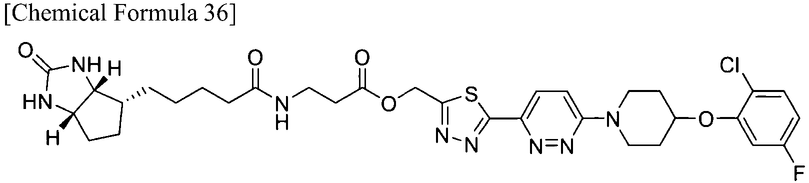

(5-(6-(4-(2-Chloro-5-fluorophenoxy)piperidin-1-yl)pyridazine-3-yl)-1,3,4-thiadiazol-2-yl)methanol (60 mg) produced in the same manner as in Example 16 was dissolved in DMF (3 mL), 3-((tert-butoxycarbonyl)amino)propanoic acid (40 mg), DMAP (26 mg), and EDCI (41 mg) were added thereto, and the mixture was stirred at room temperature for 12 hours. After the reaction, the solvent was distilled off under reduced pressure, and the residue was purified by column chromatography (eluent: ethyl acetate) to give a condensate (75 mg). This was dissolved in dichloromethane (1 mL), TFA (150 µL) was added thereto, and the mixture was stirred at room temperature for 6 hours. After the reaction, a saturated aqueous sodium hydrogen carbonate solution was added to the reaction liquid to make the reaction liquid basic, and the reaction liquid was extracted with dichloromethane. The extraction liquid was washed with water and saturated brine in this order, and dried over magnesium sulfate. The solvent was distilled off under reduced pressure, and then the residue was dried under reduced pressure to give (5-(6-(4-(2-chloro-5-fluorophenoxy)piperidin-1-yl)pyridazine-3-yl)-1,3,4-thiadiazol-2-yl)methyl 3-aminopropanoate (63 mg). This was dissolved in DMF (1 mL), and BiotinNHS (46 mg) was further added thereto, followed by stirring at room temperature for 12 hours. After the reaction, the solvent was distilled off under reduced pressure, and then the residue was purified by column chromatography (eluent: dichloromethane:methanol = 95:5) to give the target product (29 mg, yield: 34%).

¹HNMR(500MHz, CDCl₃)δ8.16(d, J = 9.6Hz, 1H), 7.34(dd, J = 6.2, 8.8Hz, 1H), 7.06(d, J = 9.8Hz, 1H), 6.96(br.t, 1H), 6.73(dd, J = 2.4, 10.0Hz, 1H), 6.69-6.66(m, 1H), 6.51(br.s, 1H), 5.66(br.s, 1H), 5.55(s, 2H), 4.69-4.68(m, 1H), 4.52-4.50(m, 1H), 4.32-4.30(m, 1H), 3.99-3.94(m, 4H), 3.57-3.54(m, 2H), 3.15-3.11(m, 1H), 2.90(dd, J = 4.6, 12.7Hz, 1H), 2.73(d, J = 12.9Hz, 1H), 2.70-2.67(m, 2H), 2.21(t, J = 7.4Hz, 2H), 2.09-2.01(m, 4H), 1.72-1.63(m, 4H), 1.42-1.39(m, 2H).

### [Example 18] Production of (5-(4-(4-(2-chloro-5-fluorophenoxy)piperidin-1-yl)phenyl)-1,3,4-thiadiazol-2-yl)methanol (Compound No.: NUT-45)

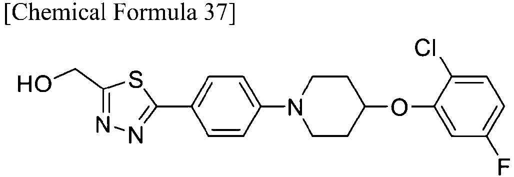

(5-(4-(4-(2-Chloro-5-fluorophenoxy)piperidin-1-yl)phenyl)-1,3,4-thiadiazol-2-yl)methyl acetate (258 mg) produced in Example 11 and lithium hydroxide monohydrate (117 mg) were used to perform reaction treatment according to the method described in Example 15, thereby giving the target product (190 mg, yield: 81%).

¹HNMR(400MHz, DMSO-d6)δ7.78(d, J = 8.9Hz, 2H), 7.47(dd, J = 6.2, 8.9Hz, 1H), 7.28(dd, J = 2.8, 11.0Hz, 1H), 7.08(d, J = 9.2Hz, 2H), 6.83(dt, J = 3.0, 8.2Hz, 1H), 4.85-4.80(m, 3H), 3.65-3.69(m, 2H), 3.39-3.28(m, 2H), 2.05-1.98(m, 2H), 1.78-1.73(m, 2H).

### [Example 19] Production of (5-(4-(4-(2-chloro-5-fluorophenoxy)piperidin-1-yl)phenyl)-1,3-thiazol-2-yl)methanol (Compound No.: NUT-63)

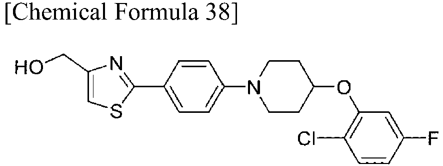

### [Step (a) of Example 19] Production of ethyl 2-(4-(4-(2-chloro-5-fluorophenoxy)piperidin-1-yl)phenyl)thiazole-4-carboxylate

Ethyl 2-(4-bromophenyl)thiazole-4-carboxylate (600 mg) and 4-(2-chloro-5-fluorophenoxy)piperidine (440 mg) were dissolved in 1,4-dioxane (15 mL), Pd₂(dpa)₃ (18 mg), Xantphos (28 mg), and cesium carbonate (963 mg) were added thereto, and the mixture was stirred at 100°C for 12 hours. After the reaction, the reaction liquid was diluted with water and then extracted with ethyl acetate. The extraction liquid was washed with water and saturated brine in this order, and dried over magnesium sulfate. The solvent was distilled off under reduced pressure, and then the residue was purified by column chromatography (eluent: ethyl acetate:hexane = 1:4) to give the target product (124 mg, yield: 14%).

¹HNMR(500MHz, CDCl₃)δ8.06(s, 1H), 7.91(d, J = 8.8Hz, 2H), 7.33(dd, J = 6.1, 8.8Hz, 1H), 6.96(d, J = 8.8Hz, 2H), 6.72(dd, J = 2.7, 10.3Hz, 1H), 6.66(dt, J = 2.7, 7.8Hz, 1H), 4.59-4.55(m, 1H), 4.45(q, J = 7.1Hz, 2H), 3.64-3.59(m, 2H), 3.39-3.34(m, 2H), 2.13-2.01(m, 4H), 1.44(t, J = 7.1Hz, 3H).

### [Step (b) of Example 19] Production of (5-(4-(4-(2-chloro-5-fluorophenoxy)piperidin-1-yl)phenyl)-1,3-thiazol-2-yl)methanol

Ethyl 2-(4-(4-(2-chloro-5-fluorophenoxy)piperidin-1-yl)phenyl)thiazole-4-carboxylate (100 mg) obtained in the above step (a) was dissolved in THF (2 mL), and the reaction solution was cooled to 0°C, LAH (12 mg) was added thereto, and the mixture was stirred at room temperature for 1 hour. After the reaction, the reaction liquid was quenched with a 10% aqueous sodium hydrogen carbonate solution, and extracted with ethyl acetate. The extraction liquid was washed with saturated brine, and the residue was purified by column chromatography (eluent: ethyl acetate:hexane = 1:1), and dried over magnesium sulfate. The solvent was distilled off under reduced pressure to give the target product (80 mg, yield: 88%).

¹HNMR(500MHz, CDCl₃)δ7.82(d, J = 8.8Hz, 2H), 7.33(dd, J = 6.1, 8.6Hz, 1H), 7.06(s, 1H), 6.95(d, J = 8.8Hz, 2H), 6.72(dd, J = 2.7, 10.0Hz, 1H), 6.66(dt, J = 2.7, 8.1Hz, 1H), 4.80(s, 2H), 4.57-4.54(m, 1H), 3.61-3.54(m, 2H), 3.35-3.30(m, 2H), 2.10-2.01(m, 4H).

The following scheme generalizes an example of producing a compound having the ring B such as a thiadiazole group substituted with an "R(C=O)O-CH₂-group" from a compound having the ring B such as a thiadiazole group substituted with methanols. 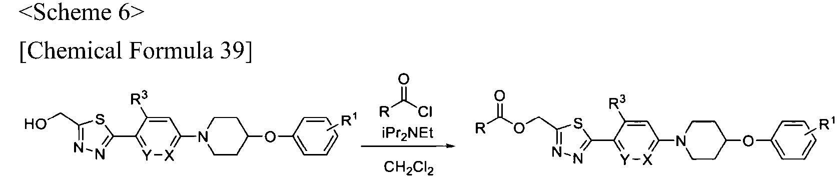 where each symbol has the same meaning as described above.

The details and specific examples will be described with reference to Examples 20 to 29 below.

### [Example 20] Production of (5-(6-(4-(2-chloro-5-fluorophenoxy)piperidin-1-yl)pyridazine-3-yl)-1,3,4-thiadiazol-2-yl)methyl isobutyrate (Compound No.: NUT-20)

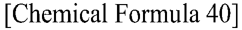

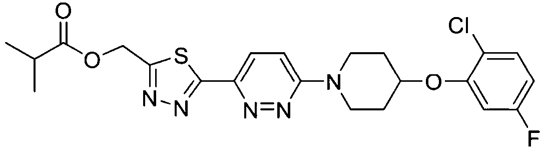

(5-(6-(4-(2-Chloro-5-fluorophenoxy)piperidin-1-yl)pyridazine-3-yl)-1,3,4-thiadiazol-2-yl)methanol (125 mg) produced in the same manner as in Example 16 was dissolved in dichloromethane (3 mL), and the reaction solution was cooled to 0°C, iPr₂NEt (78 µL), DMAP (4 mg), and isobutyl chloride (37 µL) were added thereto, and the mixture was stirred at room temperature for 10 hours. After the reaction, the reaction liquid was diluted with water and then extracted with ethyl acetate. The extraction liquid was washed with saturated brine in this order, and dried over magnesium sulfate. The solvent was distilled off under reduced pressure, and then the residue was purified by column chromatography (eluent: ethyl acetate:hexane = 1:1) to give the target product (92 mg, yield: 62%).

¹HNMR(500MHz, CDCl₃)δ8.18(d, J = 9.6Hz, 1H), 7.34(dd, J = 6.1, 8.8Hz, 1H), 7.06(d, J = 9.8Hz, 1H), 6.73(dd, J = 2.7, 10.0Hz, 1H), 6.67(dt, J = 2.7, 8.6Hz, 1H), 5.54(s, 2H), 4.69(quint., J = 4.4Hz, 1H), 4.02-3.93(m, 4H), 2.62(sept., J = 6.9Hz, 1H), 2.09-2.02(m, 4H), 1.23(d, J = 7.1Hz, 6H).

### [Example 21] Production of (5-(4-(4-(2-chloro-5-fluorophenoxy)piperidin-1-yl)phenyl)-1,3,4-thiadiazol-2-yl)methyl isobutyrate (Compound No.: NUT-29 (ADI-29))

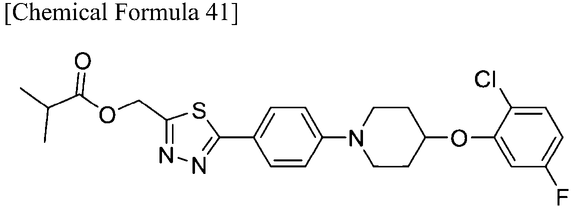

(5-(4-(4-(2-Chloro-5-fluorophenoxy)piperidin-1-yl)phenyl)-1,3,4-thiadiazol-2-yl)methanol (140 mg) produced in the same manner as in Example 18, iPr₂NEt (81 µL), DMAP (10 mg) and isobutyl chloride (81 µL) were used to perform reaction treatment according to the method described in Example 20, thereby giving the target product (148 mg, yield: 97%).

¹HNMR(500MHz, CDCl₃)δ7.86(d, J = 8.8Hz, 2H), 7.33(dd, J = 6.4, 8.8Hz, 1H), 6.98(d, J = 8.8Hz, 2H), 6.73(dd, J = 3.0, 10.3Hz, 1H), 6.69-6.65(m, 1H), 5.51(s, 2H), 4.61-4.56(m, 1H), 3.66-3.61(m, 2H), 3.42-3.38(m, 2H), 2.67(sept., J = 7.1Hz, 1H), 2.12-2.01(m, 4H), 1.23(d, J = 6.9Hz, 6H).

### [Example 22] Production of (5-(4-(4-(2-chloro-5-fluorophenoxy)piperidin-1-yl)phenyl)-1,3,4-thiadiazol-2-yl)methyl propionate (Compound No.: NUT-46)

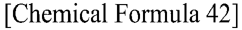

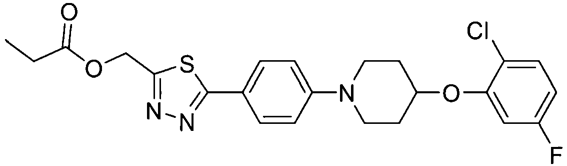

(5-(4-(4-(2-Chloro-5-fluorophenoxy)piperidin-1-yl)phenyl)-1,3,4-thiadiazol-2-yl)methanol (27 mg) produced in the same manner as in Example 18, iPr₂NEt (18 µL), DMAP (2 mg) and propionyl chloride (7 µL) were used to perform reaction treatment according to the method described in Example 20, thereby giving the target product (24 mg, yield: 78%).

¹HNMR(500MHz, CDCl₃)δ7.85(d, J = 8.6Hz, 2H), 7.33(dd, J = 6.1, 8.6Hz, 1H), 6.97(d, J = 8.6Hz, 2H), 6.73(dd, J = 2.7, 10.3Hz, 1H), 6.66(dt, J = 2.4, 8.6Hz, 1H), 5.51(s, 2H), 4.61-4.57(m, 1H), 3.65-3.60(m, 2H), 3.42-3.37(m, 2H), 2.45(q, J = 7.6Hz, 2H), 2.11-2.00(m, 4H), 1.20(t, J = 7.6Hz, 3H).

### [Example 23] Production of (5-(4-(4-(2-chloro-5-fluorophenoxy)piperidin-1-yl)phenyl)-1,3,4-thiadiazol-2-yl)methyl butyrate (Compound No.: NUT-65)

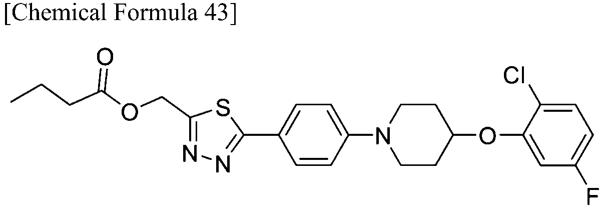

(5-(4-(4-(2-Chloro-5-fluorophenoxy)piperidin-1-yl)phenyl)-1,3,4-thiadiazol-2-yl)methanol (27 mg) produced in the same manner as in Example 18, iPr₂NEt (18 µL), DMAP (2 mg) and butyric acid chloride (7 µL) were used to perform reaction treatment according to the method described in Example 20, thereby giving the target product (21 mg, yield: 71%).

¹HNMR(500MHz, CDCl₃)δ7.84(d, J = 8.8Hz, 2H), 7.33(dd, J = 6.1, 8.8Hz, 1H), 6.97(d, J = 9.0Hz, 2H), 6.72(dd, J = 2.7, 10.0Hz, 1H), 6.66(dt, J = 2.7, 8.3Hz, 1H), 5.50(s, 2H), 4.59-4.57(m, 1H), 3.65-3.60(m, 2H), 3.41-3.37(m, 2H), 2.40(t, J = 7.3Hz, 2H), 2.11-2.01(m, 4H), 1.71(q, J = 7.6Hz, 2H), 0.98(t, J = 7.4Hz, 3H).

### [Example 24] Production of (5-(4-(4-(2-chloro-5-fluorophenoxy)piperidin-1-yl)phenyl)-1,3,4-thiadiazol-2-yl)methyl pentanoate (Compound No.: NUT-47)

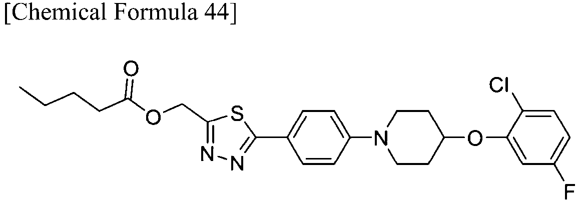

(5-(4-(4-(2-Chloro-5-fluorophenoxy)piperidin-1-yl)phenyl)-1,3,4-thiadiazol-2-yl)methanol (27 mg) produced in the similar manner as in Example 18, iPr₂NEt (18 µL), DMAP (2 mg) and valerochloride (10 µL) were used to perform reaction treatment according to the method described in Example 20, thereby giving the target product (26 mg, yield: 80%).

¹HNMR(500MHz, CDCl₃)δ7.85(d, J = 8.8Hz, 2H), 7.33(dd, J = 6.1, 8.8Hz, 1H), 6.97(d, J = 8.6Hz, 2H), 6.72(dd, J = 2.7, 10.3Hz, 1H), 6.66(dt, J = 3.0, 8.8Hz, 1H), 5.50(s, 2H), 4.60-4.54(m, 1H), 3.65-3.60(m, 2H), 3.41-3.37(m, 2H), 2.42(t, J = 7.4Hz, 2H), 2.11-1.99(m, 4H), 1.78-1.63(m, 2H), 1.40-1.34(m, 2H), 0.93(t, J = 7.4Hz, 3H).

### [Example 25] Production of (5-(4-(4-(2-chloro-5-fluorophenoxy)piperidin-1-yl)phenyl)-1,3,4-thiadiazol-2-yl)methyl cyclobutanecarboxylate (Compound No.: NUT-48)

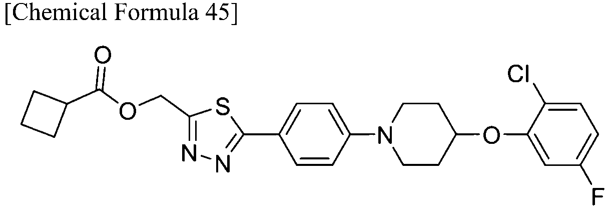

(5-(4-(4-(2-Chloro-5-fluorophenoxy)piperidin-1-yl)phenyl)-1,3,4-thiadiazol-2-yl)methanol (27 mg) produced in the same manner as in Example 18, iPr₂NEt (18 µL), DMAP (2 mg), and cyclobutanoyl chloride (9 µL) were used to perform reaction treatment in the same manner as in Example 20 to give the target product (24 mg, yield: 75%).

¹HNMR(500MHz, CDCl₃)δ7.85(d, J = 8.8Hz, 2H), 7.32(dd, J = 6.4, 8.8Hz, 1H), 6.97(d, J = 8.6Hz, 2H), 6.72(dd, J = 2.7, 10.3Hz, 1H), 6.66(dt, J = 2.5, 7.9Hz, 1H), 5.50(s, 2H), 4.59-4.57(m, 1H), 3.65-3.60(m, 2H), 3.42-3.38(m, 2H), 3.24(quint., J = 8.6Hz, 1H), 2.36-2.32(m, 2H), 2.26-2.24(m, 2H), 2.09-1.93(m, 6H).

### [Example 26] Production of (5-(4-(4-(2-chloro-5-fluorophenoxy)piperidin-1-yl)phenyl)-1,3,4-thiadiazol-2-yl)methyl benzoate (Compound No.: NUT-49)

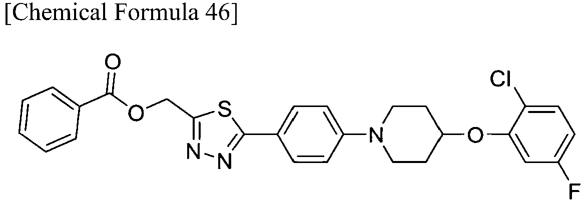

(5-(4-(4-(2-Chloro-5-fluorophenoxy)piperidin-1-yl)phenyl)-1,3,4-thiadiazol-2-yl)methanol (27 mg) produced in the same manner as in Example 18, iPr₂NEt (29 µL), DMAP (2 mg) and benzoyl chloride (16 µL) were used to perform reaction treatment according to the method described in Example 20, thereby giving the target product (29 mg, yield: 88%).

¹HNMR(500MHz, CDCl₃)δ8.10(d, J = 8.1Hz, 2H), 7.85(d, J = 8.6Hz, 2H), 7.61(t, J = 7.6Hz, 1H), 7.48(t, J = 7.8Hz, 2H), 7.32(dd, J = 6.1, 8.6Hz, 1H), 6.96(d, J = 8.6Hz, 2H), 6.72(dd, J = 2.7, 10.3Hz, 1H), 6.66(dt, J = 2.2, 7.8Hz, 1H), 5.75(s, 2H), 4.60-4.56(m, 1H), 3.65-3.60(m, 2H), 3.41-3.36(m, 2H), 2.12-2.00(m, 4H)

### [Example 27] Production of (5-(4-(4-(2-chloro-5-fluorophenoxy)piperidin-1-yl)phenyl)-1,3,4-thiadiazol-2-yl)methyl cyclopropanecarboxylate (Compound No.: NUT-50)

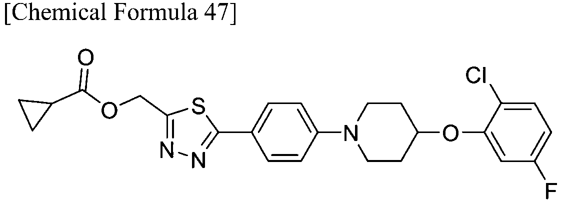

(5-(4-(4-(2-Chloro-5-fluorophenoxy)piperidin-1-yl)phenyl)-1,3,4-thiadiazol-2-yl)methanol (30 mg) produced in the similar manner as in Example 18, iPr₂NEt (18 µL), DMAP (2 mg) and cyclopropanecarbonylchloride (8 µL) were used to perform reaction treatment according to the method described in Example 20, thereby giving the target product (24 mg, yield: 70%).

¹HNMR(500MHz, CDCl₃)δ7.85(d, J = 8.8Hz, 2H), 7.33(dd, J = 6.1, 8.8Hz, 1H), 6.97(d, J = 9.1Hz, 2H), 6.72(dd, J = 2.7, 10.3Hz, 1H), 6.68-6.65(m, 1H), 5.50(s, 2H), 4.62-4.55(m, 1H), 3.65-3.60(m, 2H), 3.42-3.37(m, 2H), 2.10-2.07(m, 4H), 1.75-1.69(m, 1H), 1.11-1.08(m, 2H), 0.98-0.94(m, 2H).

### [Example 28] Production of (5-(4-(4-(2-chloro-5-fluorophenoxy)piperidin-1-yl)phenyl)-1,3,4-thiadiazol-2-yl)methyl cyclopentanecarboxylate (Compound No.: NUT-60)

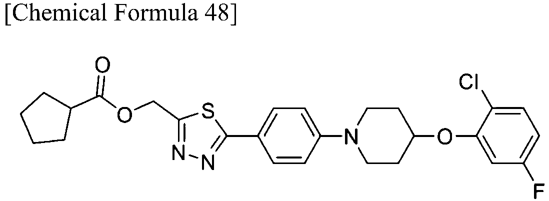

(5-(4-(4-(2-Chloro-5-fluorophenoxy)piperidin-1-yl)phenyl)-1,3,4-thiadiazol-2-yl)methanol (27 mg) produced in the similar manner as in Example 18, iPr₂NEt (16 µL), DMAP (2 mg) and cyclopentanecarbonylchloride (9 µL) were used to perform reaction treatment according to the method described in Example 20, thereby giving the target product (25 mg, yield: 81%).

¹HNMR(500MHz, CDCl₃)δ7.85(d, J = 8.6Hz, 2H), 7.33(dd, J = 6.4, 8.8Hz, 1H), 6.97(d, J = 8.8Hz, 2H), 6.73(dd, J = 2.7, 10.3Hz, 1H), 6.66(dt, J = 3.0, 8.1Hz, 1H), 5.50(s, 2H), 4.60-4.57(m, 1H), 3.65-3.60(m, 2H), 3.42-3.38(m, 2H), 3.84(quint, J = 7.8Hz, 1H), 2.11-2.00(m, 4H), 1.96-1.83(m, 4H), 1.76-1.71(m, 2H), 1.64-1.59(m, 2H).

### [Example 29] Production of (2-(4-(4-(2-chloro-5-fluorophenoxy)piperidin-1-yl)phenyl)thiazol-4-yl)methyl isobutyrate (Compound No.: NUT-64)

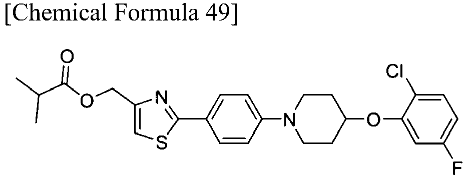

(5-(4-(4-(2-Chloro-5-fluorophenoxy)piperidin-1-yl)phenyl)-1,3,4-thiadiazol-2-yl)methanol (25 mg) produced in the same manner as in Example 18 was dissolved in dichloromethane (3 mL), the reaction solution was cooled to 0°C, iPr₂NEt (16 µL), DMAP (2 mg), and isobutyl chloride (8 µL) were added thereto, and the mixture was stirred at room temperature for 10 hours. After the reaction, the reaction liquid was diluted with water and then extracted with ethyl acetate. The extraction liquid was washed with saturated brine in this order, and dried over magnesium sulfate. The solvent was distilled off under reduced pressure, and then the residue was purified by column chromatography (eluent: ethyl acetate:hexane = 1:2) to give the target product (22 mg, yield: 74%).

¹HNMR(500MHz, CDCl₃)δ7.84(d, J = 8.3Hz, 2H), 7.32(dd, J = 6.1, 8.8Hz, 1H), 7.14(s, 1H), 6.96(d, J = 8.6Hz, 2H), 6.72(dd, J = 2.7, 10.1Hz, 1H), 6.68-6.64(m, 1H), 5.27(s, 2H), 4.57-4.54(m, 1H), 3.62-3.57(m, 2H), 3.36-3.31(m, 2H), 2.66(sept.J = 6.8Hz, 1H), 2.12-2.00(m, 4H), 1.22(d, J = 6.8Hz, 6H).

A scheme generalized from Example 29 is as follows. 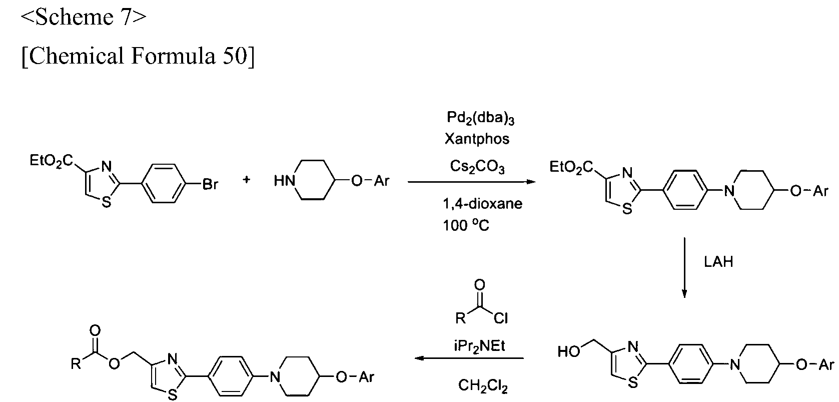 where each symbol has the same meaning as described above.

### [Example 30] Production of 5-(4-(4-(2-chloro-5-fluorophenoxy)piperidin-1-yl)phenyl)-1,3,4-oxadiazol-2-yl)methyl acetate

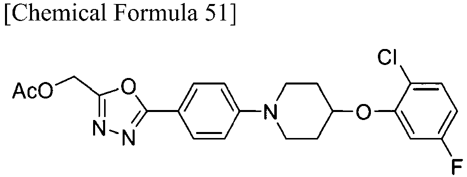

2-(2-(4-(4-(2-Chloro-5-fluorophenoxy)piperidin-1-yl)benzoyl)hydrazinyl)-2-oxoethyl acetate (200 mg) produced in step (b) of Example 11 and phosphoryl chloride (4 mL) were used to perform reaction treatment according to the method described in Example 5, thereby giving the target product (160 mg, yield: 83%).

¹HNMR(500MHz, CDCl₃)δ7.92(d, J = 9.1Hz, 2H), 7.32(dd, J = 6.1, 8.8Hz, 1H), 6.98(d, J = 8.8Hz, H), 6.72(dd, J = 3.6, 10.3Hz, 1H), 6.66(dt, J = 2.7, 8.1Hz, 1H), 5.32(s, 2H), 4.61-4.56(m, 1H), 3.66-3.61(m, 2H), 3.44-3.39(m, 2H), 2.18(s, 3H), 2.09-2.00(m, 4H).

### [Example 31] Production of (5-(4-(4-(2-chloro-5-fluorophenoxy)piperidin-1-yl)phenyl)-1,3,4-oxadiazol-2-yl)methanol (Compound No.: NUT-81)

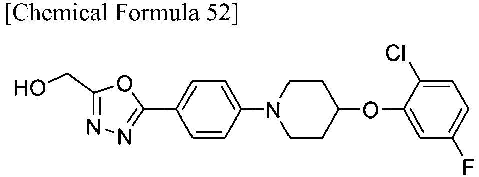

2-(2-(4-(4-(2-Chloro-5-fluorophenoxy)piperidin-1-yl)benzoyl)hydrazinyl)-2-oxoethyl acetate (130 mg) produced in step (b) of Example 11 and lithium hydroxide monohydrate (61 mg) were used to perform reaction treatment according to the method described in Example 15, thereby giving the target product (101 mg, yield: 86%).

¹HNMR(500MHz, CDCl₃)δ7.92(d, J = 8.8Hz, 2H), 7.33(dd, J = 6.1, 8.6Hz, 1H), 6.98(d, J = 8.8Hz, H), 6.72(dd, J = 2.7, 10.1Hz, 1H), 6.66(dt, J = 2.9, 8.1Hz, 1H), 4.92(d, J = 6.6Hz, 2H), 4.60-4.57(m, 1H), 3.65-3.61(m, 2H), 3.44-3.40(m, 2H), 3.11(br.s, OH), 2.09-1.99(m, 4H).

### [Example 32] Production of 5-(4-(4-(2-chloro-5-fluorophenoxy)piperidin-1-yl)phenyl)-1,3,4-oxadiazol-2-yl)methyl isobutyrate (Compound No.: NUT-67)

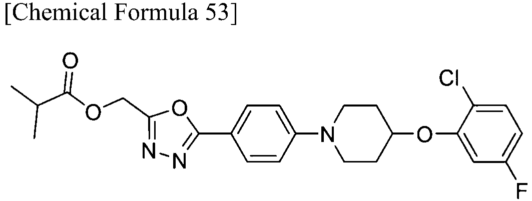

(5-(4-(4-(2-Chloro-5-fluorophenoxy)piperidin-1-yl)phenyl)-1,3,4-oxadiazol-2-yl)methanol (70 mg) produced in the same manner as in Example 31, iPr₂NEt (45 µL), DMAP (5 mg) and isobutyl chloride (22 µL) were used to perform reaction treatment according to the method described in Example 20, thereby giving the target product (68 mg, yield: 83%).

¹HNMR(500MHz, CDCl₃)δ7.93(d, J = 9.1Hz, 2H), 7.33(dd, J = 6.1, 8.8Hz, 1H), 6.99(d, J = 8.8Hz, 2H), 6.73(dd, J = 2.7, 10.0Hz, 1H), 6.67(dt, J = 2.7, 8.6Hz, 1H), 5.33(s, 2H), 4.61-4.58(m, 1H), 3.67-3.62(m, 2H), 3.45-3.40(m, 2H), 2.68(sept., J = 7.1Hz, 1H), 2.12-1.99(m, 4H), 1.23(d, J = 6.9Hz, 6H).

### [Reference Production Example 11] Production of methyl 4-(4-(2-methylphenoxy)piperidin-1-yl)benzoate

Methyl 4-bromobenzoate (1.0 g), 4-(2-methylphenoxy)piperidine (918 mg), Pd₂(dpa)₃ (44 mg), Xantphos (69 mg) and cesium carbonate (2.4 g) were used to perform reaction treatment according to the method described in Reference Production Example 8, thereby giving the target product (797 mg, yield: 51%).

¹HNMR(400MHz, CDCl₃)δ7.93(d, J = 9.1Hz, 2H), 7.18-7.14(m, 2H), 6.92-6.86(m, 4H), 4.60-4.55(m, 1H), 3.88(s, 3H), 3.64-3.59(m, 2H), 3.41-3.35(m, 2H), 2.25(s, 3H), 2.11-2.04(m, 2H), 2.00-1.94(m, 2H).

### [Reference Production Example 12] Production of methyl 4-(4-(2-fluorophenoxy)piperidin-1-yl)benzoate

Methyl 4-bromobenzoate (959 mg), 4-(2-fluorophenoxy)piperidine (870 mg), Pd₂(dpa)₃ (41 mg), Xantphos (65 mg) and cesium carbonate (2.2 g) were used to perform reaction treatment according to the method described in Reference Production Example 8, thereby giving the target product (1.1 g, yield: 77%).

¹HNMR(500MHz, CDCl₃)δ7.92(d, J = 8.8Hz, 2H), 7.12-7.02(m, 3H), 6.98-6.95(m, 1H), 6.90(d, J = 8.8Hz, 2H), 4.53-4.50(m, 1H), 3.88(s, 3H), 3.72-3.67(m, 2H), 3.33-3.28(m, 2H), 2.10-2.06(m, 2H), 1.98-1.93(m, 2H).

### [Reference Production Example 13] Production of methyl 4-(4-(2-(trifluoromethyl)phenoxy)piperidin-1-yl)benzoate

Methyl 4-bromobenzoate (758 mg) and 4-(2-(trifluoromethyl)phenoxy)piperidine (1.2 g) were dissolved in 1,2-dimethoxyethane (18 mL), Pd₂(dpa)₃ (160 mg), S-phos (144 mg), and tripotassium phosphate (1.6 g) were added thereto, and the mixture was stirred at 80°C for 12 hours. After the reaction, the reaction liquid was diluted with water and then extracted with ethyl acetate. The extraction liquid was washed with water and saturated brine in this order, and dried over magnesium sulfate. The solvent was distilled off under reduced pressure, and then the residue was purified by column chromatography (eluent: ethyl acetate:hexane = 1:4) to give the target product (334 mg, yield: 25%).

¹HNMR(500MHz, CDCl₃)δ7.92(d, J = 8.8Hz, 2H), 7.59(d, J = 7.9Hz, 1H), 7.48(t, J = 7.6Hz, 1H), 7.02-6.99(m, 2H), 6.69(d, J = 8.8Hz, 2H), 4.74-4.69(m, 1H), 3.86(s, 3H), 3.59-3.54(m, 2H), 3.46-3.42(m, 2H), 2.06-1.99(m, 4H).

### [Reference Production Example 14] Production of methyl 4-(4-(2-chloro-5-fluorophenoxy)piperidin-1-yl)-2-(methoxymethoxy)benzoate

Methyl 4-bromo-2-(methoxymethoxy)benzoate (1.43 g), 2-chloro-5-fluorophenoxy)piperidine (1.0 g), Pd₂(dpa)₃ (39 mg), Xantphos (62 mg) and cesium carbonate (2.2 g) were used to perform reaction treatment according to the method described in Reference Production Example 8, thereby giving the target product (887 mg, yield: 48%).

¹HNMR(500MHz, CDCl₃)δ7.80(d, J = 8.8Hz, 1H), 7.33(dd, J = 6.2, 8.8Hz, 1H), 6.72(dd, J = 2.7, 10.3Hz, 1H), 6.67-6.57(m, 2H), 5.25(s, 2H), 4.61-4.54(m, 1H), 3.85(s, 3H), 3.63-3.59(m, 2H), 3.54(s, 3H), 3.41-3.36(m, 2H), 2.11-1.96(m, 4H).

### [Reference Production Example 15] Production of methyl 4-(4-(2-chloro-5-fluorophenoxy)piperidin-1-yl)-2-methoxybenzoate

Methyl 4-bromo-2-methoxybenzoate (1.15 g), 2-chloro-5-fluorophenoxy)piperidine (900 mg), Pd₂(dpa)₃ (39 mg), Xantphos (62 mg) and cesium carbonate (2.2 g) were used to perform reaction treatment according to the method described in Reference Production Example 8, thereby giving the target product (511 mg, yield: 33%).

¹HNMR(500MHz, CDCl₃)δ7.81(d, J = 8.8Hz, 1H), 7.33(dd, J = 6.1, 8.8Hz, 1H), 6.72(dd, J = 2.7, 10.1Hz, 1H), 6.66(dt, J = 2.7, 8.1Hz, 1H), 6.50(dd, J = 2.2, 8.8Hz, 1H), 6.41(d, J = 2.2Hz, 1H), 4.60-4.57(m, 1H), 3.91(s, 3H), 3.85(s, 3H), 3.65-3.60(m, 2H), 3.42-3.38(m, 2H), 2.11-1.98(m, 4H).

### [Example 33] Production of (5-(2-(2-aminoethoxy)-4-(2-chloro-5-fluorophenoxy)piperidin-1-yl)phenyl)-1,3,4-thiadiazol-2-yl)methyl acetate (Compound No.: NUT-39)

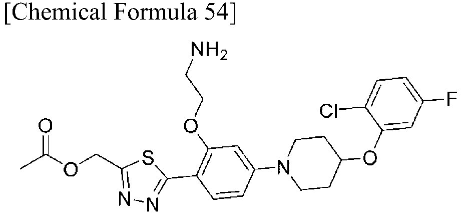

### [Step (a) of Example 33] Production of 4-(4-(2-chloro-5-fluorophenoxy)piperidin-1-yl)-2-(methoxymethoxy)benzohydrazide

Methyl 4-(4-(2-chloro-5-fluorophenoxy)piperidin-1-yl)-2-(methoxymethoxy)benzoate (887 mg) produced in Reference Production Example 14 and hydrazine monohydrate (2.7 mL) were used to perform reaction treatment according to the method described in step (a) of Example 1, thereby giving the target product as a crude product (770 mg, crude yield: 87%).

### [Step (b) of Example 33] Production of 2-(2-(4-(4-(2-chloro-5-fluorophenoxy)piperidin-1-yl)-2-(methoxymethoxy)benzoyl)hydrazinyl)-2-oxoethyl acetate

A portion (700 mg) of the crude product of 4-(4-(2-chloro-5-fluorophenoxy)piperidin-1-yl)-2-(methoxymethoxy)benzohydrazide produced in the above step (a), iPr₂NEt (346 µL) and acetoxyacetyl chloride (180 µL) were used to perform reaction treatment according to the method described in step (b) of Example 1, thereby giving the target product (700 mg).

¹HNMR(500MHz, CDCl₃)δ10.50(d, J = 6.7Hz, 1H), 9.99(d, J = 7.1Hz, 1H), 8.03(d, J = 8.8Hz, 1H), 7.32(dd, J = 6.1, 8.8Hz, 1H), 6.73-6.64(m, 4H), 5.40(s, 2H), 4.77(s, 2H), 4.60-4.55(m, 1H), 3.64-3.58(m, 5H), 3.42-3.38(m, 2H), 2.18(s, 3H), 2.10-1.98(m, 4H).

### [Step (c) of Example 33] Production of (5-(4-(4-(2-chloro-5-fluorophenoxy)piperidin-1-yl)-2-hydroxyphenyl)-1,3,4-thiadiazol-2-yl)methyl acetate

2-(2-(4-(4-(2-Chloro-5-fluorophenoxy)piperidin-1-yl)-2-(methoxymethoxy)benzoyl)hydrazinyl)-2-oxoethyl acetate (168 mg) produced in the above step (b), THF (25 mL), phosphorus pentasulfide (313 mg) and THF (4 mL) were used to perform reaction treatment according to the method described in step (c) of Example 1, thereby giving the target product (67 mg, yield: 44%).

¹HNMR(300MHz, CDCl₃)δ7.60(d, J = 9.4Hz, 0.5H), 7.35-7.30(m, 1.5H), 6.73-6.56(m, 4H), 5.46(s, 1H), 5.33(s, 1H), 4.63-4.53(m, 1H), 3.68-3.60(m, 2H), 3.46-3.37(m, 2H), 2.18(s, 1.5H), 2.17(s, 1.5H), 2.11-1.93(m, 4H).

### [Step (d) of Example 33] Production of (5-(2-(2-aminoethoxy)-4-(2-chloro-5-fluorophenoxy)piperidin-1-yl)phenyl)-1,3,4-thiadiazol-2-yl)methyl acetate

(5-(4-(4-(2-Chloro-5-fluorophenoxy)piperidin-1-yl)-2-hydroxyphenyl)-1,3,4-thiadiazol-2-yl)methyl acetate (64 mg) produced in the above step (c) was dissolved in DMF (3 mL), tert-butyl(2-bromoethyl)carbamate (36 mg) and potassium carbonate (55 mg) were added thereto, and the mixture was stirred at room temperature for 24 hours. The solution after the reaction was diluted with water, and then extracted with ethyl acetate. The solution after the extraction was washed with water and saturated brine in this order, and then dried over magnesium sulfate. Furthermore, the solvent was distilled off under reduced pressure, the residue was then dissolved in dichloromethane (1 mL), TFA (150 µL) was added thereto, and the mixture was stirred at room temperature for 6 hours. After the reaction, a saturated aqueous sodium hydrogen carbonate solution was added to the reaction liquid to make the reaction liquid basic, and the reaction liquid was extracted with dichloromethane. The extraction liquid was washed with water and saturated brine in this order, and dried over magnesium sulfate. The solvent was distilled off under reduced pressure, and then the residue was dried under reduced pressure to give the target product (58 mg, yield: 83%).

¹HNMR(500MHz, CDCl₃)δ8.34(d, J = 8.8Hz, 1H), 7.33(dd, J = 5.9, 8.8Hz, 1H), 6.72(dd, J = 2.7, 10.3Hz, 1H), 6.68-6.65(m, 2H), 6.48(d, J = 2.2Hz, 1H), 5.50(s, 2H), 4.61-4.58(m, 1H), 4.21(t, J = 5.2Hz, 2H), 3.65-3.60(m, 2H), 3.43-3.38(m, 2H), 3.28(t, J = 5.2Hz, 2H), 2.16(s, 3H), 2.10-2.00(m, 4H).

### [Example 34] Production of (5-(4-(4-(2-chloro-5-fluorophenoxy)piperidin-1-yl)pyridazine-3-yl)-2-(2-(5-((3aS,4S,6aR)-2-oxohexahydro-1H-thieno[3,4-d]imidazol-4-yl)pentaamide)ethoxy)phenyl)-1,3,4-thiadiazol-2-yl)methyl acetate (Compound No.: NUT-40)

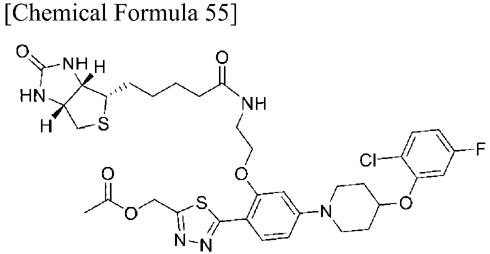

(5-(2-(2-Aminoethoxy)-4-(2-chloro-5-fluorophenoxy)piperidin-1-yl)phenyl)-1,3,4-thiadiazol-2-yl)methyl acetate (55 mg) produced in the same manner as in Example 33 was dissolved in DMF (1 mL), and BiotinNHS (46 mg) was further added thereto, followed by stirring at room temperature for 12 hours. After the reaction, the solvent was distilled off under reduced pressure, and then the residue was purified by column chromatography (eluent: dichloromethane:methanol = 95:5) to give the target product (25 mg, yield: 32%).

¹HNMR(500MHz, CDCl₃)δ8.23(d, J = 8.8Hz, 1H), 7.33(dd, J = 6.1, 8.8Hz, 1H), 6.73(dd, J = 2.7, 10.3Hz, 1H), 6.68-6.58(m, 3H), 5.50(s, 2H), 4.64-4.58(m, 1H), 4.43-4.40(m, 1H), 4.31-4.24(m, 2H), 4.20-4.16(m, 1H), 3.80-3.77(m, 2H), 3.67-3.61(m, 2H), 3.44-3.40(m, 2H), 3.05-3.01(m, 1H), 2.84(dd, J = 4.9, 12.9Hz, 1H), 2.68(d, J = 12.9Hz, 1H), 2.36-2.26(m, 4H), 2.15(s, 3H), 2.05-2.00(m, 4H), 1.70-1.58(m, 4H), 1.43-1.35(m, 2H).

### [Example 35] Production of (5-4-(4-(2-chloro-5-fluorophenoxy)piperidin-1-yl)-2-methoxyphenyl)-1,3,4-thiadiazol-2-yl)methyl acetate (Compound No.: NUT-53)

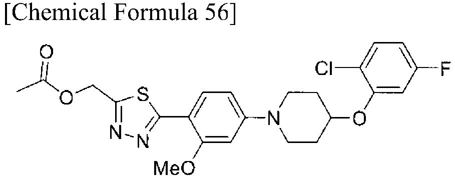

### [Step (a) of Example 35] Production of 4-(4-(2-chloro-5-fluorophenoxy)piperidin-1-yl)-2-methoxybenzohydrazide

Methyl 4-(4-(2-chloro-5-fluorophenoxy)piperidin-1-yl)-2-methoxybenzoate (510 mg) produced in Reference Production Example 15 and hydrazine monohydrate (1.0 mL) were used to perform reaction treatment according to the method described in step (a) of Example 1, thereby giving the target product as a crude product (500 mg, crude yield: 98%).

### [Step (b) of Example 35] Production of 2-(2-(4-(4-(2-chloro-5-fluorophenoxy)piperidin-1-yl)-2-methoxybenzoyl)hydrazinyl)-2-oxoethyl acetate

A portion (400 mg) of the crude product of 4-(4-(2-chloro-5-fluorophenoxy)piperidin-1-yl)-2-methoxybenzohydrazide produced in the above step (a), iPr₂NEt (262 µL) and acetoxyacetyl chloride (130 µL) were used to perform reaction treatment according to the method described in step (b) of Example 1, thereby giving the target product (182 mg).

¹HNMR(500MHz, CDCl₃)δ10.4(d, J = 6.9Hz, NH), 9.29(d, J = 6.6Hz, NH), 8.05(d, J = 8.8Hz, 1H), 7.33(dd, J = 5.9, 8.6Hz, 1H), 6.73-6.62(m, 3H), 6.41(s, 1H), 4.74(s, 2H), 4.63-4.58(m, 1H), 4.03(s, 3H), 3.65-3.61(m, 2H), 3.45-3.41(m, 2H), 2.21(s, 3H), 2.11-2.01(m, 4H).

### [Step (c) of Example 35] Production of (5-4-(4-(2-chloro-5-fluorophenoxy)piperidin-1-yl)-2-methoxyphenyl)-1,3,4-thiadiazol-2-yl)methyl acetate

2-(2-(4-(4-(2-Chloro-5-fluorophenoxy)piperidin-1-yl)-2-methoxybenzoyl)hydrazinyl)-2-oxoethyl acetate (182 mg) produced in the above step (b), phosphorus pentasulfide (180 mg) and THF (4 mL) were used to perform reaction treatment according to the method described in step (c) of Example 1, thereby giving the target product (33 mg, yield: 18%).

¹HNMR(500MHz, CDCl₃)δ8.35(d, J = 8.8Hz, 1H), 7.33(dd, J = 6.1, 8.8Hz, 1H), 6.73(dd, J = 2.7, 10.3Hz, 1H), 6.68-6.64(m, 2H), 6.49(s, 1H), 5.52(s, 2H), 4.63-4.56(m, 1H), 4.00(s, 3H), 3.66-3.61(m, 2H), 3.43-3.39(m, 2H), 2.16(s, 3H), 2.13-2.02(m, 4H).

### [Example 36] Production of (5-4-(4-(2-chloro-5-fluorophenoxy)piperidin-1-yl)-2-methoxyphenyl)-1,3,4-thiadiazol-2-yl)methanol (Compound No.: NUT-54)

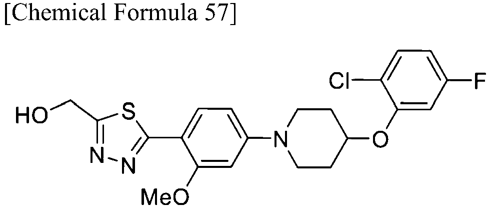

(5-4-(4-(2-Chloro-5-fluorophenoxy)piperidin-1-yl)-2-methoxyphenyl)-1,3,4-thiadiazol-2-yl)methyl acetate (28 mg) produced in the above step (c) of Example 35 and lithium hydroxide monohydrate (6 mg) were used to perform reaction treatment according to the method described in Example 15, thereby giving the target product (20 mg, yield: 78%).

¹HNMR(500MHz, CDCl₃)δ8.29(d, J = 8.8Hz, 1H), 7.33(dd, J = 2.7, 8.8Hz, 1H), 6.72(dd, J = 2.7, 10.0Hz, 1H), 6.68-6.63(m, 2H), 6.47(s, 1H), 5.09(s, 2H), 4.59-4.57(m, 1H), 3.97(s, 3H), 3.64-3.59(m, 2H), 3.41-3.37(m, 2H), 2.10-2.02(m, 4H).

### [Example 37] Production of 5-(4-(4-phenoxypiperidin-1-yl)phenyl)-1,3,4-thiadiazol-2-yl)methanol (Compound No.: NUT-68)

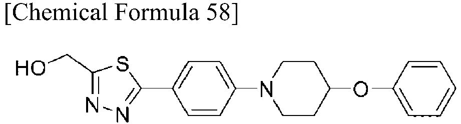

### [Step (a) of Example 37] Production of 4-(4-phenoxypiperidin-1-yl)benzohydrazide

Methyl 4-(4-phenoxy)piperidin-1-yl)benzoate (800 mg) and hydrazine monohydrate (2.5 mL) were used to perform reaction treatment according to the method described in step (a) of Example 1, thereby giving the target product as a crude product (482 mg, crude yield: 60%).

### [Step (b) of Example 37] Production of 2-(2-(4-(4-phenoxypiperidin-1-yl)benzoyl)hydrazinyl)-2-oxoethyl acetate

A portion (465 mg) of the crude product of 4-(4-phenoxypiperidin-1-yl)benzohydrazide produced in the above step (a), iPr₂NEt (312 µL) and acetoxyacetyl chloride (194 µL) were used to perform reaction treatment according to the method described in step (b) of Example 1, thereby giving the target product (466 mg).

¹HNMR(500MHz, CDCl₃)δ9.23(d, J = 5.6Hz, 1H), 8.88(d, J = 5.6Hz, 2H), 7.75(d, 8.6Hz, 2H), 7.30(t, 7.30(t, J = 7.6Hz, 2H), 6.97(t, J = 7.3Hz, 1H), 6.94(d, J = 8.6Hz, 2H), 6.91(d, J = 8.8Hz, 2H), 4.70(s, 2H), 4.56-4.54(m, 1H), 3.67-3.62(m, 2H), 3.34-3.29(m, 2H), 2.20(s, 3H),

2.10-2.06(m,2H), 1.97-1.92(m,2H).

### [Step (c) of Example 37] Production of 5-(4-(4-phenoxypiperidin-1-yl)phenyl)-1,3,4-thiadiazol-2-yl)methyl acetate

2-(2-(4-(4-Phenoxypiperidin-1-yl)benzoyl)hydrazinyl)-2-oxoethyl acetate (411 mg) produced in the above step (b) was dissolved in THF (25 mL), a Lawesson's reagent (445 mg) was added thereto, and the mixture was stirred at 80°C for 12 hours. After the reaction, the solvent was distilled off under reduced pressure, and then the residue was purified by column chromatography (eluent: ethyl acetate:hexane = 1:1) to give the target product (121 mg, yield: 29%).

¹HNMR(500MHz, CDCl₃)δ7.85(d, J = 8.3Hz, 2H), 7.31(t, J = 7.6Hz, 2H), 6.99-6.94(m, 5H), 5.50(s, 2H), 4.58-4.53(m, 1H), 3.67-3.63(m, 2H), 3.34-3.29(m, 2H), 2.17(s, 3H), 2.13-2.08(m, 2H), 1.98-1.94(m, 2H).

### [Step (d) of Example 37] Production of 5-(4-(4-phenoxypiperidin-1-yl)phenyl)-1,3,4-(thiadiazol-2-yl)methanol

5-(4-(4-Phenoxypiperidin-1-yl)phenyl)-1,3,4-thiadiazol-2-yl)methyl acetate (100 mg) produced in the above step (c) and lithium hydroxide monohydrate (51 mg) were used to perform reaction treatment according to the method described in Example 15, thereby giving the target product (88 mg, yield: 99%).

¹HNMR(500MHz, CDCl₃)δ7.84(d, J = 8.8Hz, 2H), 7.31(t, J = 7.6Hz, 1H), 6.98-6.94(m, 5H), 5.09(s, 2H), 4.57-4.54(m, 1H), 3.67-3.62(m, 2H), 3.33-3.28(m, 2H), 2.83(br.s, OH), 2.12-2.08(m, 2H), 1.99-1.93(m, 2H).

### [Example 38] Production of 5-(4-(4-(2-chlorophenoxy)piperidin-1-yl)phenyl)-1,3,4-thiadiazol-2-yl)methanol (Compound No.: NUT-74)

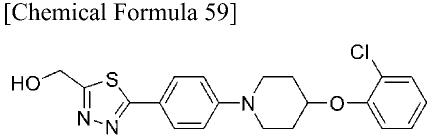

(5-(4-(4-(2-Chlorophenoxy)piperidin-1-yl)phenyl)-1,3,4-thiadiazol-2-yl)methyl acetate (75 mg) produced in step (c) of Example 12 and lithium hydroxide monohydrate (36 mg) were used to perform reaction treatment according to the method described in Example 15, thereby giving the target product (50 mg, yield: 74%).

¹HNMR(500MHz, CDCl₃)δ7.84(d, J = 8.1Hz, 2H), 7.40(dd, J = 1.5, 7.9Hz, 1H), 7.22(dt, J = 1.3, 8.6Hz, 1H), 7.01-6.92(m, 4H), 5.09(s, 2H), 3.68-3.63(m, 2H), 3.39-3.35(m, 2H), 2.82(br.s, OH), 2.10-2.02(m, 4H).

### [Example 39] Production of (5-(4-(4-(2-methylphenoxy)piperidin-1-yl)phenyl)-1,3,4-thiadiazol-2-yl)methanol (Compound No.: NUT-75)

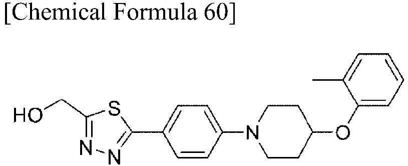

### [Step (a) of Example 39] Production of 4-(4-(2-methylphenoxy)piperidin-1-yl)benzohydrazide

Methyl 4-(4-(2-methylphenoxy)piperidin-1-yl)benzoate (750 mg) produced in Reference Production Example 11 and hydrazine monohydrate (2.5 mL) were used to perform reaction treatment according to the method described in step (a) of Example 1, thereby giving the target product as a crude product (485 mg, crude yield: 65%).

### [Step (b) of Example 39] Production of 2-(2-(4-(4-(2-methylphenoxy)piperidin-1-yl)benzoyl)hydrazinyl)-2-oxoethyl acetate

A portion (485 mg) of the crude product of 4-(4-(2-methylphenoxy)piperidin-1-yl)benzohydrazide produced in the above step (a), iPr₂NEt (312 µL) and acetoxyacetyl chloride (194 µL) were used to perform reaction treatment according to the method described in step (b) of Example 1, thereby giving the target product (380 mg).

¹HNMR(500MHz, CDCl₃)δ9.16(br.s, NH), 8.80(br.s, NH), 7.75(d, J = 8.8Hz, 2H), 7.18-7.15(m, H), 6.92(d, J = 9.1Hz, 2H), 6.89-6.86(m, 2H), 4.72(s, 2H), 4.59-4.57(m, 1H), 3.63-3.58(m, 2H), 3.39-3.35(m, 2H), 2.25(s, 3H), 2.21(s, 3H), 2.09-2.04(m, 2H), 1.99-1.95(m, 2H).

### [Step (c) of Example 39] Production of 5-(4-(4-(2-methylphenoxy)piperidin-1-yl)phenyl)-1,3,4-thiadiazol-2-yl)methyl acetate

2-(2-(4-(4-(2-Methylphenoxy)piperidin-1-yl)benzoyl)hydrazinyl)-2-oxoethyl acetate (340 mg) produced in the above step (b) and a Lawesson's reagent (355 mg) were used to perform reaction treatment according to the method described in step (c) of Example 37, thereby giving the target product (380 mg, yield: 18%).

¹HNMR(400MHz, CDCl₃)δ7.85(d, J = 9.0Hz, 2H), 7.18-7.15(m, 2H), 6.98(d, J = 9.0Hz, 2H), 6.91-6.87(m, 2H)5.50(s, 2H), 4.60-4.57(m, 1H), 3.63-3.58(m, 2H), 3.40-3.35(m, 2H), 2.25(s, 3H), 2.18(s, 3H), 2.12-2.06(m, 2H), 2.03-1.96(m, 2H).

### [Step (d) of Example 39] Production of (5-(4-(4-(2-methylphenoxy)piperidin-1-yl)phenyl)-1,3,4-thiadiazol-2-yl)methanol

5-(4-(4-(2-Methylphenoxy)piperidin-1-yl)phenyl)-1,3,4-thiadiazol-2-yl)methyl acetate (60 mg) produced in the above step (c) and lithium hydroxide monohydrate (30 mg) were used to perform reaction treatment according to the method described in Example 15, thereby giving the target product (48 mg, yield: 89%).

¹HNMR(400MHz, CDCl₃)δ7.85(d, J = 8.2Hz, 2H), 7.18-7.15(m, 2H), 6.98(d, J = 8.8Hz, 2H), 6.90-6.87(m, 2H), 5.09(s, 2H), 4.60-4.57(m, 1H), 3.62-3.57(m, 2H), 3.39-3.33(m, 2H), 2.63(br.s, OH), 2.25(s, 3H), 2.12-2.06(m, 2H), 2.03-1.96(m, 2H).

### [Example 40] Production of (5-(4-(4-(2-fluorophenoxy)piperidin-1-yl)phenyl)-1,3,4-thiadiazol-2-yl)methanol (Compound No.: NUT-76)

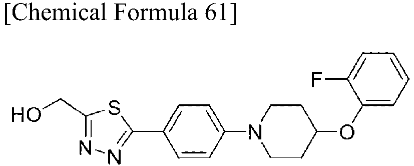

### [Step (a) of Example 40] Production of 4-(4-(2-fluorophenoxy)piperidin-1-yl)benzohydrazide

Methyl 4-(4-(2-fluorophenoxy)piperidin-1-yl)benzoate (1.0 g) produced in Reference Production Example 12 and hydrazine monohydrate (3.5 mL) were used to perform reaction treatment according to the method described in step (a) of Example 1, thereby giving the target product as a crude product (510 mg, crude yield: 51%).

### [Step (b) of Example 40] Production of 2-(2-(4-(4-(2-fluorophenoxy)piperidin-1-yl)benzoyl)hydrazinyl)-2-oxoethyl acetate

A portion (494 mg) of the crude product of 4-(4-(2-fluorophenoxy)piperidin-1-yl)benzohydrazide produced in the above step (a), iPr₂NEt (312 µL) and acetoxyacetyl chloride (194 µL) were used to perform reaction treatment according to the method described in step (b) of Example 1, thereby giving the target product (537 mg).

¹HNMR(500MHz, CDCl₃)39.09(br.s, NH), 8.73(br.s, NH), 7.74(d, J = 8.8Hz, 2H), 7.12-7.02(m, 3H), 6.99-6.94(m, 1H), 6.92(d, J = 6.9Hz, 2H), 4.72(s, 2H), 4.54-4.50(m, 1H), 3.70-3.66(m, 2H), 3.33-3.29(m, 2H), 2.21(s, 3H), 2.09-2.05(m, 2H), 1.99-1.92(m, 2H).

### [Step (c) of Example 40] Production of 5-(4-(4-(2-fluorophenoxy)piperidin-1-yl)phenyl)-1,3,4-thiadiazol-2-yl)methyl acetate

2-(2-(4-(4-(2-Fluorophenoxy)piperidin-1-yl)benzoyl)hydrazinyl)-2-oxoethyl acetate (500 mg) produced in the above step (b) and a Lawesson's reagent (518 mg) were used to perform reaction treatment according to the method described in step (c) of Example 37, thereby giving the target product (130 mg, yield: 26%).

¹HNMR(500MHz, CDCl₃)δ7.84(d, J = 8.8Hz, 2H), 7.13-7.03(m, 3H), 7.00-6.93(m, 3H), 5.50(s, 3H), 4.55-4.49(m, 1H), 3.71-3.66(m, 2H), 3.23-3.27(m, 2H), 2.17(s, 3H), 2.12-2.08(m, 2H), 2.01-1.96(m, 2H).

### [Step (d) of Example 40] Production of (5-(4-(4-(2-fluorophenoxy)piperidin-1-yl)phenyl)-1,3,4-thiadiazol-2-yl)methanol

5-(4-(4-(2-Fluorophenoxy)piperidin-1-yl)phenyl)-1,3,4-thiadiazol-2-yl)methyl acetate (100 mg) produced in the above step (c) and lithium hydroxide monohydrate (49 mg) were used to perform reaction treatment according to the method described in Example 15, thereby giving the target product (73 mg, yield: 82%).

¹HNMR(500MHz, CDCl₃)δ7.82(d, J = 8.4Hz, 2H), 7.11-7.01(m, 3H), 7.03-6.89(m, 3H), 5.07(s, 2H), 3.68-3.65(m, 2H), 3.29-3.25(m, 2H), 2.12-2.04(m, 2H), 2.01-1.91(m, 2H).

### [Example 41] Production of (5-(4-(4-(2-chloro-5-fluorophenoxy)piperidin-1-yl)phenyl)-1,3,4-thiadiazol-2-yl)methanamine (Compound No.: NUT-79)

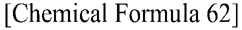

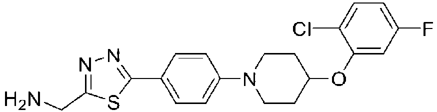

### [Step (a) of Example 41] Production of tert-butyl(2-(2-(4-(4-(2-chloro-5-fluorophenoxy)piperidin-1-yl)carbonyl)hydrazinyl)-2-oxoethyl)carbamate

4-(4-(2-Chloro-5-fluorophenoxy)piperidin-1-yl)benzohydrazide (1.0 g) produced in step (a) of Example 11 and (tert-butoxycarbonyl)glycine (480 mg) were dissolved in DMF (8 mL), Et₃N (760 µL) and HATU (1.0 g) were added thereto, and the mixture was stirred at room temperature for 24 hours. The solution after the reaction was diluted with water, and then extracted with ethyl acetate. The solution after the extraction was washed with water and saturated brine in this order, and then dried over magnesium sulfate. Furthermore, the solvent was distilled off under reduced pressure, and then the residue was purified by column chromatography (eluent: ethyl acetate) to give the target product (1.2 g, yield: 85%).

¹HNMR(500MHz, CDCl₃)δ9.09(br.s, NH), 8.68(br.s, NH), 7.74(d, J = 8.8Hz, 2H), 7.33(dd, J = 6.4, 8.8Hz, 1H), 6.90(d, J = 8.3Hz, 2H), 6.71(d, J = 10.3Hz, 1H), 6.67(dt, J = 2.7, 8.6Hz, 1H), 5.25(br.d, NH), 4.61-4.54(m, 1H), 3.98(d, J = 5.6Hz, 2H), 3.64-3.60(m, 2H), 3.40-3.37(m, 2H), 2.11-1.94(m, 4H), 1.48(s, 9H).

### [Step (b) of Example 41] Production of tert-butyl((5-(4-(4-(2-chloro-5-fluorophenoxy)piperidin-1-yl))-1,3,4-thiadiazol-2-yl)methyl)carbamate

Tert-butyl(2-(2-(4-(4-(2-chloro-5-fluorophenoxy)piperidin-1-yl)carbonyl)hydrazinyl)-2-oxoethyl)carbamate (1.0 g) produced in the above step (a) and a Lawesson's reagent (850 mg) were used to perform reaction treatment according to the method described in step (c) of Example 37, thereby giving the target product (587 mg, yield: 59%).

¹HNMR(500MHz, CDCl₃)δ7.83(d, J = 8.8Hz, 2H), 7.33(t, J = 6.6Hz, 1H), 6.97(d, J = 8.6Hz, 2H), 6.72(d, J = 10.0Hz, 1H), 6.67(t, J = 8.3Hz, 1H), 6.30(br.s, NH), 4.72(d, J = 5.2Hz, 1H), 4.62-4.52(m, 1H), 3.68-3.57(m, 2H), 3.42-3.32(m, 2H), 2.15-1.97(m, 4H), 1.49(s, 9H).

### [Step (c) of Example 41] Production of 5-(4-(4-(2-chloro-5-fluorophenoxy)piperidin-1-yl)phenyl)-1,3,4-thiadiazol-2-yl)methanamine

Tert-butyl((5-(4-(4-(2-chloro-5-fluorophenoxy)piperidin-1-yl))-1,3,4-thiadiazol-2-yl)methyl)carbamate (580 mg) produced in the above step (b) was dissolved in dichloromethane (5 mL), TFA (216 µL) was added thereto, and the mixture was stirred at room temperature for 8 hours. After the reaction, a saturated aqueous sodium hydrogen carbonate solution was added to the reaction liquid to make the reaction liquid basic, and the reaction liquid was extracted with dichloromethane. The extraction liquid was washed with water and saturated brine in this order, and dried over magnesium sulfate. The solvent was distilled off under reduced pressure, and then the residue was dried under reduced pressure to give the target product (339 mg, yield: 72%).

¹HNMR(500MHz, CDCl₃)δ7.81(d, J = 8.8Hz, 2H), 7.31(t, J = 8.6Hz, 1H), 6.95(d, J = 8.6Hz, 2H), 6.71(d, J = 10.3Hz, 1H), 6.64(dt, J = 2.5, 8.8Hz, 1H), 4.61-4.52(m, 1H), 4.28(s, 2H), 3.61-3.57(m, 2H), 3.42-3.34(m, 2H), 2.13-1.87(m, 4H).

### [Example 42] Production of N-((5-(4-(4-(2-chloro-5-fluorophenoxy)piperidin-1-yl)phenyl)-1,3,4-thiadiazol-2-yl)methyl)isobutylamide (Compound No.: NUT-52)

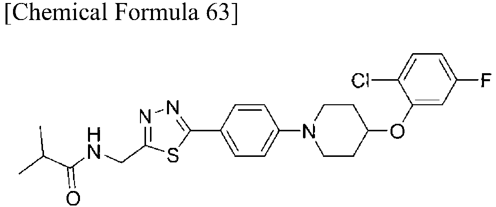

(5-(4-(4-(2-Chloro-5-fluorophenoxy)piperidin-1-yl)phenyl)-1,3,4-thiadiazol-2-yl)methanamine (42 mg) produced in the same manner as in Example 41, iPr₂NEt (21 µL), and isobutyl chloride (81 µL) were used to perform reaction treatment according to the method described in Example 20, thereby giving the target product (8 mg, yield: 17%).

¹HNMR(500MHz, CDCl₃)δ7.82(d, J = 8.6Hz, 2H), 7.33(dd, J = 6.4, 8.8Hz, 1H), 6.96(d, J = 8.8Hz, 2H), 6.72(dd, J = 2.5, 10.0Hz, 1H), 6.66(dt, J = 2.7, 8.1Hz, 1H), 6.46(t, J = 5.2Hz, NH), 4.83(d, J = 5.9Hz, 2H), 4.62-4.55(m, 1H), 3.65-3.60(m, 2H), 3.41-3.36(m, 2H), 2.46(sept, J = 6.9Hz, 1H), 2.11-2.00(m, 4H), 1.20(d, J = 6.9Hz, 6H).

### [Example 43] Production of N-((5-(4-(4-(2-chloro-5-fluorophenoxy)piperidin-1-yl)phenyl)-1,3,4-thiadiazol-2-yl)methyl)acetamide (Compound No.: NUT-80)

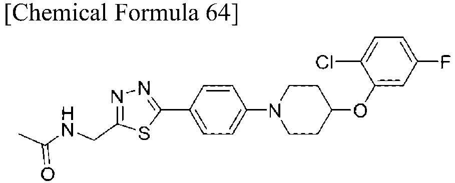

(5-(4-(4-(2-Chloro-5-fluorophenoxy)piperidin-1-yl)phenyl)-1,3,4-thiadiazol-2-yl)methanamine (42 mg) produced in the same manner as in Example 41 was dissolved in dichloromethane (4.0 mL), acetic anhydride (11 µL) was added thereto, and the mixture was stirred at room temperature for 12 hours. After the reaction, the solvent was distilled off under reduced pressure, and then the residue was purified by column chromatography (eluent: ethyl acetate) to give the target product (16 mg, yield: 35%).

¹HNMR(500MHz, CDCl₃)δ7.79(d, J = 8.8Hz, 2H), 7.1(dd, J = 5.9, 8.6Hz, 1H), 6.94(d, J = 8.8Hz, 2H), 6.80(t, J = 5.2Hz, NH), 6.70(dd, J = 2.7, 10.0Hz, 1H), 6.66-6.63(m, 1H), 4.80(d, J = 5.9Hz, 2H), 4.59-4.53(m, 1H), 3.63-3.58(m, 2H), 3.39-3.34(m, 2H), 2.10-1.99(m, 7H).

### [Example 44] Production of 5-(4-(4-(2-(trifluoromethyl)phenoxy)piperidin-1-yl)phenyl)-1,3,4-thiadiazol-2-yl)methyl acetate (Compound No.: NUT-83)

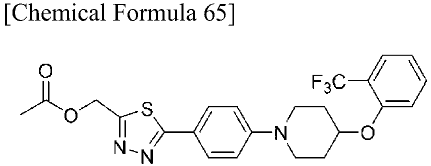

### [Step (a) of Example 44] Production of 4-(4-(2-(trifluoromethyl)phenoxy)piperidin-1-yl)benzohydrazide

Methyl 4-(4-(2-(trifluoromethyl)phenoxy)piperidin-1-yl)benzoate (334 g) produced in Reference Production Example 13 and hydrazine monohydrate (1.5 mL) were used to perform reaction treatment according to the method described in step (a) of Example 1, thereby giving the target product as a crude product (273 mg, crude yield: 82%).

### [Step (b) of Example 44] Production of 2-(2-(4-(4-(trifluoromethyl)phenoxy)piperidin-1-yl)benzoyl)hydrazinyl)-2-oxoethyl acetate

A portion (270 mg) of the crude product of 4-(4-(2-(trifluoromethyl)phenoxy)piperidin-1-yl)benzohydrazide produced in the above step (a), iPr₂NEt (148 µL) and acetoxyacetyl chloride (93 µL) were used to perform reaction treatment according to the method described in step (b) of Example 1, thereby giving the target product (300 mg).

¹HNMR(500MHz, CDCl₃)δ9.41(br.s, NH), 9.12(br.s, NH), 7.76(d, J = 8.3Hz, 2H), 7.59(d, J = 7.4Hz, 1H), 7.48(t, J = 7.6Hz, 1H), 7.04-7.00(m, 2H), 6.87(d, J = 8.6Hz, 2H), 4.75-4.69(m, 1H), 4.68(s, 2H), 3.58-3.50(m, 2H), 3.47-3.39(m, 2H), 2.17(s, 3H).

### [Step (c) of Example 44] Production of 5-(4-(4-(2-(trifluoromethyl)phenoxy)piperidin-1-yl)phenyl)-1,3,4-thiadiazol-2-yl)methyl acetate

2-(2-(4-(4-(trifluoromethyl)phenoxy)piperidin-1-yl)benzoyl)hydrazinyl)-2-oxoethyl acetate (300 mg) produced in the above step (b) and a Lawesson's reagent (278 mg) were used to perform reaction treatment according to the method described in step (c) of Example 37, thereby giving the target product (168 mg, yield: 56%).

¹HNMR(500MHz, CDCl₃)δ7.85(d, J = 8.6Hz, 2H), 7.60(d, J = 7.8Hz, 1H), 7.50(t, J = 8.1Hz, 1H), 7.03-7.00(m, 4H), 5.50(s, 2H), 4.80-4.71(m, 1H), 3.59-3.54(m, 2H), 3.47-3.43(m, 2H), 2.17(s, 3H), 2.14-2.03(m, 4H).

### [Example 45] Production of (5-(4-(4-(2-(trifluoromethyl)phenoxy)piperidin-1-yl)phenyl)-1,3,4-thiadiazol-2-yl)methanol (Compound No.: NUT-82)

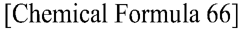

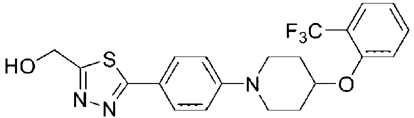

5-(4-(4-(2-(Trifluoromethyl)phenoxy)piperidin-1-yl)phenyl)-1,3,4-thiadiazol-2-yl)methyl acetate (148 mg) produced in Example 44 and lithium hydroxide monohydrate (65 mg) were used to perform reaction treatment according to the method described in Example 15, thereby giving the target product (100 mg, yield: 74%).

¹HNMR(500MHz, CDCl3)δ7.84(d, J = 8.8Hz, 2H), 7.60(d, J = 7.1Hz, 1H), 7.50(t, J = 7.6Hz, 1H), 7.04-7.00(m, 2H), 6.97(d, J = 8.8Hz, 2H), 5.09(s, 2H), 4.76-4.71(m, 1H), 3.59-3.53(m, 2H), 3.46-3.41(m, 2H), 2.75(br.s, OH), 2.14-2.01(m, 2H).

### [Example 46] Production of 5-(4-(4-(2-Chloro-5-fluorophenoxy)piperidin-1-yl)phenyl)-1,3,4-thiadiazol-2-yl)methyl glycinate hydrochloride (Compound No.: NUT-88 (ADI-88))

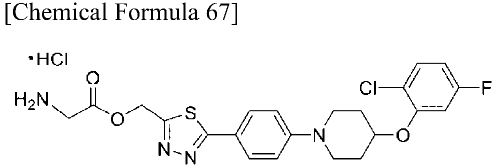

### [Step (a) of Example 46] Production of 5-(4-(4-(2-chloro-5-fluorophenoxy)piperidin-1-yl)phenyl)-1,3,4-thiadiazol-2-yl) methyl (tert-butoxycarbonyl)glycinate

(5-(4-(4-(2-Chloro-5-fluorophenoxy)piperidin-1-yl)phenyl)-1,3,4-thiadiazol-2-yl)methanol (80 mg) produced in the same manner as in Example 18 was dissolved in DMF (3 mL), (tert-butoxycarbonyl)glycine (57 mg), DMAP (41 mg), and EDCI (61 mg) were added thereto, and the mixture was stirred at room temperature for 24 hours. The solution after the reaction was diluted with water, and then extracted with ethyl acetate. The solution after the extraction was washed with water and saturated brine in this order, and then dried over magnesium sulfate. Furthermore, the solvent was distilled off under reduced pressure, and then the residue was purified by column chromatography (eluent: ethyl acetate:hexane = 1:1) to give the target product (87 mg, yield: 79%).

¹HNMR(500MHz, CDCl₃)δ7.83(d, J = 9.1Hz, 2H), 7.32(dt, J = 6.1, 8.8Hz, 1H), 6.97(d, J = 8.8Hz, 2H), 6.72(dd, J = 2.7, 10.3Hz, 1H), 4.62-4.54(m, 1H), 4.02(d, J = 5.7Hz, 2H), 3.64-3.60(m, 2H), 3.42-3.37(m, 2H), 2.10-2.01(m, 4H), 1.46(s, 9H).

### [Step (b) of Example 46] Production of (5-(4-(4-(2-chloro-5-fluorophenoxy)piperidin-1-yl)phenyl)-1,3,4-thiadiazol-2-yl)methyl glycinate hydrochloride

A solution of hydrogen chloride in 1,4-dioxane (4 M, 1.0 mL) was added to 5-(4-(4-(2-chloro-5-fluorophenoxy)piperidin-1-yl)phenyl)-1,3,4-thiadiazol-2-yl) methyl (tert-butoxycarbonyl)glycinate (87 mg) produced in the above step (a), and the mixture was stirred at room temperature for 2 hours. After the reaction, the solvent was distilled off under reduced pressure, and then the residue was dried under reduced pressure to give the target product (76 mg, yield: 99%).

¹HNMR(500MHz, DMSO-d₆)δ8.42(br.s, 3H), 7.80(d, J = 8.8Hz, 2H), 7.47(dd, J = 6.4, 8.3Hz, 1H), 7.28(d, J = 10.5Hz, 1H), 7.12(d, J = 8.8Hz, 2H), 6.83(t, J = 8.1Hz, 1H), 5.65(s, 2H), 4.86-4.78(m, 1H), 3.93(d, J = 5.4Hz, 2H), 3.37-3.33(m, 4H), 2.08-1.99(m, 2H), 1.80-1.70(m, 2H).

### [Example 47] Production of (5-(4-(4-(2-chloro-5-fluorophenoxy)piperidin-1-yl)phenyl)-1,3,4-thiadiazol-2-yl)methyl L-valine ester hydrochloride (Compound No.: NUT-89 (ADI-89))

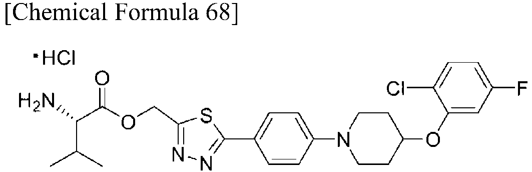

### [Step (a) of Example 47] Production of(5-(4-(4-(2-chloro-5-fluorophenoxy)piperidin-1-yl)phenyl)-1,3,4-thiadiazol-2-yl)methyl (tert-butoxycarbonyl)-L-valine ester

(5-(4-(4-(2-Chloro-5-fluorophenoxy)piperidin-1-yl)phenyl)-1,3,4-thiadiazol-2-yl)methanol (160 mg) produced in the same manner as in Example 18 was dissolved in DMF (6 mL), (tert-butoxycarbonyl)L-valine (140 mg), DMAP (82 mg), and EDCI (122 mg) were added thereto, and the mixture was stirred at room temperature for 24 hours. The solution after the reaction was diluted with water, and then extracted with ethyl acetate. The solution after the extraction was washed with water and saturated brine in this order, and then dried over magnesium sulfate. Furthermore, the solvent was distilled off under reduced pressure, and then the residue was purified by column chromatography (eluent: ethyl acetate:hexane = 1:1) to give the target product (185 mg, yield: 79%).

¹HNMR(500MHz, CDCl₃)δ7.85(d, J = 8.6Hz, 2H), 7.33(dt, J = 6.4, 8.8Hz, 1H), 6.98(d, J = 8.6Hz, 2H), 6.73(dd, J = 2.5, 10.3Hz, 1H), 6.67(dt, J = 2.5, 7.8Hz, 1H), 5.61(d, J = 13.0Hz, 1H), 5.52(d, J = 13.5Hz, 1H), 5.00(d, J = 8.8Hz, NH), 4.62-4.56(m, 1H), 4.35-4.30(m, 1H), 3.67-3.60(m, 2H), 3.44-3.37(m, 2H), 2.25-2.15(m, 1H), 2.12-1.98(m, 4H), 1.46(s, 9H), 0.99(d, J = 6.9Hz, 3H), 0.91(d, J = 6.9Hz, 3H).

### [Step (b) of Example 47] Production of (5-(4-(4-(2-chloro-5-fluorophenoxy)piperidin-1-yl)phenyl)-1,3,4-thiadiazol-2-yl)methyl L-valine ester hydrochloride

A solution of hydrogen chloride in 1,4-dioxane (4 M, 2.5 mL) was added to 5-(4-(4-(2-chloro-5-fluorophenoxy)piperidin-1-yl)phenyl)-1,3,4-thiadiazol-2-yl) methyl (tert-butoxycarbonyl)-L-valine ester (185 mg) produced in the above step (a), and the mixture was stirred at room temperature for 2 hours. After the reaction, the solvent was distilled off under reduced pressure, and then the residue was dried under reduced pressure to give the target product (150 mg, yield: 98%).

¹HNMR(500MHz, DMSO-d₆)δ8.62(br.s, 3H), 7.82(d, J = 8.6Hz, 2H), 7.47(t, J = 6.9Hz, 1H), 7.28(d, J = 11.0Hz, 1H), 7.14(d, J = 8.1Hz, 2H), 6.83(t, J = 8.4Hz, 1H), 5.72(d, J = 13.2Hz, 1H), 5.65(d, J = 13.4Hz, 1H), 4.85-4.78(m, 1H), 4.04-3.97(m, 1H), 3.41-3.33(m, 2H), 2.27-2.16(m, 1H), 2.09-2.00(m, 2H), 1.82-1.72(m, 2H), 0.99(d, J = 6.9Hz, 3H), 0.95(d, J = 6.9Hz, 3H).

### Reference Examples

The structures of the compounds (NUT-41 and 42) of Reference Examples used in Test Examples described later are as follows.

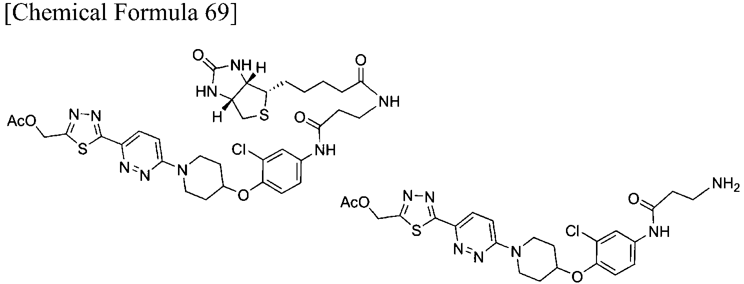

In the following Test Examples, the compounds (ADI derivatives, NUT compounds) produced above were also used for evaluation.

### [Test Example 1: Evaluation of Intracellular Accumulated Amount of S403 Phosphorylated p62 by ADI Derivative Treatment]

### (1) Experimental Method

As cells, N2a (derived from mouse neuroblastomas) and HEK293 (derived from human embryonic kidney cells) were used. The cells were seeded in a 6-well plate, and on the next day, various ADI derivatives (1 µM) were added to a medium and cultured for 24 hours. A control group without addition of ADI derivatives was provided. As the medium, DMEM (high glucose; WAKO) + 10% FBS (gibco) were used. After 24 hours, the medium was removed, and the cells were washed with PBS, and then 1 mL of PBS was added thereto, and the cells were detached by pipetting. The cells were put into a 1.5-mL tube, and centrifuged at 1200 rpm for 10 seconds to collect the cells. The supernatant was removed, the cells were suspended in 100 µL of PBS, sonicated (BRANSON, 5 W, 10 seconds), mixed with 2 × SDS-sample buffer at 1:1, and 20 µL was subjected to SDS-PAGE for Western blotting. As primary antibodies, a home-made anti-S403-P-p62 monoclonal antibody (clone # 4F6, Matsumoto et al., Mol Cell. 44 (2) 279-289, 2011) and an anti-p62 antibody (MBL, PM045) were used.

### (2) Results

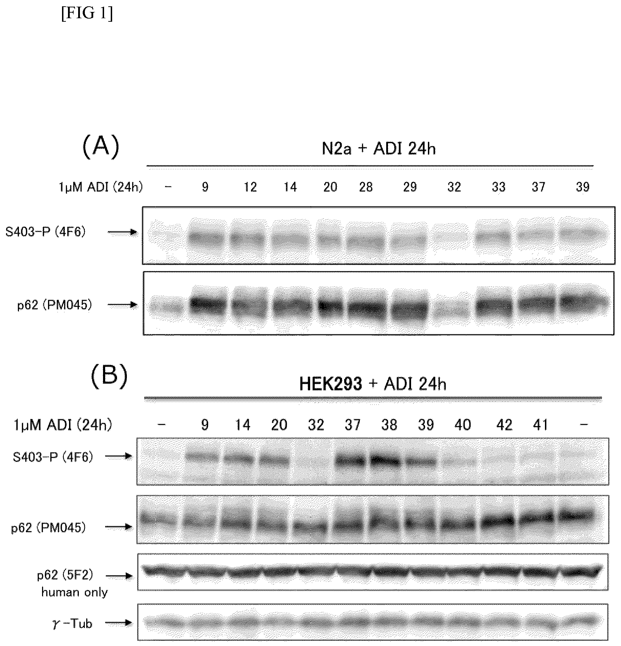

The results are shown in FIG. 1. (A) shows results of N2a cells, and (B) shows results of HEK293 cells. Both (A) and (B) show increases in total amount of p62 protein and accumulated amount of S403 phosphorylated p62 protein in the cells treated with ADI derivatives as compared with the control group. From these results, it was revealed that the ADI derivatives have an activity of promoting S403 phosphorylation of p62 protein.

### [Test Example 2: Study on Phosphorylation Promoting Mechanism of ADI Derivative]

### (1) Experimental Method

As the cells, used were mouse neuroblast cells (N2a+RG-p62 cells), which constantly and stably expressed mouse p62 protein (RFP-GFP-p62) in which red fluorescent proteins (RFP) and green fluorescent proteins (GFP) were linearly linked. The N2a+RG-p62 cells were prepared by introducing a pRFP-GFP-mp62 vector, which was prepared by introducing GFP-mp62 (Matsumoto et al., Scientific Reports 8 (1) 2018) into pRFP-C1-DEST (Matsumoto et al., Human molecular genetics 24 (15) 4429-4442, 2015) using the gateway system, into N2a cells using LipofectAmine3000 and selecting stably expressing lines. The ADI derivatives used were ADI-14 and ADI-29. The N2a+RG-p62 cells were seeded in 6-well plates and, on the next day, cultured for 24 hours with treatment with the ADI derivative (1 µM) alone, co-treatment with the ADI derivative (1 µM) and bafilomycin A (0.5 µM, hereinafter "BafA"), and co-treatment with the ADI derivative (1 µM) and BX795 (0.5 µM). As controls, a non-treated group, a BafA-treated group, and a BX795-treated group were provided. In the same manner as in Test Example 1, the cells were collected after 24 hours to prepare a cellular extraction liquid, and the intracellular accumulated amount of S403 phosphorylated p62 contained in the extraction liquid was evaluated by Western blotting. The same antibodies as in Test Example 1 were used. BafA is a compound having an activity of inhibiting the degradation of autophagosomes by lysosomes by inhibiting fusion of the autophagosomes and the lysosomes, and BX795 is a compound having a TBK1 inhibitory activity.

### (2) Results

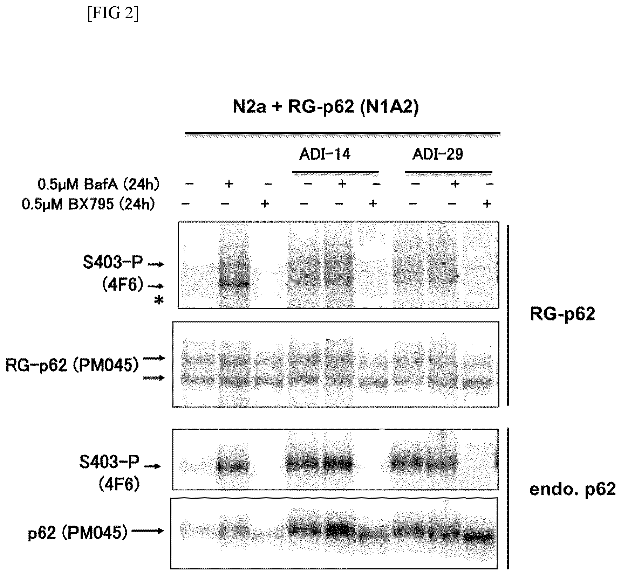

The results are shown in FIG. 2. In the figure, "RG-p62" represents RFP-GFP-p62 protein stably expressed by introducing the vector, and "endo.p62" represents endogenous p62 protein. As in Test Example 1, the accumulated amount of S403 phosphorylated p62 increased in the ADI-14-treated group and the ADI-29-treated group. Since phosphorylated p62 was constantly degraded, the degradation was inhibited by the addition of BafA in the control group, the ADI-14-treated group, and the ADI-29-treated group, and S403 phosphorylated p62 was accumulated in the cells. When the TBK1 inhibitor (BX795) was added, S403 phosphorylated p62 was not accumulated in any of the control group, the ADI-14 treated group, and the ADI-29 treated group. That is, it was shown that the activity of the ADI derivatives for promoting S403 phosphorylation of p62 is suppressed by the TBK1 inhibitor. Therefore, it was suggested that ADI promotes the S403 phosphorylation of p62 by inducing the activation of TBK1.

### [Test Example 3: Study on Gene Expression-Induced by ADI Derivative]

### (1) Experimental Method

### (1-1) Cell Test

N2a cells were seeded on a 6-well plate, and, on the next day, ADI-14 and ADI-29 were each added at a final concentration of 1 µM, and the cells were cultured for 24 hours. After 24 hours, the cells were collected, total RNA was extracted using TRI Reagent (trade name, Molecular Research Center Inc.), 2 µg of RNA was reverse-transcribed using PRIME script MAX (TAKARA) to synthesize cDNA, and quantitative PCR was performed using THUNDERBIRD SYBR qPCR Mix (TOYOBO). Seven types of genes to be measured were used, including Atf3 (Activating transcription factor 3), Ddit3 (DNA damage-inducible transcript 3), Gadd45a (Growth Arrest and DNA Damage-inducible 45), Nfil3 (Nuclear factor, interleukin 3 regulated), Ppp1r15a (Protein phosphatase 1 regulatory subunit 15A), p62/Sqstm1 (p62/sequestosome1), and Trib3 (Tribbles Pseudokinase 3). Genes estimated to be increased in expression by ADI treatment by performing RNA-seq of ADI-treated N2a cells and untreated N2a cells and comprehensively analyzing genes having a large difference in expression level were selected as the genes to be measured. The expression level of each gene (mRNA) was calculated by the ΔΔCt method as a relative mRNA level to the control (without ADI derivative treatment) based on the mRNA expression level of the Gapdh gene.

### (1-2) Animal Test

ADI-14 and ADI-29 dissolved in DMSO were each dissolved in olive oil (containing DMSO at a final concentration of 1%), and 50 µg of each solution was forcibly orally administered to C57BL/6J mice every other day for 2 weeks. After the administration period, the mice were euthanized, and the hippocampus was collected. Total RNA was extracted, cDNA was synthesized, and quantitative PCR was performed, in the same manner as in (1) above. Seven types of genes to be measured were used, including Atf3, Ddit3, Gadd45a, Nfil3, Ppp1r15a, p62/Sqstm1, and Trib3 of mice. The expression level of each gene (mRNA) was also calculated by the same method as in the above (1).

The primers used for the quantitative PCR are shown below.
Aft3_RT_F: GCTGGAGTCAGTTACCGTCAA (SEQ ID NO: 2)
Aft3_RT_R: CGCCTCCTTTTCCTCTCAT (SEQ ID NO: 3)
Ddit3_RT_F: TCACACGCACATCCCAAA (SEQ ID NO: 4)
Ddit3_RT_R: CCTAGTTCTTCCTTGCTCTTCC (SEQ ID NO: 5)
Gadd45a _RT_F: CCACGCTGATGCAAGGATTA (SEQ ID NO: 6)
Gadd45a_RT_R: TTCTTCAGGCTCACCTCTCT (SEQ ID NO: 7)
Nfil3_RT_F: CTTTCTTTTCCCCCTCACG (SEQ ID NO: 8)
Nfil3_RT_R: CAGGAGCCTTTCATGGGTTA (SEQ ID NO: 9)
Ppp1r15a_RT_F: GACACAGAGGAAGAGGAAGATG (SEQ ID NO: 10)
Ppp1r15a_RT_R: TAGCAGGAGTGGAAGAGGAA (SEQ ID NO: 11)
p62/Sqstm1_RT_F: GAAGCTGCCCTATACCCACA (SEQ ID NO: 12)
p62/Sqstm1_RT_R: TGGGAGAGGGACTCAATCAG (SEQ ID NO: 13)
Trib3_RT_F: CGCTTTGTCTTCAGCAACTGT (SEQ ID NO: 14)
Trib3_RT_R: TCATCTGATCCAGTCATCACG (SEQ ID NO: 15)
Gapdh_RT_F: ATGGTGAAGGTCGGTGTGA (SEQ ID NO: 16)
Gapdh_RT_F: AATCTCCACTTTGCCACTGC (SEQ ID NO: 17)

### (2) Results

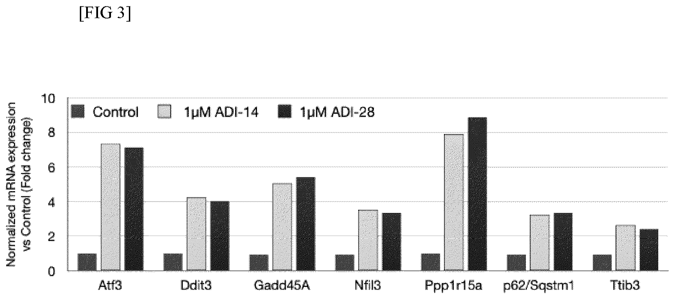

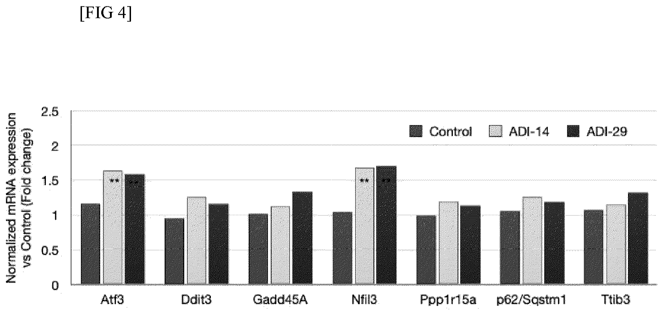

The results of the cell test are shown in FIG. 3. In all the measured genes, the expression levels were significantly increased by the ADI derivative treatment. The results of the animal test are shown in FIG. 4. Oral administration of the ADI derivatives significantly increased the expression levels of Atf3 and Nfil3 of mRNA adjusted from the hippocampus of the mouse brain. These results suggested that the ADI derivatives promote aggrephagy by selective autophagy induction accompanied by expression of the genes Atf3 and Nfil3.

### [Test Example 4: Study on Effect of ADI Derivative for Promoting Aggrephagy by Using Tau Aggregation Cell Line]

### (1) Experimental Method

Tau aggregation cell lines (G1F1B, G1E3B) were self-made and used. Tau aggregation cell lines (G1F1B, G1E3B) are cell lines in which fibrillized GFP-hTau-2N4R-P301L proteins obtained by introducing tau seeds into HEK293 cells stably expressing GFP-hTau-2N4R-P301L in which GFP is fused to the N-terminus of full-length human tau protein (2N4R) containing P301L mutation, which is a mutation of FTDP-17 (familial frontotemporal dementia associated with parkinsonism linked to chromosome 17), constantly maintain the tau aggregates when the tau seeds are delivered to daughter cells during cellular division, and are cells prepared with the HEK293 cells according to the method for preparing a tau aggregation cell line of N2a cells described in Matsumoto et al. (International Journal of Molecular Sciences 19 (5) 2018). The tau aggregation cell lines were seeded in a 12-well plate at 1 × 10⁵ cells/2 mL per well, and cultured in a DMEM + 10% FBS medium. On the next day, ADI-14 was added to three wells to attain a final concentration of 1 µM, DMSO was added to three wells as a control, and nine fixed points per well were photographed once an hour with IncuCyto ZOOM (ESSEN Bioscience) built in a 5% CO₂ incubator to measure the number of aggregates (A) and cell densities (% confluency; B) over time.

### (2) Results

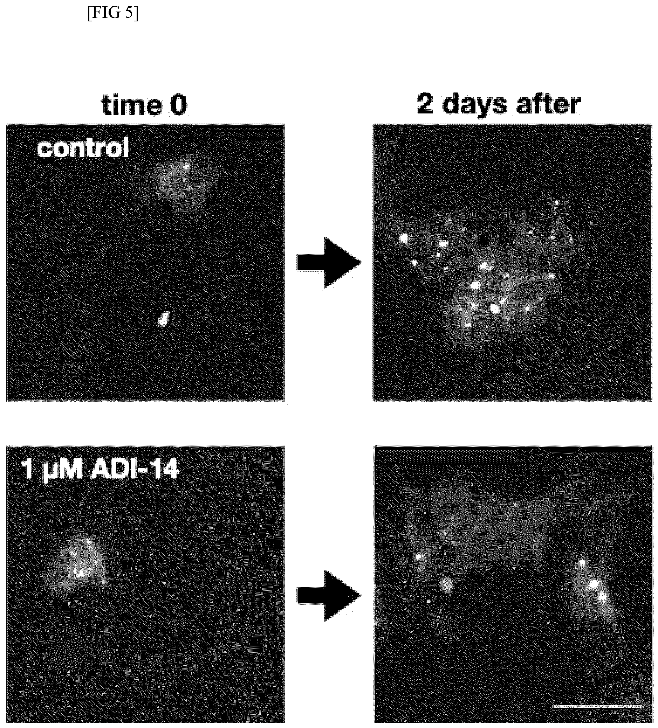

FIG. 5 shows images obtained by tracking and photographing, over time, cells at an arbitrary time defined as "time 0" and the same cells (including daughter cells) after 2 days. The number of aggregates increased after 2 days in the control group, and the number of aggregates did not increase even after 2 days in the ADI-14 group. This result is considered to be due to the fact that aggrephagy was induced by ADI-14, the tau aggregates were degraded, and the seeds were not delivered to daughter cells, resulting in the formation of cells that did not form new aggregates.

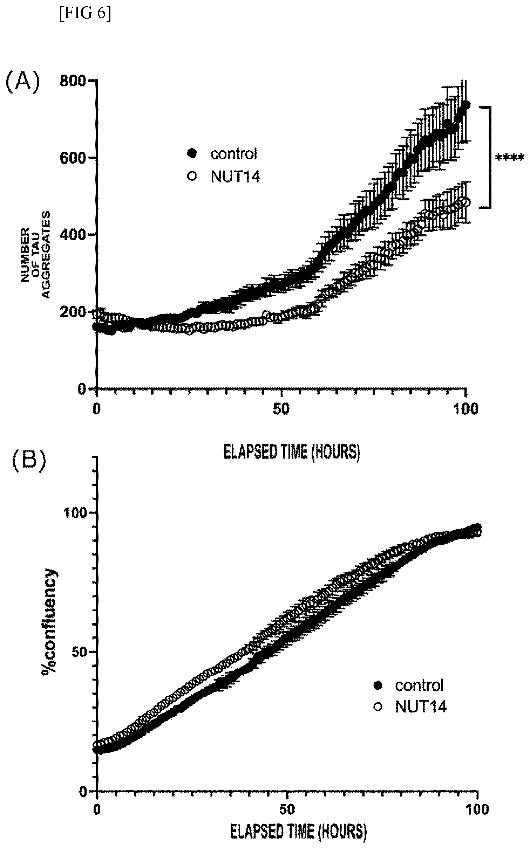

FIG. 6(A) shows a temporal change in average number of aggregates every 1 hour, with the time about 1 hour after addition of a drug at which fixed-point observation is started as t = 0. An error bar indicates the standard error of the number of aggregates (SEM) among the three wells. It was shown that the addition of ADI-14 significantly (****; p < 0.0001) decreased the number of tau aggregates. FIG. 6(B) shows an average value of proportions of cells occupying the visual field (defined as cell densities) in the three wells when a proportion in a state where cells occupy the entire visual field is 100. An error bar indicates the standard error of the number of aggregates (SEM) among the three wells. It was shown that the addition of ADI-14 did not inhibit the proliferation of the cells. These results revealed that the decrease in number of tau aggregates by ADI-14 was not due to inhibition of the proliferation of the cells.

### [Test Example 5: Verification of Effect of Administration of ADI Derivative on Neurodegenerative Disease Model Animal Forming Amyloid Protein Aggregate]

### (1) Experimental Method

### (1-1) Preparation of Dosing Solution

The ADI derivative ADI-29 (m. w.: 489.99) (100 mg) was dissolved in 2 mL of DMSO (Fujifilm) to prepare 100 mM of a stock solution. The stock solution was stored at -20°C. Ten (10) µL of the stock solution was dissolved in 1 mL of sterile olive oil (Fujifilm), and the mixture was well stirred and then allowed to stand at 65°C overnight to prepare a dosing solution (1 mM ADI-29, 0.5 mg/mL) dissolved in olive oil. In one adjustment of the dosing solution, 10 1-mL dosing solutions were prepared and stored at 4°C, and stored at room temperature for 1 week before dosing.

### (1-2) Neurodegenerative Disease Model Animal

PS19 mice (B6; C3-Tg (Prnp-MAPT*P301S) PS19Vle/J, Jackson Laboratory) were used as neurodegenerative disease model animals forming amyloid protein aggregates. The PS19 mouse is a tauopathy model mouse in which a mutant human tau (MAPT) P301S-1N4R isoform derived from frontotemporal lobar degeneration (FTDP-17) associated with familial parkinsonism linked to chromosome 17 is incorporated under a prion promoter, and formation of hyperphosphorylated tau aggregates in nerve cells and brain atrophy are confirmed at 8 to 10 months of age.

### (1-3) Administration Schedule

ADI-29 administration was initiated in 36-week-old PS19 mice. The above dosing solution (100 µL) was forcibly intragastrically administered using a feeding needle three times a week (ADI-29 administration group, n = 8). The administration period was 10 weeks. The control group (n = 7) was orally administered with 100 µL of olive oil.

### (1-4) Detection of Phosphorylated Tau Aggregate in Brain

Two days after the final administration, the mice were euthanized, and the brains were removed and fixed by immersion in formalin. Sagittal sections were prepared from formalin-fixed cerebral hemispheres and immunostained with an anti-phosphorylated tau (S202/T205) antibody (AT8, Thermo Fisher Scientific) used when detecting human tauopathy pathology to confirm tau aggregates.

### (1-5) Measurement of Thickness of CA1 Nerve Cell Layer of Hippocampus

It is known that in PS19 mice, the accumulation of AT8-positive tau aggregates causes atrophy of the hippocampus due to nerve cell death in the hippocampal nerve cell layer. Therefore, in order to measure the thickness of the CA1 nerve cell layer of the hippocampus, NeuN proteins specifically expressed in nerve cells were stained by immunofluorescence (Alexa568) with a rabbit anti-NeuN monoclonal antibody (Abcam ab177487), and the hippocampus region was photographed with a KEYENCE all-in-one microscope (BX-810) using a 40× objective lens. The width of the CA1 nerve cell layer above the dentate gyrus of the hippocampus was measured at five sites per section, and the values for two sections per mouse (total of 10 sites/mouse) were analyzed by using PRISM9 statistical software.

### (2) Results

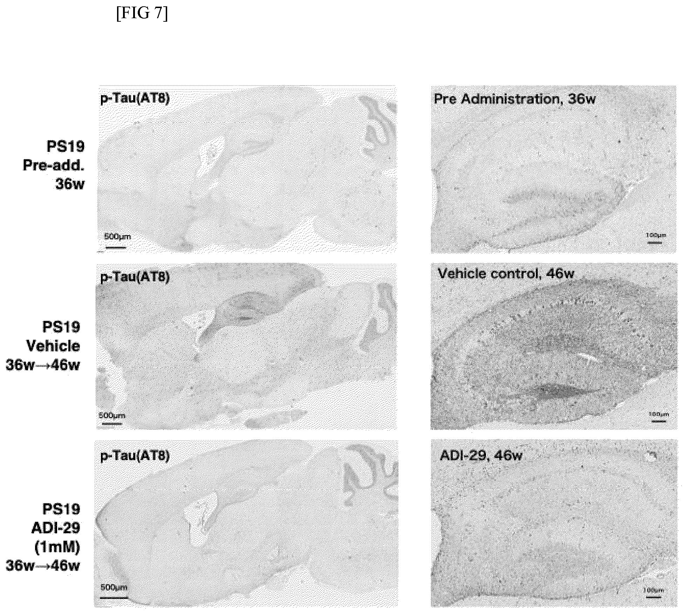

The results of the brain tissue sections immunostained with the anti-phosphorylated tau antibody are shown in FIG. 7. In the control group, the AT8-positive tau was observed as aggregates in the whole brain region including the cortex, hippocampus and brainstem, and particularly in the hippocampus, the AT8-positive tau accumulated in the nerve cell bodies in the CA1 region could be confirmed. On the other hand, in the ADI-29 administration group, although AT8-positive tau was detected in parts of brain stems and hippocampus, AT8-positive tau aggregates accumulated in the nerve cell bodies observed in the CA1 region and the like were hardly observed.

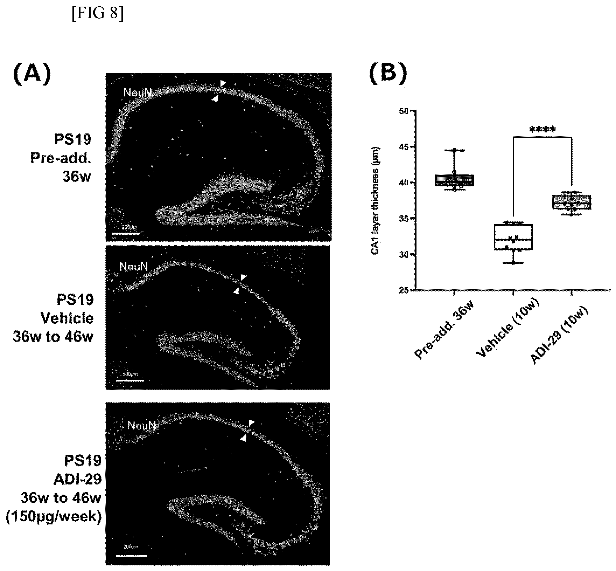

The results of measuring the thickness of the CA1 nerve cell layer of the hippocampus are shown in FIG. 8. FIG. 8(A) shows images of brain tissue sections stained by immunofluorescence with an anti-NeuN monoclonal antibody, and FIG. 8(B) shows graphs of the measured values of the width of the CA1 nerve cell layer (width between the arrowheads in (A)). As is clear from FIGS. 8(A) and 8(B), atrophy of the CA1 nerve cell layer of the 46-week-old PS19 mice was significantly suppressed in the ADI-29 administration group. This result indicates that ADI-29 inhibits nerve cell toxicity caused by mutant tau, and has a therapeutic effect for preventing formation of phosphorylated tau aggregates and nerve cell death.

### [Test Example 6: Study on Effect of ADI Derivative for suppressing Number of Aggregates by Using Tau Aggregation Cell Line]

### (1) Experimental Method

The tau aggregation cell line (G1F1B) used in Test Example 4 was used. The tau aggregation cell line was seeded at 5 × 10⁴ cells/well in a 24-well plate, and, on the next day, 1 mL of a medium (DMEM + 10% FBS) and ADI derivatives at a final concentration of 1 µM were added. DMSO was added to the control. The number of fluorescently labeled tau aggregates and the density (confluency) of cells by transmitted light were measured at nine sites per well every hour with IncuCyte ZOOM using a 20× objective lens. The averages of the measured values at the nine sites were converted to relative values with the number of aggregates at 0 hours being 1, and nonlinear curve fit (logistic growth) analysis was performed by using PRISM software according to the formula Y = YM*Y0/((YM - Y0)*exp(-k*x) + Y0) to determine YM (Ymax: multiple of increase in aggregates reached as theoretical value) as a parameter. The measurement was performed for 120 hours, and data analysis was performed until 100 hours during which the cell proliferation was maintained and the data was stable.

The results are shown in Table 1. For the relative aggregate inhibiting activity, the compounds having a strong activity were expressed as +++, those having a weak activity were expressed as +, and those having an intermediate activity were expressed as ++. The results of two wells per compound were averaged.

**[Table 1]**

| Compound | YM | |
|---|---|---|
| cont | 6.533 | - |
| ADI-9 | 1.941 | +++ |
| ADI-10 | 4.454 | + |
| ADI-11 | 2.634 | +++ |
| ADI-12 | 2.886 | ++ |
| ADI-14 | 4.762 | + |
| ADI-25 | 3.818 | + |
| ADI-27 | 2.963 | ++ |
| ADI-29 | 1.705 | +++ |
| ADI-31 | 2.151 | +++ |
| ADI-45 | 1.842 | +++ |
| ADI-51 | 4.080 | + |
| ADI-54 | 2.347 | +++ |
| ADI-63 | 3.885 | ++ |
| ADI-68 | 4.706 | + |
| ADI-74 | 3.075 | ++ |
| ADI-75 | 4.343 | + |
| ADI-79 | 2.963 | ++ |
| ADI-80 | 2.883 | +++ |
| ADI-81 | 2.408 | +++ |
| ADI-82 | 2.209 | +++ |
| ADI-88 | 1.595 | +++ |
| ADI-89 | 1.880 | +++ |

The present invention is not limited to the above-described embodiments, test examples, and examples, and various modifications can be made within the scope of the claims, and embodiments obtained by appropriately combining technical means disclosed in different embodiments are also included in the technical scope of the present invention. All of the scientific literatures and patent literatures described herein are incorporated herein by reference.

## Claims

1. A composition for promoting degradation of a protein aggregate, the composition comprising a compound represented by General Formula (I) described below or a salt thereof: 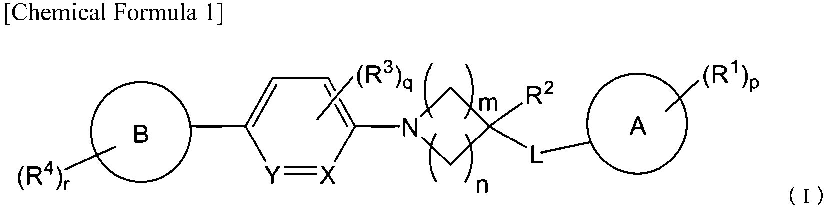
where R¹s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylaminoamide group, a carboxyl group, an amino group, a nitro group, a C₁₋₆ alkylthio group, a C₁₋₆ haloalkylthio group, a C₁₋₆ alkylsulfinyl group, a C₁₋₆ haloalkylsulfinyl group, a C₁₋₆ alkylsulfonyl group, and a C₁₋₆ haloalkylsulfonyl group;
R² is a hydrogen atom, a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a cyano group, a C(=S)NH₂ group, an amide group, or an SF₅ group;
R³s are each independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylamino group, a C₁₋₆ alkoxyamino group, a carboxyl group, and an amino group;
R⁴ are selected from the group consisting of a halogen atom, a C₁₋₆ alkyl group, a C₁₋₆ haloalkyl group, a hydroxyl group, a hydroxy C₁₋₆ alkyl group, a carboxyl group, a C₁₋₆ alkylamino group, an amino group, and a functional group represented by a formula described below: 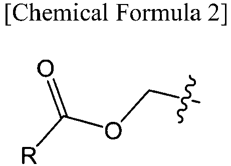
(where R is a halogen atom, a C₁₋₆ alkyl group, a C₁₋₆ haloalkyl group, a C₃₋₈ cycloalkyl group, a C₁₋₆ alkylamino group, a C₁₋₆ haloalkylamino group, a C₁₋₆ alkylamide group, a C₁₋₆ haloalkylamide group, or a phenyl group, which is optionally substituted with a halogen atom, a C₁₋₆ alkyl group, or a C₁₋₆ haloalkyl group);
L is -O-, -S-, -NH-, a C₁₋₆ alkylene group, which is optionally substituted with R, a C₂₋₆ alkenylene group, which is optionally substituted with R, a C₂₋₆ alkynylene group, which is optionally substituted with R, or a chemical bond;
X and Y are the same or different and are each N, CH or CR³;
a ring A is an aromatic hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom;
a ring B is an aromatic ring hydrocarbon having from 5 to 10 carbon atoms or a 5- or 6-membered aromatic heterocycle containing from 1 to 4 heteroatoms selected from the group consisting of a nitrogen atom, a sulfur atom, and an oxygen atom, the heteroatoms including at least one nitrogen atom;
p is 0, 1, 2, 3, 4 or 5;
q is 0, 1 or 2;
r is 0, 1, 2 or 3;
when p, q and/or r are 2 or more, two or more R¹s, R³s and/or R⁴s each optionally form a ring together with a part of a ring to be substituted;
m and n are the same or different and are each 1, 2 or 3; and
one or more of R¹s, R³s and Rs are optionally substituted with biotin or a derivative thereof.

2. The composition according to claim 1, wherein the ring B is selected from the group consisting of
phenyl, naphthyl, oxazolyl, thiazolyl, imidazolyl, pyrrolyl, pyrazolyl, isoxazolyl, isothiazolyl, 1,2,4-oxadiazol-5-yl, 1,2,4-oxadiazol-3-yl, 1,3,4-oxadiazolyl, 1,2,5-oxadiazolyl, 1,2,3-oxadiazolyl, 1,2,4-thiadiazol-5-yl, 1,2,4-thiadiazol-3-yl, 1,2,5-thiadiazolyl, 1,3,4-thiadiazolyl, 1,2,3-thiadiazolyl, 1,2,4-triazolyl, 1,2,3-triazolyl, and tetrazolyl.

3. The composition according to claim 1, wherein the ring B has any one of structures described below: 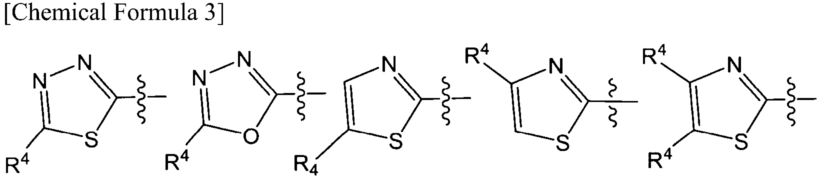 where R⁴ is as defined in claim 1.

4. The composition according to claim 1, wherein R⁴ is: 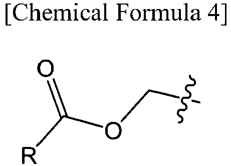 where R is as defined in claim 1.

5. The composition according to claim 1, wherein 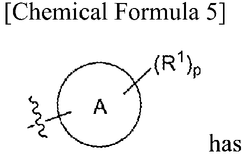 has any one of structures described below 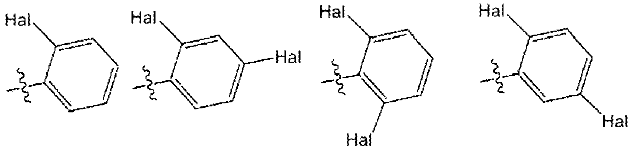 where Hals are the same or different and are each halogen or C₁₋₆ haloalkyl.

6. The composition according to claim 1, wherein X and Y are both CH.

7. A pharmaceutical composition for prevention or treatment of a neurodegenerative disease associated with protein aggregate formation, the pharmaceutical composition comprising the composition according to any one of claims 1 to 6.

8. The pharmaceutical composition according to claim 7, wherein the neurodegenerative disease associated with protein aggregate formation is selected from the group consisting of Alzheimer's disease, progressive supranuclear palsy, corticobasal degeneration, frontotemporal lobar degeneration, Parkinson's disease, multiple system atrophy, dementia with Lewy bodies, REM sleep behavior disorder, striatonigral degeneration, Creutzfeldt-Jakob disease, Gerstmann-Straussler-Scheinker disease, amyotrophic lateral sclerosis, Huntington's disease, spinocerebellar degeneration, and dentatorubropallidoluysian atrophy.

9. A composition for promoting p62/SQSTM1 phosphorylation, the composition comprising the compound represented by General Formula (I) described in claim 1 or a salt thereof.

10. The composition according to claim 8, wherein a phosphorylation promoting site is serine at position 403 in an amino acid sequence of human p62/SQSTM1 shown in SEQ ID NO: 1, or serine corresponding to the serine at position 403 of SEQ ID NO: 1 in an amino acid sequence of p62/SQSTM1 of a non-human animal.

11. A compound represented by General Formula (II) described below or a salt thereof: 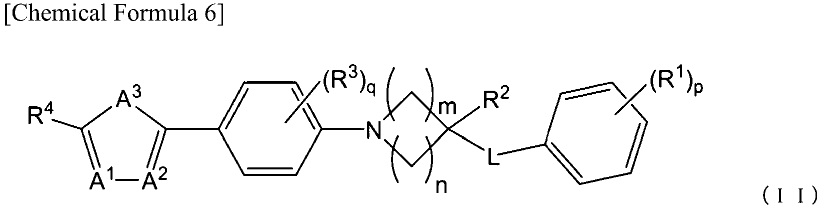
where R¹s are independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylaminoamide group, a carboxyl group, an amino group, a nitro group, a C₁₋₆ alkylthio group, a C₁₋₆ haloalkylthio group, a C₁₋₆ alkylsulfinyl group, a C₁₋₆ haloalkylsulfinyl group, a C₁₋₆ alkylsulfonyl group, and a C₁₋₆ haloalkylsulfonyl group;
R² is a hydrogen atom, a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a cyano group, a C(=S)NH₂ group, an amide group, or an SF₅ group;
R³s are independently selected from the group consisting of a halogen atom, a C₁₋₆ alkyl group, a C₂₋₆ alkenyl group, a C₂₋₆ alkynyl group, a C₁₋₆ alkoxy group, a C₃₋₈ cycloalkyl group, a C₁₋₆ haloalkyl group, a C₁₋₆ haloalkoxy group, a C₁₋₆ alkylamino group, a C₁₋₆ alkoxyamino group, a carboxyl group, and an amino group;
R⁴s are selected from the group consisting of a halogen atom, a C₁₋₆ alkyl group, a C₁₋₆ haloalkyl group, a hydroxyl group, a hydroxy C₁₋₆ alkyl group, a carboxyl group, a C₁₋₆ alkylamino group, an amino group, and a functional group represented by a formula described below: 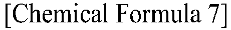 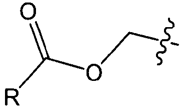
(where R is a halogen atom, a C₁₋₆ alkyl group, a C₁₋₆ haloalkyl group, a C₃₋₈ cycloalkyl group, a C₁₋₆ alkylamino group, a C₁₋₆ haloalkylamino group, a C₁₋₆ alkylaminoamide group, a C₁₋₆ haloalkylamide group, or a phenyl group, which is optionally substituted with a C₁₋₆ alkyl group, a C₁₋₆ haloalkyl group, or a halogen atom);
L is -O-, -S-, -NH-, -NR-, C₁₋₆ alkylene, which is optionally substituted with R, C₂₋₆ alkenylene, which is optionally substituted with R, C₂₋₆ alkynylene, which is optionally substituted with R, or a chemical bond;
A¹, A² and A³ are the same or different, and are independently selected from the group consisting of a sulfur atom, a nitrogen atom, CR⁴ (where R⁴ is a halogen atom, a C₁₋₆ alkyl group, a C₁₋₆ haloalkyl group, a hydroxyl group, a hydroxy C₁₋₆ alkyl group, a carboxyl group, or an amino group) and CH;
p is 1, 2 or 3;
q is 0 or 1; and
m and n are the same or different, and are each 1, 2, or 3,
provided that (i) compounds in which A¹ and A² are nitrogen atoms, A³ is an oxygen atom, and R⁴ is CH₂OH, and (ii) compounds in which A¹ and A² are nitrogen atoms, A³ is a sulfur atom, and R⁴ is CH₂-C(=O)-OCH₃ are excluded.

12. A compound represented by a formula described below or a salt thereof:

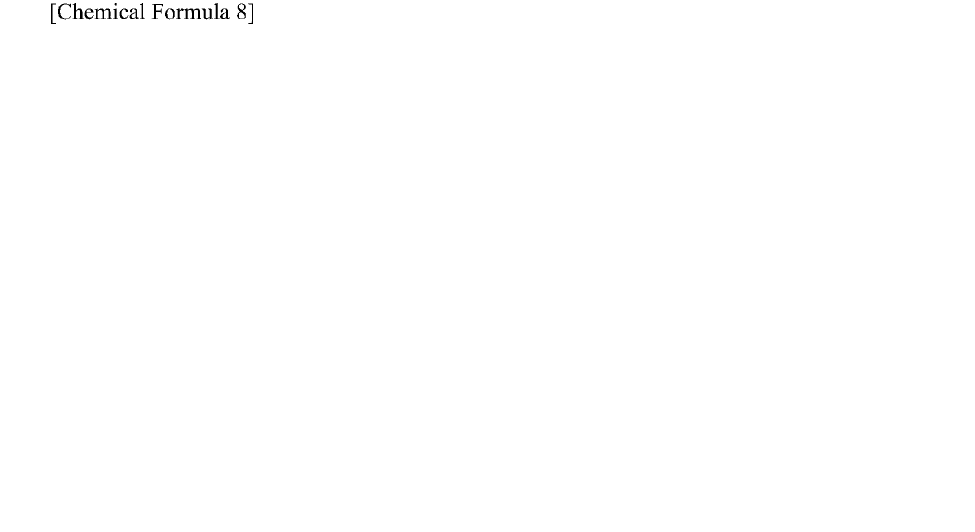

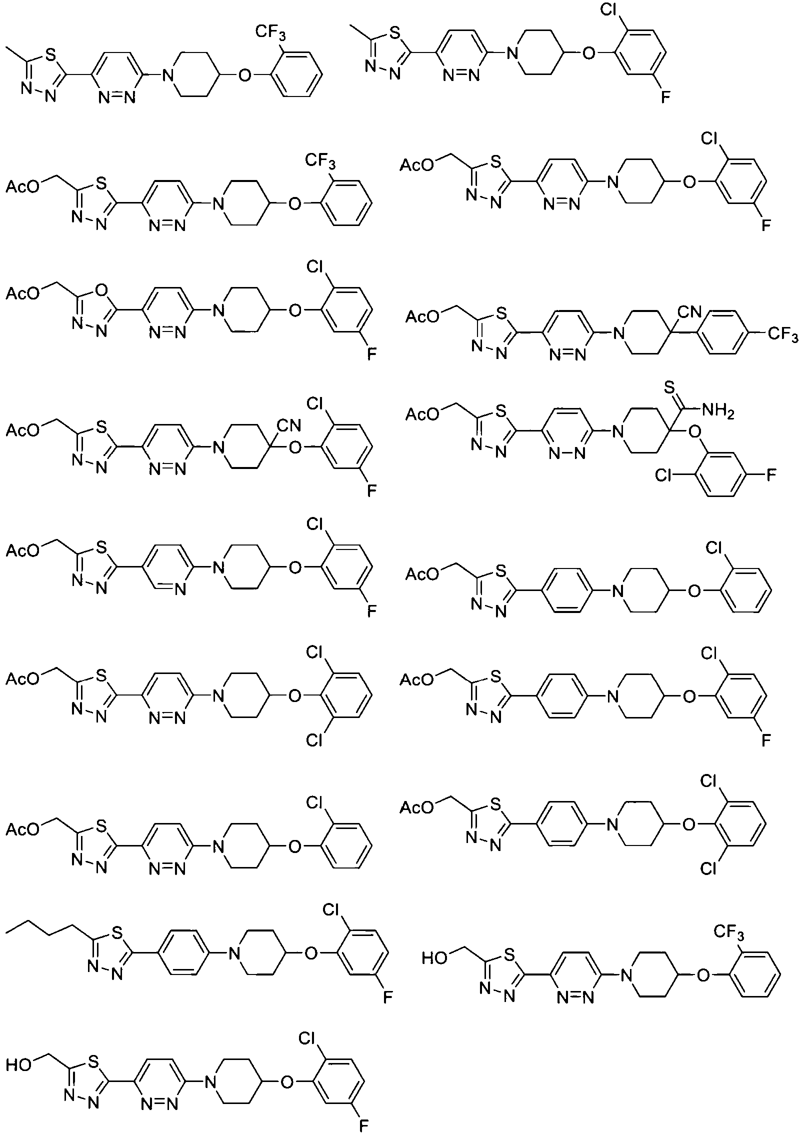

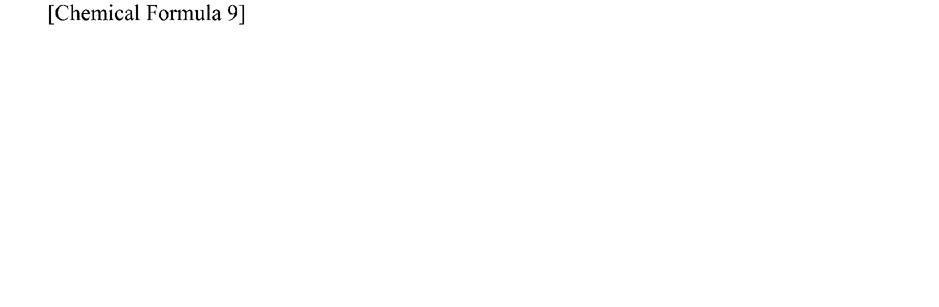

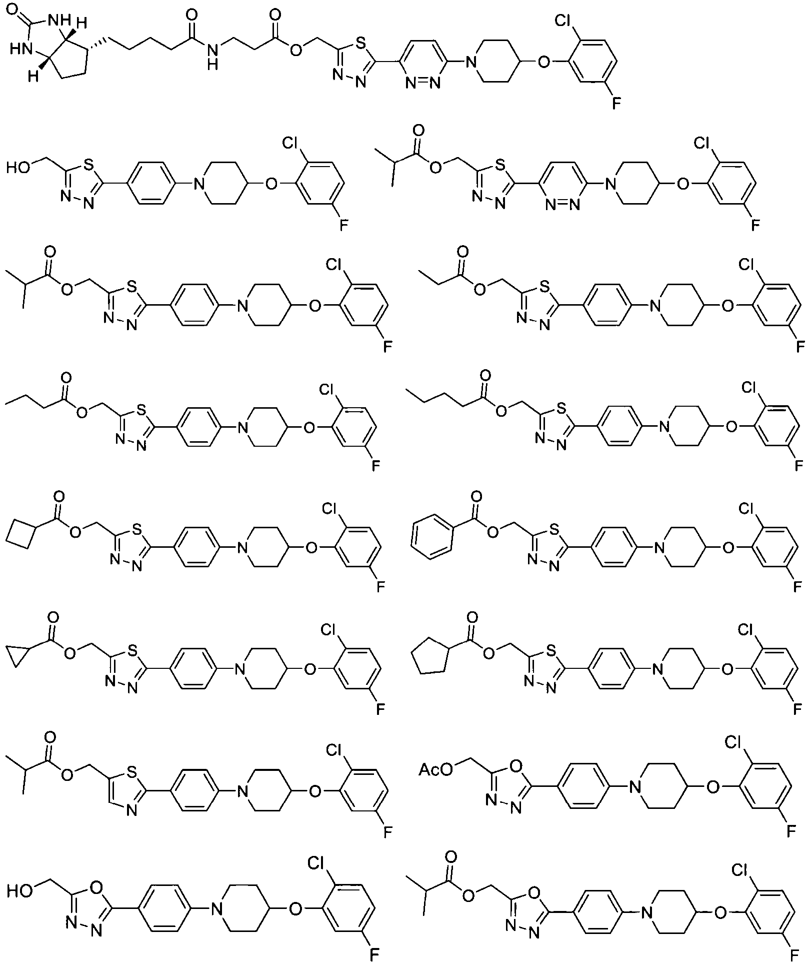

13. A compound represented by a formula described below or a salt thereof:
